# EUROPEAN PATENT SPECIFICATION

(11) **EP 1 159 276 B1**
(45) Date of publication and mention of the grant of the patent: **22.10.2003**
(21) Application number: 00909294.1
(22) Date of filing: 02.03.2000
(51) Int. Cl.: C07D 405/04, C07D 307/83, C09B 23/00, C09B 23/02, C09B 23/10, C07D 405/12, C07D 405/14, C07D 407/06

(54) **BENZOFURAN-2-ONES AS COLORANTS FOR ORGANIC MATERIALS**
BENZOFURAN-2-ONES ALS FÄRBEMITTEL FÜR ORGANISCHE MATERIALIEN
BENZOFURAN-2-ONE

(30) Priority: 10.03.1999 CH 44799
(43) Date of publication of application: 05.12.2001
(73) Proprietor: Ciba Specialty Chemicals Holding Inc., 4057 Basel (CH)
(72) Inventor: FEILER, Leonhard, D-79395 Neuenburg (DE); RUCH, Thomas, CH-4057 Basel (CH); WALLQUIST, Olof, CH-4106 Therwilt (CH); NESVADBA, Peter, CH-1723 Marly (CH)
(86) International application number: PCT/EP00/01808
(87) International publication number: WO 00/053597

(56) References cited:
- EP-A- 0 632 102
- EP-A- 0 788 890
- EP-A- 0 921 435
- WO-A-00/24736
- WO-A-80/01566
- WO-A-99/13007
- BURKE A J ET AL: "Flavonoid Epoxides. Part 20.Some Unusual Reactions of Dimethyldioxirane (DMD) with Flavonoid Compounds" TETRAHEDRON,NL,ELSEVIER SCIENCE PUBLISHERS, AMSTERDAM, vol. 53, no. 25, 23 June 1997 (1997-06-23), pages 8491-8500, XP004105780 ISSN: 0040-4020
- NICOLAIDES, DEMETROIS N. ET AL: "Reactions of ethoxycarbonylmethylene(triphenyl)phosphor ane with some ortho-quinones in the presence of triphenylphosphine, alcohols and acetic anhydride" J. CHEM. SOC., PERKIN TRANS. 1 (1992), (2), 283-9 , XP000952596
- TAKEUCHI, YASUO ET AL: "The Wittig reaction of benzofuran-2,3-diones" CHEM. PHARM. BULL. (1990), 38(8), 2265-7 , XP000952605
- GADRE, S. Y. ET AL: "Acid-catalyzed isomerization of Z-.alpha.-phenylcinnamic acids and their conversion to isomeric isoaurones" SYNTH. COMMUN. (1988), 18(9), 1015-27 , XP000952628
- SOLIMAN, F. M. ET AL: "The reaction of phosphonium ylides with 1,2-benzo[a]phenazine-8,9- dione, naphtho[2,1-b]furan-1,2-dione, benzo[b]thiophene-2,3-dione and 1,2,3-indantrione" PHOSPHORUS SULFUR (1988), 35(1-2), 41-6 , XP000952618
- SUNITHA, K. ET AL: "A reinvestigation of the Claisen rearrangement of methyl.gamma.-(aryloxy)crotonates. A convenient synthesis of 3-ethylidenebenzofuran-2(3H)-ones" TETRAHEDRON (1987), 43(14), 3269-78 , XP000952595
- CHATTERJEA, JNANENDRAN N. ET AL: "Acylation of 2-alkylnaphtho[2,1-b]furan" J. CHEM. RES., SYNOP. (1979), (11), 356 , XP002149646
- FRIEDRICHSEN, WILLY: "Photoreactions of 4,6-dimethylbenzofuran-2,3-dione" JUSTUS LIEBIGS ANN. CHEM. (1975), (9), 1545-62 , XP000952604
- CHAN, JAMES H. T. ET AL: "Annelated furans. XVII. Wittig reaction of benzofuranones" AUST. J. CHEM. (1975), 28(5), 1097-111 , XP000952616
- GIERER, JOSEF ET AL: "Lignin chromophores. I. Synthesis of chromophores of the 2,4'- and 4,4'-dihydroxystilbene types" ACTA CHEM. SCAND., SER. B (1974), 28(7), 717-29 , XP000952603
- CHAN, H. T. J. ET AL: "Annelated furans. IX. Wittig reaction of benzofuranones" SYN. COMMUN. (1972), 2(6), 409-14 , XP000952620

## Description

The invention relates to novel benzofuran-2-ones, to processes for preparing them and to their use as colorants for organic materials, especially organic materials of high or low molecular weight.

Benzofuran-2-ones as stabilizers for polymers are known, for example, from WO 80/01566. EP-A-921 435 discloses benzofuran-2-ones. EP-A-632 102 discloses that benzofuran-2-ones can be used for the mass colouring of plastics. These products, however, go only part-way towards meeting the present-day requirements in terms of application properties.

The object of the present invention was therefore to provide benzofuran-2-ones which in particular possess good solubilities in addition to giving good performance properties such as heat and light fastness and strong, transparent and bright colorations. The object was further to provide an economic process for preparing the novel benzofuran-2-ones, in accordance with the present-day requirements of an environmental process.

The present invention accordingly provides compounds of the formulae (la) or (Ic) ${\text{Q}}_{\text{1}} {\text{=X}}_{\text{1}} \text{(Ia)}$${\text{Q}}_{\text{1}} {\text{=X}}_{\text{2}} {\text{=Q}}_{\text{2}} \text{(Ic)}$in which
Q₁ is a benzofuran-2-one of the formula (IIa), and
Q₂ is a benzofuran-2-one of the formula (IIb) 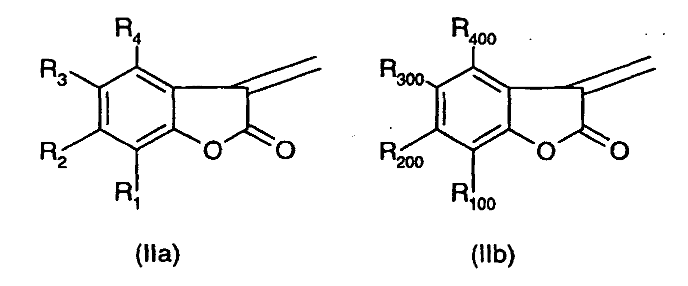 in which
R₁, R₂, R₃, R₄, R₁₀₀, R₂₀₀, R₃₀₀ or R₄₀₀ independently of one another are hydrogen, halogen, hydroxyl, cyano, ether, nitro, an amine, amide, imine, urethane, sulfonamide, ester, carboxylic acid or sulfonic acid radical or carboxylic salt, sulfonic salt or substituted or unsubstituted C₁-C₂₄alkyl, C₁-C₂₄alkoxy, C₁-C₂₄alkylthio, C₅-C₁₂cycloalkyl, C₅-C₁₂cycloalkoxy, C₅-C₁₂cycloalkylthio, C₂-C₂₄alkenyl, C₆-C₂₄aryl, C₇-C₂₅aralkyl, C₆-C₂₄aryloxy, C₆-C₂₄arylthio, A₅-A₁₈heteroaryl, A₅-A₁₈heteroaryloxy or A₅-A₁₈heteroarylthio, or
R₁ and R₂, R₂ and R₃, R₃ and R₄ or R₁₀₀ and R₂₀₀, or R₂₀₀ and R₃₀₀, R₃₀₀ and R₄₀₀, independently of one another in each case together are divalent, substituted or unsubstituted radicals, such as polycyclic radicals or 1,3-butadien-1,4-ylene or -CH=CH-NH-, the two last radicals forming an additional fused-on 5- or 6-membered ring, with the proviso that Q₁ and Q₂ are different and
X₁ is a hydrazone or imine radical, with the proviso that, if R₁, R₂, R₃ and R₄ are hydrogen, or ate least one R₁, R₂, R₃ or R₄ is methyl, the hydrazone radical is excluded, or, if R₁, R₂, R₃ or R₄ is hydrogen, X₁ is not phenylimine- or 4-dimethylamine-phenylimine, or X₁ is a methylene radical, 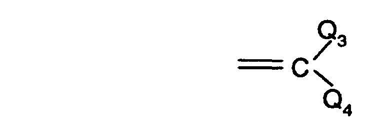 in which
Q₃ is a substituted or unsubstituted primary or secondary amine radical and Q₄ is hydrogen or a substituted or unsubstituted C₁-C₂₄alkyl, -CO-(C₁-C₂₄alkyl), -CO-O-(C₁-C₂₄alkyl), C₁-C₂₄alkoxy, C₁-C₂₄alkylthio,
C₅-C₁₂cycloalkyl, C₅-C₁₂cycloalkoxy, C₅-C₁₂cycloalkylthio, C₂-C₂₄ alkenyl, C₆-C₂₄aryl, -CO-O-(C₆-C₂₄aryl), -CO-(C₆-C₂₄aryl), C₆-C₂₄aryloxy, C₆-C₁₂arylthio, C₇-C₂₅aralkyl,
A₅-A₁₈heteroaryl, A₅-A₁₈heteroaryloxy or A₅-A₁₈heteroarylthio,
or
Q₃ and Q₄ together are a lactam, quinomethylene, hydantoin, acenaphthenequinone, azlactone, pyrazolonyl, barbituric acid, isoindolinone or isoindoline radical, with the proviso that
Q₄ is not hydrogen if R₃ is hydrogen, methoxy or hydroxyl and R₁, R₂ and R₄ are hydrogen,
and
X₂ is a tetravalent 5- or 6-membered heterocyclic ring, or is 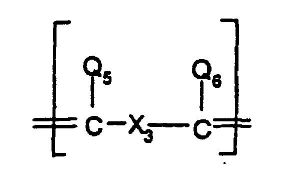 in which
X₃ is a single bond, unsubstituted or substituted C₆-C₂₄arylene, A₅-A₁₈heteroarylene, 1,2-phenylene, 1,3-phenylene, 1,4-phenylene or naphthylene, or a tetravalent polyether, polyimine, polyamine radical, or bi(C₆-C₂₄)arylene, bi(A₅-A₁₈)heteroarylene, C₂-C₂₄alkenylene, in which bi(C₆-C₂₄)arylene, bi(A₅-A₁₈)heteroarylene or C₂-C₂₄alkenylene can be interrupted by one or more intermediate units such as -CH=CH-, -CH=N-, -N=N-, -CR₄₄R₄₂-, -CO-, -COO-, -OCO-, -NR₄₂CO-, -CONR₄₂-, -O-, -S-, -SO-, -SO₂- or -NR₄₂-, in which
R₄₂ and R₄₄ independently of one another are hydrogen, substituted or unsubstituted C₁-C₂₄alkyl, C₅-C₁₂cycloalkyl, C₂-C₂₄ alkenyl, C₆-C₂₄aryl, C₇-C₂₅aralkyl or A₅-A₁₈heteroaryl, and
Q₅ and Q₆ independently of one another are hydrogen, C₆-C₂₄aryl, C₆-C₂₄aryloxy, C₁-C₂₄alkyl, C₁-C₂₄alkoxy, C₁-C₂₄alkylthio, C₅-C₁₂cycloalkyl, C₅-C₁₂cycloalkoxy, C₅-C₁₂cycloalkylthio, C₂-C₂₄alkenyl, C₆-C₂₄aryl, C₆-C₂₄aryloxy, C₆-C₂₄arylthio or A₅-A₁₈heteroaryl, A₅-A₁₈heteroaryloxy, A₅-A₁₈heteroarylthio, with the proviso that if R₂, R₄, R₂₀₀, R₄₀₀ are hydrogen and R₁, R₃, R₁₀₀ and R₃₀₀ are tert-butyl or hydrogen or R₃, R₄, R₃₀₀, R₄₀₀ are hydrogen and R₁ and R₂, R₁₀₀ and R₂₀₀ together are divalent, unsubstituted 1,3-butadien-1,4-ylene radicals, forming an additional fused 6-membered ring, and Q₅ and Q₆ are hydrogen, X₃ is not 1,4-phenylene,
or
X₂ is 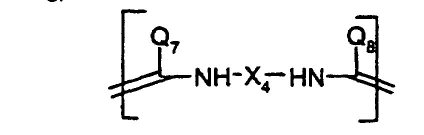 in which
Q₇ and Q₈ independently of one another are hydrogen, C₆-C₂₄aryl, C₆-C₂₄aryloxy, C₁-C₂₄alkyl, C₁-C₂₄ alkoxy, C₁-C₂₄alkylthio, C₅-C₁₂cycloalkyl, C₅-C₁₂cycloalkoxy, C₅-C₁₂cycloalkylthio, C₂-C₂₄alkenyl, C₆-C₂₄aryl, C₆-C₂₄aryloxy, C₆-C₂₄arylthio or A₅-A₁₈heteroaryl, A₅-A₁₈heteroaryloxy, A₅-A₁₈heteroarylthio, and
X₄ is C₆-C₂₄arylene, A₅-A₁₈heteroarylene,a polymethylidene or divalent polyether, polyimine, polyamine radical, or bi(C₆-C₂₄)arylene, bi(A₅-A₁₈)heteroarylene, C₂-C₂₄alkenylene, in which bi(C₆-C₂₄)arylene, bi(A₅-A₁₈)heteroarylene or C₂-C₂₄alkenylene can be interrupted by one or more intermediate units such as -CH=CH-, -CH=N-, -N=N-, -CR₄₄R₄₂-, -CO-, -COO-, -OCO-, -NR₄₂CO-, -CONR₄₂-, -O-, -S-, -SO-, -SO₂- or -NR₄₂-, or
X₂ is

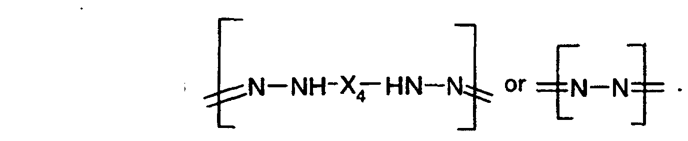

The invention also embraces tautomeric and polymorphic structures of the compounds of the formula (la) or (Ic).

In one preferred embodiment of the invention, R₁, R₂, R₃, R₄, R₁₀₀, R₂₀₀, R₃₀₀ or R₄₀₀ independently of one another are hydrogen, halogen, hydroxyl, cyano, NO₂, NR₅R₆, NR₇COR₅, NR₇COOR₅, N=CR₅R₆, CONR₇R₈, OR₅, COOR₅, (C₁-C₁₂alkyl)-COOR₅, COO⁻X⁺, SR₅, SOR₅, SO₂R₅, SO₂NR₇R₈, SO₃R₅ or SO₃⁻X⁺, or are unsubstituted or mono- or poly-halogen-, -hydroxyl-, -COOR₆-, or -COO⁻X⁺ -substituted C₁-C₁₈alkyl, C₅-C₁₆cycloalkyl, which can be uninterrupted or interrupted one or more times by O or NR₆ or are A₅-A₁₂heteroaryl, C₇-C₁₈aralkyl, C₆-C₁₀aryl or C₁-C₆alkyl unsubstituted or substituted one or more times by halogen, nitro, OR₆, SR₆, NR₇R₈, CONR₇R₈, COOR₆, COO⁻X⁺, SO₂NR₇R₈, SO₃⁻X⁺ or NR₇COR₆, or
R₁ and R₂, R₂ and R₃, R₃ and R₄, or R₁₀₀ and R₂₀₀, or R₂₀₀ and R₃₀₀, R₃₀₀ and R₄₀₀, are, for example, divalent substituted or unsubstituted radicals, such as 1,3-butadien-1,4-ylene, or -CH=CH-NH- which form an additional fused-on 5- or 6-membered ring,
R₅ is unsubstituted or mono- or poly-halogen-, -hydroxyl-, -oxo-, -cyano-, -COOR₆- or -COO⁻X⁺-substituted C₁-C₂₅alkyl, C₅-C₁₂cycloalkyl or C₂-C₂₄alkenyl which can be uninterrupted or interrupted one or more times by O, S or NR₆, or is A₅-A₁₈heteroaryl, C₇-C₁₈aralkyl or C₆-C₁₈aryl unsubstituted or substituted one or more times by halogen, nitro, cyano, OR₆, SR₆, NR₇R₈, CONR₇R₈, COOR₆, COO⁻X⁺, SO₂R₆, SO₂NR₇R₈, SO₃R₆, SO₃⁻X⁺, NR₇COR₆ or NR₇COOR₆,
R₆ is hydrogen, unsubstituted or mono- or poly-halogen-, -hydroxyl-, -oxo- or -cyano-substituted C₁-C₂₅alkyl or C₂-C₂₄alkenyl which can be uninterrupted or interrupted one or more times by O, S or NR₇, or is A₅-A₁₈heteroaryl, C₇-C₁₈aralkyl or C₆-C₁₈aryl unsubstituted or substituted one or more times by halogen, nitro, cyano, hydroxyl, OR₇, SR₇, NR₇R₈, CONR₇R₈, COOR₇, COOH or COO⁻X⁺,
R₇ and R₈ independently of one another are hydrogen, C₆-C₁₈aryl, C₇-C₁₈aralkyl, unsubstituted or mono- or poly-halogen-, -hydroxyl- or -C₁-C₁₈alkyl-, or C₁-C₁₂alkoxy-substituted C₁-C₂₄alkyl, C₆-C₁₄aryl, C₇-C₁₆aralkyl or C₂-C₂₄alkenyl, or
R₇ and R₈ together with the nitrogen are unsubstituted or mono- to tetra-C₁-C₄alkyl-substituted pyrrolidine, piperidine, piperazine or morpholine, or are carbazole, phenoxazine or phenothiazine,
X⁺ is an alkali metal cation such as Li⁺, Na⁺, K⁺ or an alkaline earth metal cation such as Mg⁺⁺_{½}, Ca⁺⁺_{½}, Sr⁺⁺_{½}, Ba⁺⁺_{½} or cations from Group 11 of the IUPAC form of the Periodic Table such as Cu⁺, Cu⁺⁺_{½} or from Group 12 of the IUPAC form of the Periodic Table such as Zn⁺⁺_{½} or from Group 13 of the IUPAC form of the Periodic Table such as Al⁺⁺⁺_{1/₃} or an ammonium radical [NR₇R₈R₁₀R₁₁]⁺, and
R₁₀ and R₁₁ independently of one another are hydrogen, C₁-C₂₄alkyl, C₅-C₂₄aryl or C₇-C₂₅aralkyl.

Preferably, at least one R₁, R₂, R₃, R₄, R₁₀₀, R₂₀₀, R₃₀₀ or R₄₀₀ independently of one another are C₃-C₂₅alkyl, which can be branched or unbranched, and also are hydroxyl, or R₁ and R₂, R₂ and R₃, R₃ and R₄ or R₁₀₀ and R₂₀₀, or R₂₀₀ and R₃₀₀, R₃₀₀ and R₄₀₀, independently of one another in each case together are divalent radicals, such as polycyclic radicals or 1,3-butadiene, 1,4-ylene or -CH=CH-NH-, the latter resulting in an additional fused-on 5- or 6-membered ring, and in a further preferred embodiment of the invention are substituted by hydroxyl.

A further preferred embodiment of the present invention comprises the compounds of the formula (Ia) in which at least two of the substituents R₁, R₂, R₃ or R₄ are not hydrogen, preferably R₁ and R₃ are not hydrogen and, with particular preference, R₁ and R₃ independently of one another are COOH or COO(C₁-C₁₂alkyl)-substituted or unsubstituted C₁-C₈alkyl or C₁-C₈alkoxy, and, with very particular preference, R₁ and R₃ independently of one another are C₁-C₄alkyl which is unsubstituted or substituted by COOH or COO(C₁-C₄alkyl), in particular by CH₂CH₂COOH, CH₂CH₂COOCH₂CH₃, CH₂CH₂CH₂COOCH₂CH₃, CH₂CH₂COOCH₃ or CH₂CH₂CH₂COOCH₃, and are in particular tert-butyl, or C₁-C₄alkoxy, especially methoxy, and
compounds of the formula (Ic) in which at least two of the substituents R₁, R₂, R₃ or R₄ and two of the substituents R₁₀₀, R₂₀₀, R₃₀₀ or R₄₀₀ are not hydrogen, preferably R₁ and R₃ and R₁₀₀ and R₃₀₀ are not hydrogen and, with particular preference, R₁ and R₃ independently of one another are COOH- or COO(C₁-C₁₂alkyl)-substituted or unsubstituted C₁-C₈alkyl or C₁-C₈alkoxy, and, with very particular preference, R₁, R₃, R₁₀₀ and R₃₀₀ independently of one another are C₁-C₄alkyl which is unsubstituted or substituted by COOH or COO(C₁-C₄alkyl), in particular by CH₂CH₂COOH, CH₂CH₂COOCH₂CH₃, CH₂CH₂CH₂COOCH₂CH₃, CH₂CH₂COOCH₃ or CH₂CH₂CH₂COOCH₃, and in particular are tert-butyl, or C₁-C₄alkoxy, especially methoxy.

Also found have been processes for preparing the compounds of the formula (I), and their use.

Alkyl, alkenyl or alkylene can be straight-chain or branched or can be interrupted by oxygen, nitrogen and/or sulfur atoms. C₁-C₂₄Alkyl is therefore, for example, with very particular preference, C₁-C₄alkyl, such as methyl, ethyl, n-propyl, isopropyl, n-butyl, sec-butyl, isobutyl, tert-butyl, cyclobutyl, with particular preference C₁-C₆alkyl, which corresponds to the definition given for C₁-C₄alkyl, and additionally is n-pentyl, 2-pentyl, 3-pentyl, 2,2-dimethylpropyl, cyclopentyl, cyclohexyl, n-hexyl, and preferably C₁-C₈alkyl, which corresponds to the definition given for C₁-C₆alkyl, and additionally is n-octyl, 1,1',3,3'-tetramethylbutyl, 2-ethylhexyl, and in particular C₁-C₁₂alkyl, which corresponds to the definition given for C₁-C₈alkyl, and additionally is trimethylcyclohexyl, decyl, menthyl, thujyl, bornyl, 1-adamantyl, 2-adamantyl or dodecyl, and also C₁-C₁₅alkyl, which corresponds to the definition given for C₁-C₁₂alkyl, and additionally is pentadecyl or tetradecyl, and, furthermore, hexadecyl, heptadecyl, octadecyl, eicosyl, heneicosyl, docosyl or tetracosyl.

C₁-C₂₄Alkylene is therefore, for example, methylene, ethylene, n-propylene, isopropylene, n-butylene, sec-butylene, isobutylene, tert-butylene, cyclobutylene, n-pentylene, 2-pentylene, 3-pentylene, 2,2-dimethylpropylene, cyclopentylene, cyclohexylene, n-hexylene, n-octylene, 1,1,3,3-tetramethylbutylene, 2-ethylhexylene, nonylene, trimethylcyclohexylene, decylene, menthylene, thujylene, bornylene, 1-adamantylene, 2-adamantylene, dodecylene, tetradecylene, hexadecylene, heptadecylene, octadecylene, eicosylene, heneicosylene, docosylene or tetracosylene.

C₂-C₂₄Alkenyl is C₂-C₂₄alkyl and preferably C₂-C₁₂alkenyl which is mono- or polyunsaturated, it being possible, if desired, for two or more double bonds to be isolated or conjugated, for example vinyl, allyl, 2-propen-2-yl, 2-buten-1-yl, 3-buten-1-yl, 1,3-butadien-2-yl, 2-cyclobuten-1-yl, 2-penten-1-yl, 3-penten-2-yl, 2-methyl-1-buten-3-yl, 2-methyl-3-buten-2-yl, 3-methyl-2-buten-1-yl, 1,4-pentadien-3-yl, 2-cyclopenten-1-yl, 2-cyclohexen-1-yl, 3-cyclohexen-1-yl, 2,4-cyclohexadien-1-yl, 2,5-hexadien-2-yl, 1-*p*-menthen-8-yl, 4(10)-thujen-10-yl, 2-norbornen-1-yl, 2,5-norbornadien-1-yl, 7,7-dimethyl-2,4-norcaradien-3-yl or the various isomers of hexenyl, octenyl, nonenyl, decenyl or dodecenyl.

C₁-C₂₄Alkoxy is O-C₁-C₂₄alkyl, preferably C₁-C₆alkoxy and, with particular preference, O-C₁-C₄alkyl, the alkyl radicals being as defined above.

C₁-C₂₄ Alkylthio is S-C₁-C₂₄alkyl, preferably C₁-C₆alkthio and, with particular preference, S-C₁-C₄alkyl, the alkyl radicals being as defined above.

C₁-C₅Acyl is, for example, -CO-methyl, -CO-ethyl, -CO-propyl, -CO-iso-propyl, -CO-sec-butyl, -CO-tert-butyl, -CO-n-butyl,-OCO-n-pentyl or -CO-sec-amyl, -CO-tert-amyl.

C₂-C₂₄Alkyl, or C₂-C₁₂alkyl, interrupted by an oxygen atom, O, nitrogen atom, N, sulfur atom, S, is, for example, C₄alkyl, such as especially -CH₂-CH₂-O-CH₂-CH₃, -CH₂-CH₂-NH-CH₂-CH₃, or -CH₂-CH₂-S-CH₂-CH₃. C₂-C₁₂Alkyl doubly interrupted by O, N or S is for example C₆alkyl, such as especially -CH₂-CH₂-O-CH₂-CH₂-O-CH₂-CH₃, -CH₂-CH₂-NH-CH₂-CH₂-NH-CH₂-CH₃, -CH₂-CH₂-S-CH₂-CH₂-S-CH₂-CH₃, O- and N-interrupted C₂-C₁₂alkyl is for example C₆alkyl, such as especially -CH₂-CH₂-O-CH₂-CH₂-NH-CH₂-CH₃. Oxo-substituted C₂-C₁₂alkyl is for example C₂alkyl, such as especially -C(=O)-CH₃. Oxo-substituted and O-interrupted C₂-C₁₂alkyl is for example C₈alkyl, such as especially -(CH₂)₃-O-C(=O)-C(CH₃)₃, -C(=O)-(CH₂)₆-OCH₃ or -C(CH₃)₂-COO-(CH₂)₃-CH₃.

C₂-C₂₄Alkylene interrupted by an oxygen radical, O, nitrogen radical, N, sulfur radical, S; is for example C₄alkylene, such as especially -CH₂-CH₂-O-CH₂-CH₂-, -CH₂-CH₂-NH-CH₂-CH₂-, or -CH₂-CH₂-S-CH₂-CH₂-. C₂-C₂₄Alkylene doubly interrupted by O, N or S is for example C₆alkylene, such as especially -CH₂-CH₂-O-CH₂-CH₂-O-CH₂-CH₂-, -CH₂-CH₂-NH-CH₂-CH₂-NH-CH₂-CH₂-, -CH₂-CH₂-S-CH₂-CH₂-S-CH₂-CH₂-, O- and N-interrupted C₂-C₁₂alkylene is for example C₆alkyl, such as especially -CH₂-CH₂-O-CH₂-CH₂-NH-CH₂-CH₂-. Oxo-substituted C₂-C₂₄alkylene is for example C₂alkylene, such as especially -C(=O)-CH₂-. Oxo-substituted and O-interrupted C₁-C₂₄alkylene is for example C₈alkylene, such as especially -(CH₂)₃-O-C(=O)-C(CH₃)₂-, -C(=O)-(CH₂)₆-OCH₂- or -C(CH₃)₂-COO-(CH₂)₃-CH₂-. Single or multiple substitution by halogen, hydroxyl, oxo or cyano, and single or multiple interruption by O, S or N, generally alter only slightly the chemical reactivity of an alkyl, alkenyl or alkylenyl group.

C₅-C₁₂Cycloalkyl is for example cyclopentyl, cyclohexyl, cycloheptyl or cyclooctyl, preferably C₅-C₆cycloalkyl such as cyclopentyl or cyclohexyl.

C₅-C₁₂Cycloalkylene is for example cyclopentylene, cyclohexylene, cycloheptylene, cyclooctylene or cyclododecylene such as 2-cyclopenten-1-yl, 2-cyclohexen-1-yl, 3-cyclohexen-1-yl, 2,4-cyclohexadien-1-yl or 1-*p*-menthen-8-yl, 4(10)-thujen-10-yl, 2-norbornen-1-yl, 2,5-norbornadien-1-yl, 7,7-dimethyl-2,4-norcaradien-3-yl, preferably C₅-C₆-cycloalkylene such as cyclopentylene or cyclohexylene.

Cycloalkyl or cycloalkylene can also be interrupted by heteroatoms such as, for example, -NH-, -S-, -O- or by units such as -CO-, -CONH₂, -CONH, -NH₂CO-, -COO- or -OCO-, and is for example piperazinyl or piperazinylene, phthalimide, piperazine-2,5-dienylene, tetrahydrofuryl, tetrahydropyryl, 2-pyrrolidonyl, thiolanyl, oxazolanyl, tetrahydroimidazolyl, tetrahydrothiazolyl, piperidinyl, dioxanyl.

C₅-C₁₂Cycloalkoxy is O-C₅-C₁₂cycloalkyl, preferably C₅-C₆cycloalkyl, the cycloalkyl radicals being as defined above.

C₅-C₁₂Cycloalkylthio is S-C₅-C₁₂cycloalkyl, preferably C₅-C₆cycloalkyl, the cycloalkyl radicals being as defined above.

Divalent polycyclic radicals are for example naphthyl, anthranyl or anthranylfuranoyl radicals.

A polycycle which can be interrupted by heteroatoms such as O, N, S or P is for example an aromatic, aliphatic or aromatic and aliphatic polycycle such as polyethers, for example a crown ether, and also polyamines or polythioethers, or for example octahydroquinolizine or tetradecahydroacridine.

Aralkyl and aryl is preferably C₇-C₁₂aralkyl or C₆-C₁₂aryl.

C₇-C₂₅Aralkyl is for example benzyl, 2-benzyl-2-propyl, β-phenylethyl, α,α-dimethylbenzyl, ω-phenylbutyl, ω,ω-dimethyl-ω-phenylbutyl, ω-phenyldodecyl, ω-phenyloctadecyl, ω-phenyleicosyl or ω-phenyldocosyl, preferably C₇-C₁₈aralkyl such as benzyl, 2-benzyl-2-propyl, β-phenylethyl, α,α-dimethylbenzyl, ω-phenylbutyl, ω,ω-dimethyl-ω-phenylbutyl, ω-phenyldodecyl or ω-phenyloctadecyl, and with particular preference C₇-C₁₂aralkyl such as benzyl, 2-benzyl-2-propyl, β-phenylethyl, α,α-dimethylbenzyl, ω-phenylbutyl, ω,ω-dimethyl-ω-phenylbutyl or ω-phenyldodecyl.

C₇-C₁₂Aralkyl is for example benzyl, 2-benzyl-2-propyl, β-phenylethyl, 9-fluorenyl, α,α-dimethylbenzyl, ω-phenylbutyl or ω,ω-dimethyl-ω-phenylbutyl.

C₆-C₂₄Aryl is for example phenyl, 1-naphthyl, 2-naphthyl, 4-biphenylyl, phenanthryl, 2- or 9-fluorenyl, anthraquinonyl or anthracenyl, preferably C₆-C₁₂aryl such as phenyl, 1-naphthyl, 2-naphthyl, 4-biphenylyl.

C₆-C₁₂aryl is for example phenyl, 1-naphthyl, 2-naphthyl, 4-biphenylyl or 2-fluorenyl.

C₆-C₂₄Aryloxy is O-C₆-C₂₄aryl, preferably C₆-C₁₂aryl, the aryl radicals being as defined above.

C₆-C₂₄Arylthio is S-C₆-C₂₄aryl, preferably C₆-C₁₂aryl, the aryl radicals being as defined above.

C₆-C₂₄Arylene is for example phenylene, 1-naphthylene, 2-naphthylene, 4-biphenylene, phenanthrylene, 2- or 9-fluorenylene, anthraquinonylene or anthracenylene, preferably C₆-C₁₂arylene such as phenylene, 1-naphthylene, 2-naphthylene or 4-biphenylene.

Bi(C₆-C₂₄)arylene is preferably biphenylene, especially 1,4- or 1,3-biphenylenes.

5- or 6-membered heterocyclic ring is A₅-A₆heteroaryl or C₅-C₆cycloalkyl or C₅-C₆cycloalkylene interrupted by heteroatoms such as O, S, N.

A₅-A₁₈Heteroaryl is a polyunsaturated heterocyclic structure, preferably a monocyclic structure, such as preferably A₅-A₆heteroaryl, and bicyclic heteroaromatic radical, and is a heteroaromatic structure comprising 5 to 18 atoms, selected from C, N, O and S, which includes at least 6 conjugated π electrons. Heteroaryl is for example thienyl, benzo[b]thienyl, dibenzo[b,d]thienyl, thianthrenyl, furyl, furfuryl, 2H-pyranyl, benzofuranyl, isobenzofuranyl, benzimidazolyl, benzothiazolyl, dibenzofuranyl, phenoxythiinyl, pyrrolyl, imidazolyl, pyrazolyl, pyridyl, bipyridyl, triazinyl, pyrimidinyl, pyrazinyl, pyridazinyl, indolizinyl, isoindolyl, indolyl, indazolyl, purinyl, quinolizinyl, quinolyl, isoquinolyl, phthalazinyl, naphthyridinyl, quinoxalinyl, quinazolinyl, cinnolinyl, pteridinyl, carbazolyl, carbolinyl, benzotriazolyl, benzoxazolyl, phenanthridinyl, acridinyl, perimidinyl, phenanthrolinyl, phenazinyl, isothiazolyl, phenothiazinyl, isoxazolyl, furazanyl or phenoxazinyl.

A₅-A₁₈Heteroaryloxy is O-A₅-A₁₈heteroaryl, the heteroaryl radicals being as defined above.

A₅-A₁₈Heteroarylthio is S-A₅-A₁₈heteroaryl, the heteroaryl radicals being as defined above.

A₅-A₁₈Heteroarylene is a polyunsaturated heterocyclic structure, preferably a monocyclic structure, such as preferably A₅-A₆heteroarylene, and bicyclic heteroaromatic radical, and is a heteroaromatic structure comprising 5 to 18 atoms, selected from C, N, O and S, which includes at least 6 conjugated π electrons. Heteroarylene is thienylene, benzo[b]thienylene, dibenzo[b,d]thienylene, thianthrenylene, furylene, furfurylene, 2H-pyranylene, benzofuranylene, isobenzofuranylene, dibenzofuranylene, phenoxythinylene, pyrrolylene, imidazolylene, pyrazolylene, pyridylene, bipyridylene, benzimidazolylene, benzothiazolylene, triazinylene, pyrimidinylene, pyrazinylene, pyridazinylene, indolizinylene, isoindolylene, indolylene, indazolylene, purinylene, quinolizinylene, quinolylene, isoquinolylene, phthalazinylene, naphthyridinylene, quinoxalinylene, quinazolinylene, cinnolinylene, pteridinylene, carbazolylene, carbolinylene, benzotriazolylene, benzoxazolylene, phenanthridinylene, acridinylene, perimidinylene, phenanthrolinylene, phenazinylene, isothiazolylene, phenothiazinylene, isoxazolylene, furazanylene or phenoxazinylene.

Bi(A₅-A₁₈)heteroarylene is for example bipyridylene, bipyrrolylen, piperazinedionylen, quinodimethylene, imidazolonylen, isoindolinylen, and anthraquinoylfuranoylen, preferably bi(A₅-A₁₀)heteroarylene and, with particular preference, piperazinedionylen and isoindolinylen.

Radical in primary or secondary amine radical is hydrogen or in hydrazone or amide radical is substituted or unsubstituted C₁-C₂₄alkyl, -CO-(C₁-C₂₄alkyl), -CO-O-(C₁-C₂₄alkyl), -CO-O-(C₆-C₂₄aryl), -CO-(C₆-C₂₄aryl), C₁-C₂₄alkoxy, C₁-C₂₄alkylthio, C₅-C₁₂cycloalkyl, C₅-C₁₂cycloalkoxy, C₅-C₁₂cycloalkylthio, C₂-C₂₄alkenyl, a primary or secondary amine radical, C₆-C₂₄aryl, C₆-C₂₄aryloxy, C₆-C₁₂arylthio, C₇-C₂₅aralkyl or A₅-A₁₈heteroaryl.

Halogen is chlorine, bromine, fluorine or iodine, preferably fluorine or chlorine.

C₂-C₁₂alkyl or C₂-C₁₂alkenyl substituted one or more times by halogen, hydroxyl, C₁-C₁₂alkoxy or cyano is for example 2-chloroethyl, trifluoromethyl, pentafluoroethyl, β,β,β-trifluoroethyl, trichlorovinyl, ω-chloropropyl, ω-bromobutyl, perfluorohexyl, perfluorododecyl, 2-hydroxyethyl, 2-methoxyethyl, 2-ethoxyethyl, 2-butoxyethyl, 2,3-dihydroxypropyl, 2,3-dimethoxypropyl, 2,3-dimethoxypropyl or 2-cyanoethyl, preferably trifluoromethyl, 2-hydroxyethyl, 2-methoxyethyl, 2-ethoxyethyl or 2-cyanoethyl.

One particularly preferred embodiment of the present invention comprises compounds of the formulae (Ia), (Ib) or (Ic) in which
X₁ is a compound selected from the group of the compounds of the formulae (III), (IV), (VI) and (VIII) 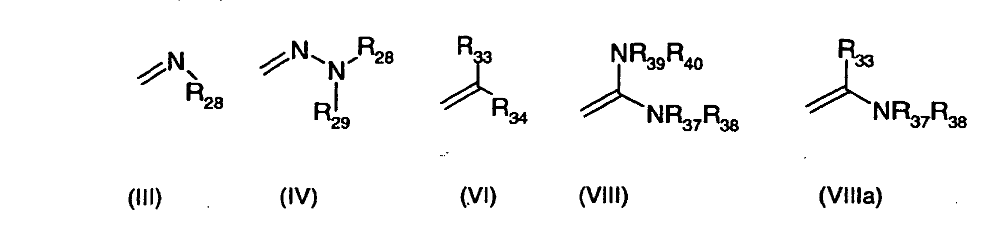 in which
R₂₈, R₂₉, R₃₇, R₃₈, R₃₉ and R₄₀ independently of one another are hydrogen or substituted or unsubstituted C₁-C₂₄alkyl, C₁-C₂₄alkoxy, C₅-C₁₂cycloalkoxy, C₅-C₁₂-cycloalkylthio, C₅-C₆cycloalkyl, C₂-C₂₄alkenyl, C₆-C₂₄aryl, C₅-C₂₄aryloxy, C₅-C₂₄arylthio, C₇-C₂₅aralkyl, a primary or secondary amine radical, A₅-A₁₈heteroaryl, A₅-A₁₈heteroaryloxy, A₅-A₁₈heteroarylthio, and with particular preference are C₆-C₁₂aryl, C₇-C₁₃aralkyl, a primary or secondary amine radical, or A₅-A₈heteroaryl, with the proviso that R₂₈ is not phenyl or 4-dimethylaminophenyl if R₁, R₂, R₃ and R₄ are hydrogen, or compound of the formula (IV) is excluded if R₁, R₂, R₃ and R₄ is hydrogen or one R₁, R₂, R₃ and R₄ is methyl,
and
R₃₃ and R₃₄ independently of one another correspond to the definition of R₂₈, with the proviso that R₃₃ and R₃₄ are not phenyl or
R₃₃ is not hydrogen and R₃₄ is not methyl, 4-aminophenyl, 4-dimethylaminophenyl or -OCO-4-(1-chlorophenylene) if R₁, R₂, R₃ and R₄ are hydrogen,
or R₃₃ is not hydrogen and R₃₄ is not 4-aminophenyl if R₁ and R₃ are tert-C₅H₁₁alkyl, or R₃₃ is not hydrogen and R₃₄ is not 2-hydroxyphenyl if R₁ and R₃ are tert-butyl, or R₃₃ is not hydrogen and R₃₄ is not a primary or secondary amine radical if R₃ is hydrogen, methoxy or hydroxyl and R₁, R₂ and R₄ are hydrogen, or
R₃₃ is not hydrogen and R₃₄ is not a secondary amine radical if R₁, R₂, R₃ and R₄ are hydrogen,
or
R₃₃ and R₃₄ together are a lactam, quinomethylene, hydantoin, acenaphthenequinone, azlactone, pyrazolonyl, barbituric acid, isoindolinone or isoindoline radical, and
X₂ is a compound selected from the group of compounds of the formulae (IX), (X), (XI), (XII) and (XIV), 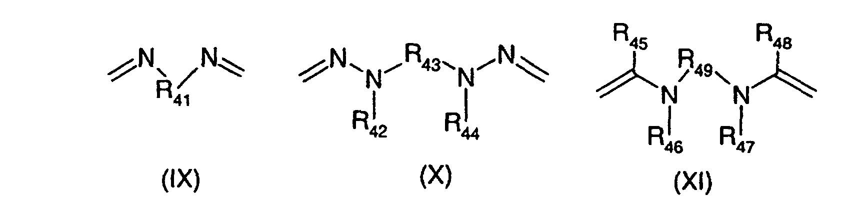 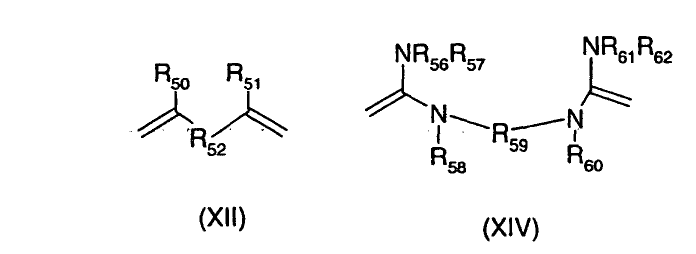 in which
R₄₂, R₄₄, R₄₆, R₄₇, R₅₀, R₅₁, R₅₆, R₅₇, R₅₈, R₆₀, R₆₁ and R₆₂ independently of one another are hydrogen or substituted or unsubstituted C₁-C₂₄alkyl, C₅-C₁₂cycloalkyl, C₂-C₂₄ alkenyl, C₆-C₂₄aryl, C₇-C₂₅aralkyl, or A₅-A₁₈heteroaryl, divalent polyether, polyimine, polyamine radical, bi(C₆-C₂₄)arylene, bi(A₅-A₁₈)heteroarylene, C₂-C₂₄alkenylene, in which bi(C₆-C₂₄)arylene, bi(A₅-A₁₈)heteroarylene or C₂-C₂₄alkenylene can be interrupted by one or more intermediate units such as -CH=CH-, -CH=N-, -N=N-, -CR₄₄R₄₂-, -CO-, -COO-, -OCO-, -NR₄₂CO-, -CONR₄₂-, -O-, -S-, -SO-, -SO₂- or -NR₄₂-,
in which
R₄₂ and R₄₄ independently of one another are hydrogen, substituted or unsubstituted C₁-C₂₄alkyl, C₅-C₁₂cycloalkyl, C₂-C₂₄alkenyl, C₆-C₂₄aryl, C₇-C₂₅aralkyl or A₅-A₁₈heteroaryl, and with particular preference are C₁-C₂₄alkyl, C₆-C₁₂aryl, C₇-C₁₂aralkyl or A₅-A₈heteroaryl, and
R₄₅ and R₄₈ independently of one another are hydrogen, C₁-C₂₄alkyl, C₁-C₂₄alkoxy, C₁-C₂₄alkylthio, C₅-C₁₂cycloalkyl, C₅-C₁₂cycloalkoxy, C₅-C₁₂cycloalkylthio, C₂-C₂₄alkenyl, C₅-C₂₄aryl, C₇-C₂₅aralkyl, C₅-C₂₄aryloxy, C₅-C₂₄arylthio or A₅-A₁₈heteroaryl, A₅-A₁₈heteroaryloxy, A₅-A₁₈heteroarylthio, and
R₄₁, R₄₃, R₅₂, R₄₉ and R₅₉ independently of one another are a direct bond or substituted or unsubstituted C₆-C₂₄arylene, A₅-A₁₈heteroarylene, C₅-C₁₂cycloalkylene or bi(C₆-C₂₄)arylene, bi(A₅-A₁₈)heteroarylene, C₂-C₂₄alkenylene, in which bi(C₆-C₂₄)arylene, bi(A₅-A₁₈)heteroarylene or C₂-C₂₄alkenylene can be interrupted by one or more intermediate units such as -CH=CH-, -CH=N-, -N=N-, -CR₄₄R₄₂-, -CO-, -COO-, -OCO-, -NR₄₂CO-, -CONR₄₂-, -O-, -S-, -SO-, -SO₂- or -NR₄₂-, in which
in which
R₄₂ and R₄₄ independently of one another are hydrogen, substituted or unsubstituted C₁-C₂₄alkyl, C₅-C₁₂cycloalkyl, C₂-C₂₄alkenyl, C₆-C₂₄aryl, C₇-C₂₅aralkyl, or A₅-A₁₈heteroaryl.

A further preferred embodiment of the present invention comprises compounds of the formula (XVI) 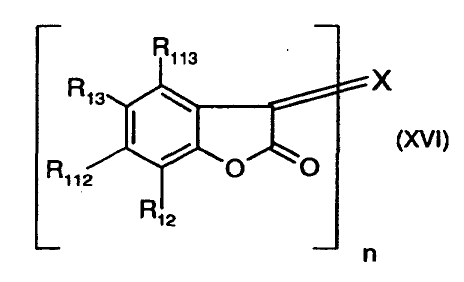 in which
n is 1
X is X₁, and corresponds to the above definition of X₁, and
R₁₂, R₁₁₂, R₁₃ and R₁₁₃ independently of one another are hydrogen, halogen, OH, NO₂, R₁₄, OR₁₄, OC₉-C₁₈alkyl or SC₉-C₁₈alkyl,
in which
R₁₄ is C₁-C₂₄alkyl which is unsubstituted or substituted one or more times by oxo or by COO⁻X₅⁺ and which can be uninterrupted or interrupted one or more times by O, N and/or S, or is C₇-C₁₈aralkyl or C₆-C₁₂aryl unsubstituted or substituted one or more times by halogen, OR₁₆, NR₁₆R₁₇, COOR₁₆, CONR₁₆R₁₇, NR₁₈COR₁₆ or NR₁₈COOR₁₆,
X₅⁺ is a cation H⁺, Na⁺, K⁺, Mg⁺⁺_{½}, Ca⁺⁺_{½}, Zn⁺⁺_{½}, Al⁺⁺⁺_{1/3}, or [NR₁₆R₁₇R₁₈R₁₉]⁺, and
R₁₆ and R₁₇ independently of one another are hydrogen, C₆-C₁₂aryl, C₇-C₁₀aralkyl, or C₁-C₈alkyl which is unsubstituted or substituted one or more times by halogen, hydroxyl or C₁-C₄alkoxy, or
R₁₆ and R₁₇ in NR₁₆R₁₇ or CONR₁₆R₁₇, together with the nitrogen atom connecting them, are pyrrolidine, piperidine, piperazine or morpholine each of which is unsubstituted or substituted from one to four times by C₁-C₄alkyl,
and R₁₈ and R₁₉ independently of one another are hydrogen, C₁-C₈alkyl, C₆-C₁₀aryl or C₆-C₁₂aralkyl,
R₁₂ and R₁₁₂, R₁₁₂ and R₁₃, R₁₃ and R₁₁₃ can also independently of one another each together be divalent substituted or unsubstituted radicals, such as polycyclic radicals, preferably naphthylene or 1,3-butadien-1,4-ylene or -CH=CH-NH-, the latter resulting in an additional fused-on 5- or 6-membered ring, preferably a phenylene ring,
with the proviso that, if R₁₂, R₁₁₂, R₁₃ and R₁₁₃ are hydrogen, or at least one R₁₂, R₁₁₂, R₁₃ and R₁₁₃ is methyl, X₁ is not a hydrazone radical, or if R₁₂, R₁₁₂, R₁₃ and R₁₁₃ are hydrogen, X₁ is not phenylimine- or 4-dimethylamine-phenylimine, or if X₁ is a methylene radical, 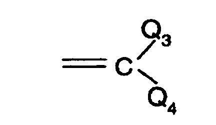 Q₄ is not hydrogen if R₁₃ is hydrogen, methoxy or hydroxyl, and R₁₂, R₁₁₂ and R₁₁₃ are hydrogen.

Preferably, R₁₂, R₁₁₂, R₁₃ and R₁₁₃ or the above divalent substituted or unsubstituted radicals independently of one another are hydrogen, OH, OR₂₂, COOR₁₆, CONR₇R₈, chlorine, COO⁻X₆⁺, R₂₂, C₂H₅-COOH, C₂H₅-COO(C₁-C₁₂alkyl) or C₂H₅-COO(C₁-C₁₂alkyl) which can be uninterrupted or interrupted one or more times by O, N, S, and in which C₁-C₁₂alkyl can be interrupted, and with very particular preference are OH, chlorine, COO⁻X₆⁺, COOR₁₆ or CONR₇R₈
in which
R₂₂ is substituted or unsubstituted C₆-C₁₂aryl, C₇-C₁₂aralkyl or is C₁-C₈alkyl which is unsubstituted or substituted one or more times by oxo, cyano, COOR₁₆ or COO⁻X₆⁺ and which can be uninterrupted or interrupted one or more times by O, N, S,
X₆⁺ is H⁺, an alkali metal cation such as Na⁺, K⁺ or an alkaline earth metal cation such as Mg⁺⁺_{½}, Ca⁺⁺_{½}, or cations from Group 12 of the IUPAC form of the Periodic Table such as Zn⁺⁺_{½} or from Group 13 of the IUPAC form of the Periodic Table such as Al⁺⁺⁺_{1/3}, or an ammonium radical [NR₂₄R₂₅R₂₆R₂₇]⁺, in which
R₂₄, R₂₅ and R₂₆ independently of one another are C₁-C₄alkyl or phenyl, and
R₂₇ is hydrogen, C₁-C₈alkyl, C₆-C₁₂aryl or C₇-C₁₂aralkyl,

Preferably, R₁₂ and R₁₃ and R₁₁₂ and R₁₁₃ are not hydrogen, and with particular preference independently of one another are tert-butyl, O-CH₃, CH₂CH₂COOH or CH₂CH₂COO(C₁-C₁₂alkyl), CH₂CH₂CH₂COO(C₁-C₁₂alkyl), and with very particular preference R₁₂ is tert-butyl and
R₁₃ is tert-butyl, O-CH₃, CH₂CH₂COOH or CH₂CH₂COO(C₁-C₁₂alkyl), CH₂CH₂CH₂COO(C₁-C₁₂alkyl) and
R₁₁₂ and R₁₁₃ are hydrogen,
or
R₁₁₃ is hydrogen or OH,
or
R₁₂ together with R₁₁₂ is a divalent fused-on phenylene radical and
R₁₃ and R₁₁₃ are hydrogen,
or
P₁₃ together with R₁₁₃ is a divalent fused-on phenylene radical and R₁₂ and R₁₁₂ are hydrogen.

Further in particular, with very particular preference,
R₁₂ is tert-butyl and
R₁₃ is tert-butyl, O-CH₃, CH₂CH₂COOH or CH₂CH₂COOCH₂CH₃, CH₂CH₂CH₂COOCH₂CH₃, CH₂CH₂COOCH₃, CH₂CH₂CH₂COOCH₃, and
R₁₁₂ is Hydrogen.

One particularly preferred embodiment of the present invention comprises compounds of the formulae (la) or (Ic) in which X₁ is a hydrogen radical of the formula (IV) and X₂ is a compound of the formulae (X), especially =N-NR₆₃R₆₄, and with very particular preference is a compound of the formula (XVII) 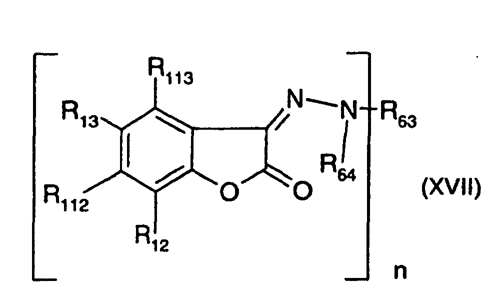 in which
n is 1
R₆₄ independently of R₆₃ is a radical as defined under R₆₃ or hydrogen, and
R₆₃ is substituted or unsubstituted C₁-C₁₂alkyl, C₅-C₁₂cycloalkyl, C₂-C₆alkenyl, C₆-C₁₂aryl, C₇-C₁₃aralkyl, or A₅-A₁₂heteroaryl, especially anthranyl, and with particular preference is C₆-C₁₂aryl, C₇-C₁₂aralkyl or A₅-A₈heteroaryl, and with very particular preference is a compound selected from the group of the compounds of the formulae (XVIII), (IXX), (XX) and (XXI), 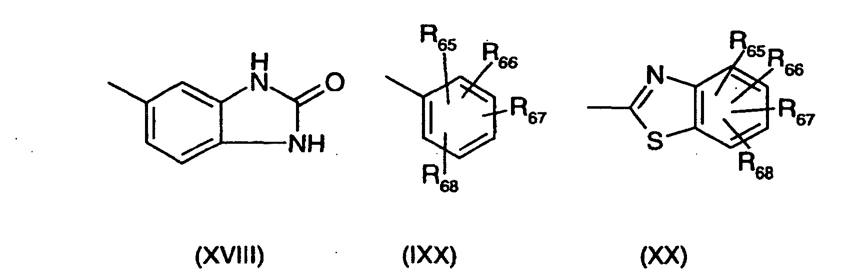 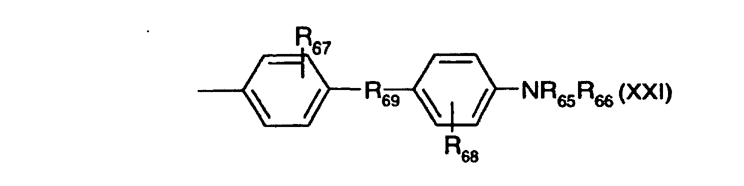 in which
R₆₅ and R₆₆ independently of one another are hydrogen, hydroxyl, cyano, nitro, halogen, especially fluoro or chloro, are C₅-C₆cycloalkyl, unsubstituted or R₆₇-substituted phenyl or are C₁-C₁₈alkyl, C₁-C₁₈alkoxy, in which alkyl especially is methyl, ethyl, propyl, isopropyl, n-butyl, isobutyl, sec-butyl, tert-butyl, n-pentyl, sec-amyl, tert-amyl, hexyl or 2,2-dimethylbutyl, and
R₆₈ independently of R₆₇ has the same definition as R₆₇, and
R₆₉ is a direct bond -CH₂-, -CH(CH₃)-, -C(CH₃)₂-, -CH=N-, -N=N-, -O-, S-, -SO-, -SO₂- or -NR₆₅-,
R₆₇ is hydrogen, nitro, cyano, halogen such as fluorine, chlorine, bromine, iodine or C₁-C₈-alkyl, C₁-C₆alkoxy, unsubstituted or NR₆₅R₆₆-substituted C₅-C₆cycloalkyl, and is preferably nitro, chlorine, C₁-C₆alkyl or C₁-C₆alkoxy,
with the proviso that, if R₁, R₂, R₃ and R₄ are hydrogen or one R₁, R₂, R₃ or R₄ is methyl, the hydrazone radical is excluded.

A further particularly preferred embodiment of the present invention comprises compounds of the formula (la) or (Ic) in which X₁ is an imine radical of the formula (III) and X₂ is a compound of the formula (IX), especially = N-R₇₇ and, with very particular preference, is a compound of the formula (XXIV) 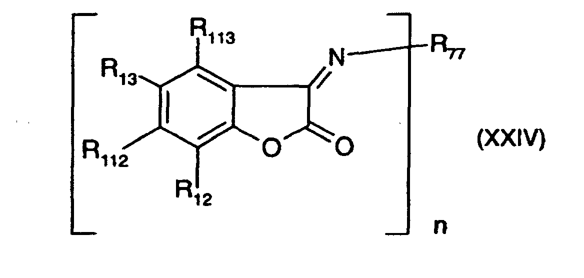 in which, n is 1,
R₇₇ is substituted or unsubstituted C₁-C₁₂alkyl, C₅-C₆cycloalkyl, C₂-C₆alkenyl, C₆-C₁₂aryl, C₇-C₁₃aralkyl, or A₅-A₁₂heteroaryl, with the proviso that in formula (XXIV), if R₁₂, R₁₁₂, R₁₃ or R₁₁₃ are hydrogen, R₇₇ is not unsubstituted phenylimine or 4-dimethylaminephenylimine,
with very particular preference is a compound of the formula (IXX), especially with very particular preference is a compound of the formula (IXX) in which R₆₅, R₆₆, R₆₇ or R₆₈ independently of one another are hydroxyl or hydrogen, with the proviso that in formula (XXIV), if R₁₂, B₁₁₂, R₁₃ or R₁₁₃ are hydrogen, R₇₇ is not unsubstituted phenylimine- or 4-dimethylaminephenylimine.

Furthermore, the present invention provides with particular preference compounds of the formula (la) or (Ic) in which X₁ is a compound of the formula (VIII) and X₂ is a compound of the formula (XI) or (XIV), in particular a compound of the formula (XXV)

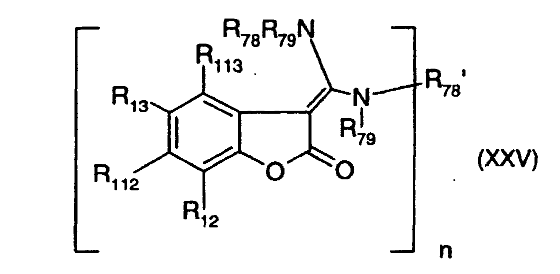

in which,
n is 1,
R_{78,} R₇₈' and R₇₉ independently of one another are hydrogen or substituted or unsubstituted C₁-C₁₂alkyl, C₁-C₁₂alkoxy, C₁-C₁₂alkylthio, C₅-C₆cycloalkoxy, C₅-C₆cycloalkylthio, C₆-C₂₄aryloxy, C₆-C₂₄arylthio or A₅-A₁₂heteroaryloxy, A₅-A₁₂heteroarylthio, C₅-C₆cycloalkyl, C₂-C₁₂alkenyl, C₆-C₁₂aryl, C₇-C₁₃aralkyl, or A₅-A₁₂heteroaryl or dependently of one another are hydrogen, and, with very particular preference,
independently of one another are a compound of the formula (IXX) or hydrogen.

The present invention further provides, in one particularly preferred embodiment, compounds of the formula (la) or (Ic), in which X₁ is a compound of the formula (VI) or (VII) and X₂ is a compound of the formula (XII), in particular and with very particular preference is a compound of the formula (XXVI) 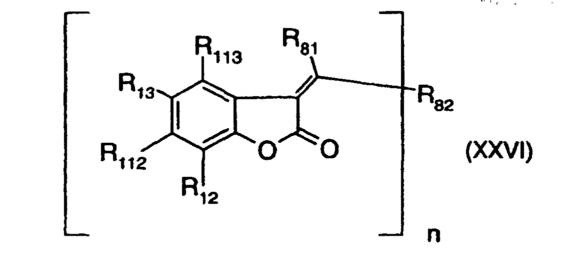 n is 1
R₈₁ is a primary or secondary amine radical, and R₈₂ is hydrogen or unsubstituted or substituted C₁-C₁₂alkyl, -CO-(C₁-C₂₄alkyl), -CO-O-(C₁-C₂₄alkyl), C₆-C₁₂aryloxy, C₁-C₁₂alkoxy, C₁-C₁₂alkylthio, C₅-C₁₂cycloalkyl, C₅-C₁₂cycloalkoxy, C₂-C₁₂alkenyl, a primary or secondary amine radical, C₆-C₁₈aryl, -CO-O-(C₆-C₂₄aryl), -CO-(C₆-C₂₄aryl), C₆-C₁₈aryloxy, C₆-C₁₈arylthio or A₅-A₁₂heteroaryl, A₅-A₁₂heteroaryloxy, A₅-A₁₂heteroarylthio, with particular preference are anthranyl, substituted or unsubstituted phenyl, C₅-C₁₂cycloalkyl, A₅-A₁₂heteroaryl such as benzimidazolyl or benzothiazolyl, or are -CO-O-(C₆-C₂₄aryl), -CO-(C₆-C₂₄aryl), C₆-C₁₈aryloxy, C₆-C₁₈arylthio or A₅-A₁₂heteroaryl, A₅-A₁₂heteroaryloxy, A₅-A₁₂heteroarylthio, and C₅-C₁₂aryloxy, C₁-C₆alkoxy, C₅-C₆cycloalkoxy, and
R₈₁ and R₈₂ together are a lactam, quinomethylene, hydantoin, acenaphthenequinone, azlactone, pyrazolonyl, barbituric acid, isoindolinone or isoindoline radical, of the formulae (XXVII, XXVIIa-XXVIId), (XXVIII), (IXXX, IXXXa-IXXXc) or (IXC). 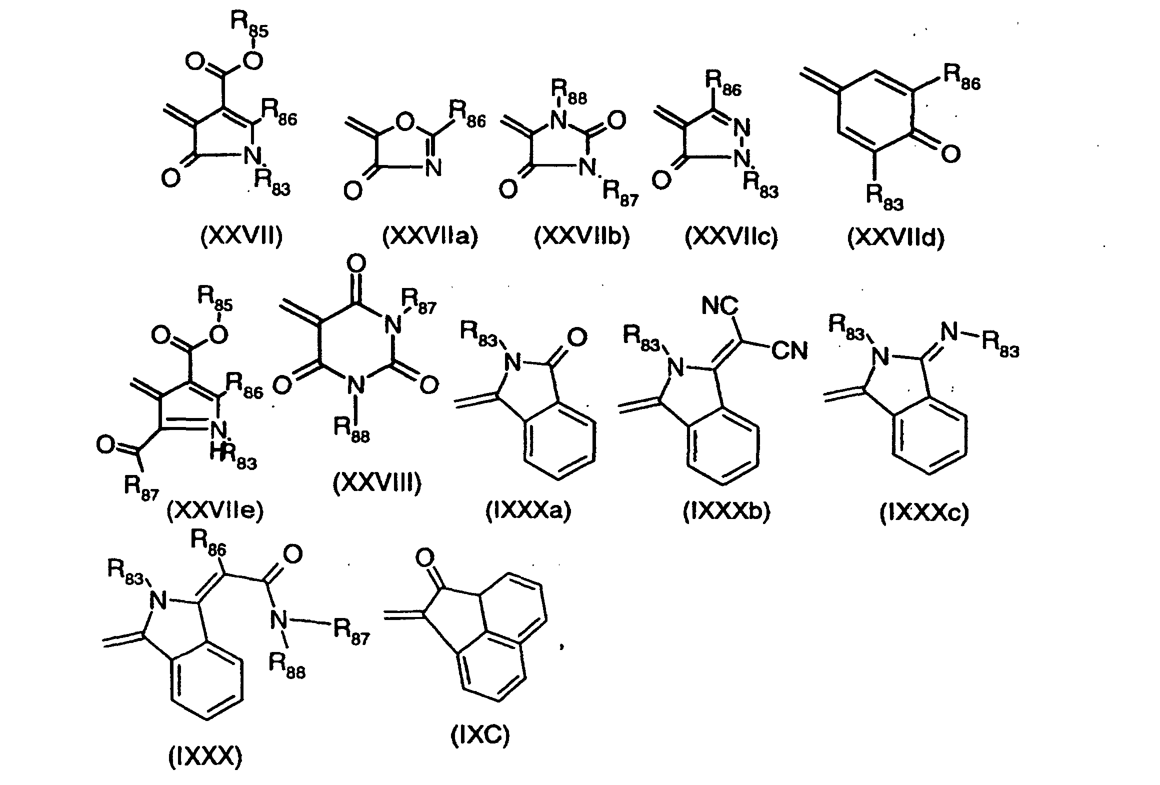 with particular preference are a compound of the formula (XXVIII), (IXC), (IXXX), (IXXXb), (IXXXa) or (XXVIIa),
in which
R₈₃, R₈₅, R₈₇ and R₈₈ independently of one another are substituted or unsubstituted C₁-C₁₈alkyl, -CO-(C₁-C₂₄alkyl), -CO-O-(C₁-C₂₄alkyl), C₅-C₁₂cycloalkyl, C₂-C₁₈ alkenyl, C₆-C₁₈aryl, -CO-O-(C₆-C₂₄aryl), -CO-(C₆-C₂₄aryl), C₇-C₁₉aralkyl or A₅-A₁₈heteroaryl, and with particular preference are C₆-C₁₂aryl, C₇-C₁₃aralkyl or A₅-A₈heteroaryl, and
R₈₆ is hydrogen or substituted or unsubstituted C₁-C₁₈alkyl, C₁-C₁₈ alkoxy, C₁-C₁₈ alkylthio, C₅-C₁₂cycloalkyl, C₅-C₁₂cycloalkoxy, C₅-C₁₂cycloalkylthio, C₂-C₁₈alkenyl, C₆-C₁₈aryl or C₇-C₁₉aralkyl,
with the proviso that
R₈₂ is not hydrogen if R₁₃ is hydrogen, methoxy or hydroxyl and R₁₂, R₁₁₂ and R₁₁₃ are hydrogen.

With particular preference, furthermore, n is 1, R₈₁, is NH₂ and R₈₂ is substituted or unsubstituted phenyl.

The compounds of the formula (I) according to the invention are generally obtained by C-H-acidic coupling reaction of a benzofuran-2-one with a couplable compound in the presence of an acidic or basic catalyst (in analogy to Organikum, 19th Edition 1993, pp. 459-495).

Accordingly, the present invention likewise provides a process for preparing the benzofuran-2-ones (la) by reacting benzofuran-2-one (XXXa) 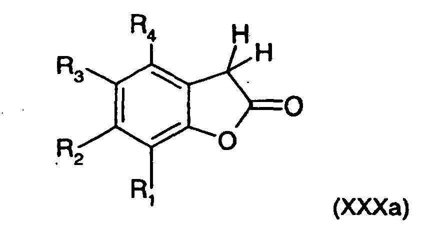 with a compound of the formula (XXXIa), (XXXIIa), (XXXIIIa), (XXXIVa) or (XXXVa) (X 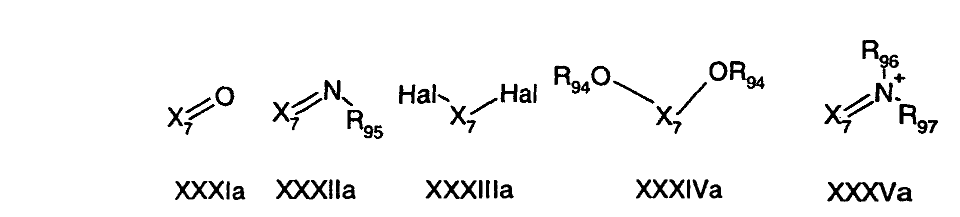 in which
Hal is halogen, and
R₉₄ is substituted or unsubstituted C₁-C₂₄alkyl, C₅-C₁₂cycloalkyl, C₂-C₂₄alkenyl,
C₆-C₂₄aryl, C₇-C₂₅aralkyl or A₅-A₁₈heteroaryl, and
R₉₅ is hydrogen or hydroxyl,
R₉₆ and R₉₇ independently of one another are C₆-C₁₂aryl, especially phenyl, or are C₁-C₅acyl, C₆-C₁₂aralkyl, C₁-C₄alkyl, and R₉₆ and R₉₇ in particular are methyl, or phenyl and CH₃CO, or phenyl and methyl, and
X₇ is a methylene radical, 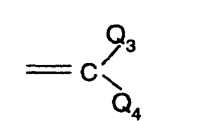 in which
Q₃ is a substituted or unsubstituted primary or secondary amine radical and Q₄ is hydrogen or a substituted or unsubstituted C₁-C₂₄alkyl, -CO-(C₁-C₂₄alkyl), -CO-O-(C₁-C₂₄alkyl), C₁-C₂₄alkoxy, C₁-C₂₄alkylthio, C₅-C₁₂cycloalkyl, C₅-C₁₂cycloalkoxy, C₅-C₁₂cycloalkylthio, C₂-C₂₄ alkenyl, C₆-C₂₄aryl, -CO-O-(C₆-C₂₄aryl), -CO-(C₆-C₂₄aryl), C₆-C₂₄aryloxy, C₆-C₁₂arylthio, C₇-C₂₅aralkyl, A₅-A₁₈heteroaryl, A₅-A₁₈heteroaryloxy or A₅-A₁₈heteroarylthio,
or
Q₃ and Q₄ together are a lactam, quinomethylene, hydantoin, acenaphthenequinone, azlactone, pyrazolonyl, barbituric acid, isoindolinone or isoindoline radical,
with the proviso that Q₄ is not hydrogen if R₃ is hydrogen, methoxy or hydroxyl and R₁, R₂ and R₄ are hydrogen.

The reaction is usually started by bringing benzofuran-2-one (XXXa) into contact with a compound of the formulae (XXXIa), (XXXIIa), (XXXIIIa), (XXXIVa) or (XXXVa) in accordance with methods known per se, for example by mixing the starting materials or adding one starting material dropwise to the other.

The molar ratio of a compound (XXXa) to a compound of the formulae (XXXIa), (XXXIIa), (XXXIIIa), (XXXIVa) or (XXXVa) is generally chosen in the range from 0.8:1 to 3:1; preferably, the molar ratio lies within the range from 0.9:1 to 2:1.

If desired, the reaction can be carried out in an organic solvent or in a melt; preferably, the reaction is carried out in an organic solvent.

The molar ratio of organic solvent to the compound (XXXa) is generally chosen in the range from 500:1 to 1:2, preferably from 100:1 to 1:1.

The reaction temperature is usually chosen in the range from -20 to 250°C; preferably from 0 to 200°C, the reaction temperature chosen preferably being a temperature at which the reaction mixture boils; it lies within the region of the boiling temperature of the solvent used.

The pressure chosen is preferably atmospheric pressure.

The reaction time is usually chosen as a function of the reactivity of the starting materials, and of the chosen temperature, and is generally within the range from 10 minutes to 48 hours.

If desired, the reaction can be carried out in the presence of a catalyst. .

In general, the molar ratio of the catalyst to the compound of the formulae (XXXIa), (XXXIIa), (XXXIIIa), (XXXIVa) or (XXXVa) is chosen in the range from 0.001:1 to 5:1, preferably in the range from 0.001:1 to 1:1.

Both acidic and basic catalysts are suitable.

Examples of acidic catalysts which can be used as inorganic acids, such as hydrochloric, phosphoric, hydrobromic or sulfuric acid, or zinc chloride, aluminium chloride or boron trifluoride, or organic acids or alkyl acids such as formic, acetic, propionic, chloroacetic or trifluoroacetic acid, or sulfonic acids such as arylsulfonic acids such as p-toluene- or methanesulfonic acid or silicates such as Fulkat 40 (Pontecchio Marconi), Katalysator K10 (Süd Chemie) or Katalysator Rudex (Rudex Nebelova Bratislava).

Examples of suitable basic catalysts are organic amines such as triethylamine, dialkylamine, piperidine, pyrrolidine, pyridine, morpholine, N,N-dimethylaniline, or aliphatic alkoxides, such as, for example, sodium methoxide, ethoxide, propoxide or butoxide or potassium tert-butoxide, or aromatic alkoxides such as phenoxide, for example, or carboxylic salts such as, for example, sodium or potassium acetate, or alkali metal or alkaline earth metal oxides, hydroxides, hydrides or carbonates, such as, for example, sodium or potassium hydroxide, sodium or potassium hydride, calcium oxide, magnesium oxide, sodium carbonate, potassium carbonate, sodium hydrogen carbonate, or ammonia or tetrabutylammonium hydroxide.

As the solvent it is common to choose organic solvents, especially inert organic solvents, such as, for example, ethers such as tetrahydrofuran, dioxane, diethyl ether, methyl tert-butyl ether, glycols and their ethers such as, for example, mono-, di-, tri-, tetraethylene glycol, propylene glycol, the methyl, ethyl and butyl ethers thereof, or C₅-C₁₂alkanes such as pentane, hexane, heptane, octane, nonane, decane, undecane, dodecane or C₅-C₁₂cycloalkane such as cyclopentane, cyclohexane, cycloheptane, cyclooctane, cyclononane, cyclodecane or cyclododecane or, in particular, halogenated alkanes such as dichloromethane, dichloroethane, trichloromethane, tetrachloromethane, dichloroethylene, trichloroethane or tetrachloroethane, or aryls such as benzene, toluene, or xylene, chloro-, dichloro-, trichlorobenzene, or alcohols such as methanol, ethanol, propanol, sec-propanol, butanol, or carboxylic acids such as, for example, formic acid, acetic acid, propionic acid, or esters such as ethyl acetate, polar aprotic solvents such as N,N-dimethylformamide, N-methylpyrrolidine, dimethylacetamide or dimethyl sulfoxide.

Furthermore, the reaction is carried out, with particular preference, under an inert gas atmosphere. The inert gas used can comprise noble gases, preferably helium and argon, and also nitrogen.

It has been found advantageous in the process of the invention to use additions of binding agents such as anhydrides, especially acetic anhydride, or to use physical methods, such as distillation, for example, to remove leaving groups that are formed.

The molar ratio of anhydride to the compound of the formulae (XXXIa), (XXXIIa), (XXXIIIa), (XXXIVa) or (XXXVa) lies within the range from 0.1:1 to 5:1, preferably in the range from 0.5:1 to 2:1.

The product can be isolated by the conventional methods, such as by filtration and, if desired, by subsequent washing of the filter residue with, for example, water and/or an organic solvent such as methanol, and drying of the moist filter residue.

Normally, the organic phase, for example, comprising the reaction product, is washed with water and subsequently concentrated, preferably to dryness. In a further variant of working up, the organic reaction product can also be concentrated by evaporation directly and then purified by means, for example, of recrystallization or column-chromatographic separation. In the case of recrystallization, isolation is normally done by filtration and subsequent washing of the filter residue with, preferably, a solvent in which the reaction product is poorly soluble. The column-chromatographed organic phase comprising the reaction product can be concentrated by evaporation directly. If desired, the reaction products can be dried after isolation. This is generally done using conventional drying apparatus such as drying ovens or paddle dryers.

In the process of the invention it has been found advantageous to work up the product by adding, for example, water and/or acid and then subjecting the crude product to extraction with an organic solvent, such as toluene. In general, the organic phase, comprising the crude product, is washed with water and then concentrated by evaporation. If desired, the crude product is subsequently recrystallized. In general, for this purpose, the crude product is admixed with an organic solvent such as methanol, for example, and the resulting mixture is heated at boiling. Normally, the boiling temperature is maintained until all of the product has dissolved. Subsequently, the mixture is cooled, usually to a temperature in the range from -20 to 40°C, and filtered, the product being obtained as the filter residue. Normally, the filter residue is subsequently dried in vacuo in the range from 40 to 200°C, preferably in the range from 60 to 120°C.

In addition, the present invention also provides a process for preparing the benzofuran-2-ones (Ic) by reacting benzofuran-2-one (XXXa) and a compound of the formula (XXXb) 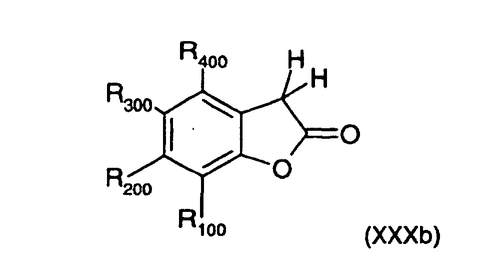 with a compound of the formulae (XXXIb), (XXXIIb), (XXXIIIb), (XXXIVb) or (XXXVb) 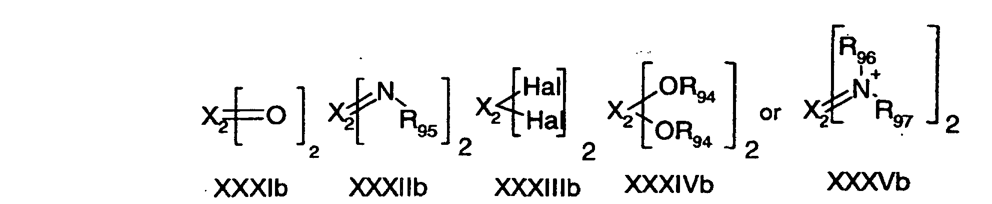 in which
X₂ is X₈ and corresponds to the definition of X₂, with the exception of X₈ 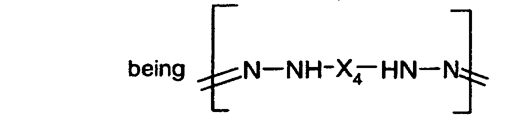 or 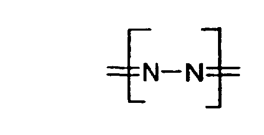 with the proviso that the compounds of formula (XXXa) and (XXXb) are different.

The reaction is normally started by bringing benzofuran-2-ones of the formula (XXXa) or (XXXa) and (XXXb) into contact with a compound of the formulae (XXXIb), (XXXIIb), (XXXIVb) or (XXXVb), in analogy to known methods, for example by mixing the starting components, or adding one starting component dropwise to the other, it being possible to react the benzofuran-2-ones (XXXa), or (XXXa) and (XXXb), in two portions or one portion.

In general, the molar ratio of a compound (XXXa), or (XXXa) and (XXXb), to a compound of the formulae (XXXIb), (XXXIIb), (XXXIIIb), (XXXIVb) or (XXXVb) is chosen in the range from 1:0.9 to 1:0.2; preferably, the molar ratio is situated within the range from 1:0.6 to 1:0.4.

If desired, the reaction can be carried out in an organic solvent or in a melt; preferably, the reaction is carried out in an organic solvent.

The molar ratio of organic solvent to the compound (XXXa), or (XXXa) and (XXXb), is generally chosen in the range from 500:1 to 1:2, preferably from 100:1 to 1:1.

The reaction temperature is usually chosen within the range from -20 to 250°C, preferably from 0 to 200°C; in general it has been found advantageous to choose as the reaction temperature a temperature at which the reaction mixture boils; it lies within the range of the boiling temperature of the solvent used.

The pressure chosen is preferably atmospheric pressure.

The reaction time is usually chosen as a function of the reactivity of the starting materials, and of the chosen temperature; it is generally situated within the range from 10 minutes to 48 hours.

If desired, the reaction can be carried out in the presence of a catalyst.

In general, the molar ratio of the catalyst to the compound of the formulae (XXXIb), XXXIIb), (XXXIIIb), (XXXIVb) or (XXXVb) is chosen in the range from 0.001:1 to 5:1, preferably in the range from 0.001:1 to 1:1.

Both acidic and basic catalysts are suitable.

Catalysts and solvents are as defined above.

Furthermore, the reaction is carried out, with particular preference, under an inert gas atmosphere. The inert gas used can comprise noble gases, preferably helium and argon, and also nitrogen.

It has been found advantageous in the process of the invention to use additions of binding agents such as anhydrides, especially acetic anhydride, or to use physical methods, such as distillation, for example, to remove leaving groups that are formed.

The molar ratio of anhydride to the compound of the formulae (XXXIb), (XXXIIb), (XXXIIIb), (XXXIVb) or (XXXVb) lies within the range from 0.1:1 to 5:1, preferably within the range from 0.5:1 to 2:1.

Working up and isolation are carried out as described above.

In addition, the present invention further provides a process for preparing the benzofuran-2-ones (la) by reacting 3-oxobenzofuran-2-one (XXXVIa) 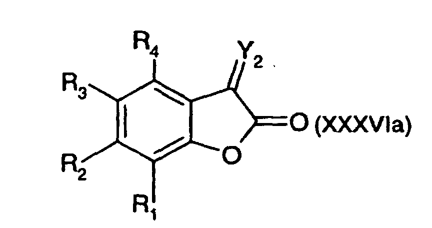 with a compound of the formula (XXXVIIa) 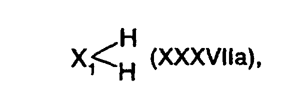 in which
Y₂ is O, NR₉₅ or N⁺(R₉₆R₉₇), NO or two chlorine atoms, the chlorine atoms each forming a single bond with the benzofuran-2-one (Ia).

The reaction is usually started by bringing 3-oxobenzofuran-2-one (XXXVIa) into contact with a compound of the formulae (XXXVIIa) in accordance with methods known per se, for example by mixing the starting materials or adding one starting material dropwise to the other.

The molar ratio of a compound (XXXVIa) to a compound of the formulae (XXXVIIa) is generally chosen in the range from 0.8:1 to 3:1; preferably, the molar ratio lies within the range from 0.9:1 to 2:1.

If desired, the reaction can be carried out in an organic solvent or in a melt; preferably, the reaction is carried out in an organic solvent.

The molar ratio of organic solvent to the compound (XXXVIa) is generally chosen in the range from 500:1 to 1:2, preferably from 100:1 to 1:1.

The reaction temperature is usually chosen in the range from -20 to 250°C, preferably from 0 to 200°C, the reaction temperature chosen preferably being a temperature at which the reaction mixture boils; it lies within the region of the boiling temperature of the solvent used.

The pressure chosen is preferably atmospheric pressure.

The reaction time is usually chosen as a function of the reactivity of the starting materials, and of the chosen temperature, and lies generally within the range from 10 minutes to 48 hours.

If desired, the reaction can be carried out in the presence of a catalyst.

In general, the molar ratio of the catalyst to the compound of the formulae (XXXVIIa) is chosen in the range from 0.001:1 to 5:1, preferably in the range from 0.001:1 to 1:1.

Both acidic and basic catalysts are suitable.

Catalysts and solvents are as defined above.

Furthermore, the reaction can, if desired, be carried out under an inert gas atmosphere. The inert gas used can comprise noble gases, preferably helium and argon, and also nitrogen.

It has been found advantageous in the process of the invention to use additions of binding agents such as anhydrides, especially acetic anhydride, or to use physical methods, such as distillation, for example, to remove leaving groups that are formed.

The molar ratio of anhydride to the compound of the formulae (XXXIa), (XXXIIa), (XXXIIIa), (XXXIVa) or (XXXVa) usually lies within the range from 0.1:1 to 5:1, preferably within the range from 0.5:1 to 2:1.

Working up and isolation are carried out as described above.

A further embodiment of the process of the invention comprises preparing the benzofuran-2-ones (Ic) by reacting 3-oxobenzofuran-2-one (XXXVIa) and a compound of the formula (XXXVIb) 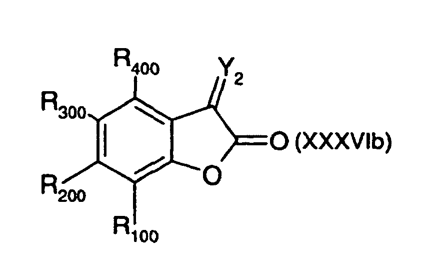 with a compound of the formula (XXXVIIb) 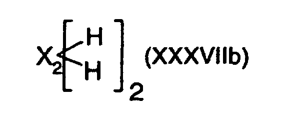 with the proviso that the compound of formula (XXXVIa) and (XXXVIb) are different.

The reaction is normally started by bringing 3-oxobenzofuran-2-ones of the formula (XXXVIa), or (XXXVIa) and (XXXVIb) into contact with a compound of the formula (XXXVIIb) in analogy to known methods, for example by mixing the starting components, or adding one starting component dropwise to the other, it being possible to react the 3-oxobenzofuran-2-ones (XXXVIa), or (XXXVIa) and (XXXVIb), in two portions or one portion.

In general, the molar ratio of a compound (XXXVIa), or (XXXVIa) and (XXXVIb), to a compound of the formulae (XXXVIIb) is chosen in the range from 1:0.9 to 1:0.2; preferably, the molar ratio lies within the range from 1:0.6 to 1:0.4.

If desired, the reaction can be carried out in an organic solvent or in a melt; preferably, the reaction is carried out in an organic solvent.

The molar ratio of organic solvent to the compound (XXXVIa), or (XXXVIa) and (XXXVIb), is generally chosen in the range from 500:1 to 1:2, preferably from 100:1 to 1:1.

The reaction temperature is usually chosen within the range from -20 to 250°C, preferably from 0 to 200°C; in general it has been found advantageous to choose as the reaction temperature a temperature at which the reaction mixture boils; it lies within the range of the boiling temperature of the solvent used.

The pressure chosen is preferably atmospheric pressure.

The reaction time is usually chosen as a function of the reactivity of the starting materials, and of the chosen temperature; it lies generally within the range from 10 minutes to 48 hours.

If desired, the reaction can be carried out in the presence of a catalyst.

In general, the molar ratio of the catalyst to the compound of the formulae (XXXVIIb) is chosen in the range from 0.001:1 to 5:1, preferably in the range from 0.001:1 to 1:1.

Both acidic and basic catalysts are suitable.

Catalysts and solvents are as defined above.

Furthermore, the reaction is carried out, with particular preference, under an inert gas atmosphere. The inert gas used can comprise noble gases, preferably helium and argon, and also nitrogen.

It has been found advantageous in the process of the invention to use additions of binding agents such as anhydrides, especially acetic anhydride, or to use physical methods, such as distillation, for example, to remove leaving groups that are formed.

The molar ratio of anhydride to the compound of the formula (XXXVIIb) lies within the range from 0.1:1 to 5:1, preferably within the range from 0.5:1 to 2:1.

Working up and isolation are carried out as described above.

The starting compounds (XXXa) and (XXXb) are available commercially or are readily obtainable from phenols by reaction with glyoxal, for example, in accordance with the process of H.-D. Becker, K. Gustafsson, J. Org. Chem. 42, 2966 (1977).

The starting compounds of the formulae (XXXI a or b) can be prepared, for example, in analogy to processes from EP-B 632 102 or Advanced Organic Chemistry, Jerry March, Ed. 1977, p. 824; those of the formulae (XXXII a or b), for example, in analogy to processes from Advanced Organic Chemistry, Jerry March, Ed. 1977, p. 817, or Advanced Organic Chemistry, Jerry March, Ed. 1977, p. 824; those of the formulae (XXXIII a or b), for example, in analogy to processes from US 2 701 252; those of the formulae (XXXIV a or b), for example, in analogy to processes from Advanced Organic Chemistry, Jerry March, Ed. 1977, p. 810; or those of the formulae (XXXV a or b), for example, in analogy to processes from EP-B 632 102; or those of the formulae (XXXVII a or b), for example, in analogy to processes from DE-A1 1 952 962; or they are available commercially.

The 3-oxobenzofuran-2-ones can be prepared, for example, in analogy to known methods for the preparation of 3-unsubstituted furanones and 3-oxofuranone compounds. 3-Unsubstituted furanones, for example, can be prepared in analogy to the process of H.-D. Becker, K. Gustafsson, J. Org. Chem. 42, 2966 (1977) from phenols by reaction with glyoxal.

3-Oxobenzofuran-2-ones (XXXVIa) and (XXXVIb) can be prepared, moreover, by oxidizing 3-unsubstituted benzofuran-2-ones, or by oxidizing 3-hydroxy-3-oxobenzofuran-2-ones in accordance with conventional methods for oxidizing hydroxy compounds to keto compounds. These methods are described, for example, in Houben-Weyl, Methoden der Organischen Chemie, 4th Edition, Volume 4/1a & 4/1b. In J. Org. Chem., 56, 6110 (1991), the oxidation with nitroxides is described by Z- Ma, J.M. Bobbitt. 3-Hydroxy-3-oxobenzofuran-2-ones can be prepared in analogy to the process described in US 5 614 572. In addition, 3-oxobenzofuran-2-ones can be prepared in analogy to the process described by D.J. Zwaneburg and W.A.P. Reyen in Synthesis, 624, 1976.

One particularly preferred embodiment of the present invention comprises the novel aminohydroxy compounds of the formula (XLIa) or (XLIb) 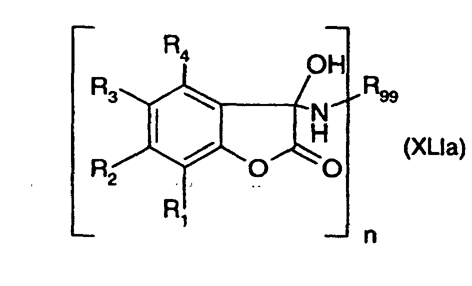 or 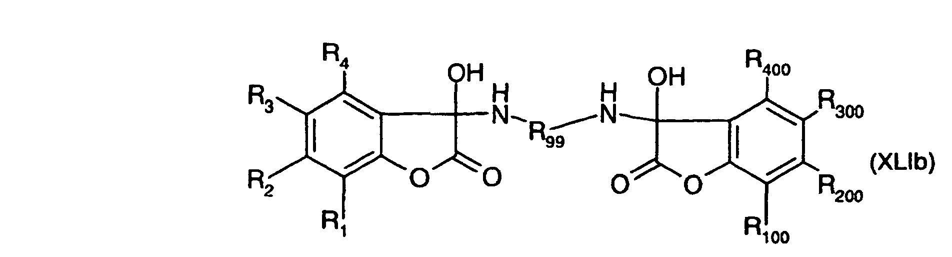 in which
n is 1 and
R₉₉ is hydrogen or substituted or unsubstituted C₁-C₂₄alkyl, C₅-C₁₂cycloalkyl, C₂-C₂₄alkenyl, C₆-C₂₄aryl, C₇-C₂₅aralkyl, or A₅-A₁₈heteroaryl,
preferably A₅-A₁₈heteroaryl, C₆-C₂₄aryl unsubstituted or substituted by halogen, hydroxyl, cyano, ether, nitro, an amine, amide, imine, urethane, sulfonamide, ester, carboxylic acid, sulfonic acid radical or carboxylic salt, sulfonic salt, especially compound of the formula (XVIII).

The present invention further provides a process for preparing amine hydroxy compounds of the formulae (XLIa) or (XLIb) by reacting 3-oxobenzofuran-2-one (XXXVIa) with a compound of the formula (XXXVIIa) 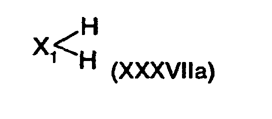 or
3-oxobenzofuran-2-one (XXXVIa), or (XXXVIa) and (XXXVIb), with a compound of the formula (XXXVIIb)

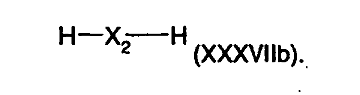

A further particularly preferred embodiment of the present invention comprises a process for preparing the compound of the formulae (XLIa) or (XLIb) by reacting 3-oxobenzofuran-2-one (XXXVIa) with a compound of the formula (XXXVIIa), or 3-oxobenzofuran-2-one (XXXVIa), or (XXXVIa) and (XXXVIb), with a compound of the formula (XXXVIIb) in the presence of a catalyst, in particular of a silicate as catalyst.

Very particular preference is further given to a process for preparing the compound of the formula (XLI) by reacting 3-oxobenzofuran-2-one (XXXVIa) with a compound of the formula (XXXVIIa), or 3-oxobenzofuran-2-one (XXXVIa), or (XXXVIa) and (XXXVIb), with a compound of the formula (XXXVIIb) in the presence of a catalyst, in particular of a silicate as catalyst, at temperatures in the range from 0 to 200°C, preferably from 20 to 160°C, with particular preference from 20 to 40°C.

The present invention further provides a process for preparing the benzofuran-2-ones (la) or (Ic) in which X₁ is a compound of the formula (IV) and X₂ is a compound of the formula (X) by coupling diazotized amines to coupling components in an aqueous medium, by reacting benzofuran-2-one (XXXa) or (XXXb) with a diazonium salt of the formula (XXXVIIIa) 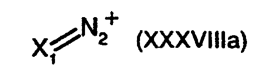 or benzofuran-2-one (XXXa), or (XXXa) and (XXXb), with a diazonium salt of the formula (XXXVIIIb)

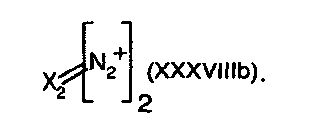

The reaction is normally started by bringing the diazonium salt, generally in the form of a solution, into contact with benzofuran-2-one, for example by mixing the starting materials or by adding one starting material dropwise to the other. The sequence of the addition is generally unimportant; preferably, benzofuran-2-one is added to a solution of the diazonium salt. Benzofuran-2-one can be in the form of a solution, dispersion or suspension, preferably a solution.

For the preparation of the benzofuran-2-ones of the formula (la) it is common to choose the molar ratio of a compound (XXXa) to a compound of the formula (XXXVIIIa) in the range from 0.8:1 to 3:1; preferably, the molar ratio lies within the range from 0.9:1 to 2:1.

For the preparation of the benzofuran-2-ones of the formula (Ic), the molar ratio of a compound (XXXa) or (XXXa) and (XXXb) to a compound of the formula (XXXVIIIb) is generally chosen in the range from 1:0.9 to 1:0.2; preferably, the molar ratio is situated within the range from 1:0.66 to 1:0.4

The solvent chosen for the solution, dispersion or suspension is generally water, sodium acetate, sodium formate or an organic solvent such as formic, acetic, propionic acid, especially glycol ethers such as ethylene glycol monoethyl ether, or mixtures of these solvents, especially mixtures containing water.

The molar ratio of solvent to the compound (XXXa), or (XXXa) and (XXXb), is generally chosen in the range from 500:1 to 1:2, preferably from 100:1 to 1:1.

The reaction temperature is usually chosen in the range from -20 to 100°C, preferably from 0 to 50°C.

The pressure chosen is preferably atmospheric pressure.

The reaction time is usually chosen as a function of the reactivity of the starting materials, and of the chosen temperature, and is generally situated within the range from 10 minutes to 48 hours.

If desired, the reaction can be carried out in the presence of nonionogenic, anionic or cationic surface-active substances, which may have a cloud point in aqueous medium. If desired, it is also possible to use further auxiliaries, such as natural or synthetic resins or resin derivatives, or additives customary for paints, printing inks or plastics.

The product can be isolated in accordance with the customary methods, such as by adding water and then carrying out filtration, for example, or directly by filtration. If desired, the filter residue can be washed with, for example, water and/or an organic solvent such as methanol, and then dried.

If desired, in one preferred embodiment of the process of the invention, the crude product can be heated at boiling, or recrystallized, in an organic solvent and then isolated. In general, for this purpose, the crude product is admixed with an organic solvent and the resulting mixture is heated at boiling for from 1 to 24 hours. It is subsequently cooled, usually to a temperature in the range from -20 to 40°C, and the mixture is filtered, the product being obtained as the filter residue. Normally, the filter residue is subsequently dried in vacuo in the range from 40 to 200°C, preferably in the range from 60 to 120°C.

The starting materials of the formulae (XXXVIIIa) or (XXXVIIIb) are readily obtainable in accordance, for example, with Houben-Weyl, Volume 10/3.

The present invention likewise provides a process for preparing benzofuran-2-ones (Ia) or (Ic) in which X₁ is X₁₀ and X₁ is a compound of the formula (V) 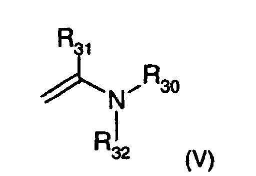 in which
R₃₁ is hydrogen or -NR₈₉R₉₀, in which
R₃₀, R_{32,} R₈₉ and R₉₀ independently of one another are hydrogen, C₁-C₂₄ alkoxy, C₁-C₂₄alkylthio, C₅-C₁₂cycloalkoxy, C₅-C₁₂cycloalkylthio, C₅-C₂₄aryloxy, -thio or A₅-A₁₈hetereoaryloxy, -thio, or are C₆-C₂₄aryl-substituted secondary or tertiary amine or C₆-C₂₄aryl, or with X₂ is a compound of the formula (XI), by formylation and subsequent reaction with an amine, by reacting benzofuran-2-one (XXXa) with a formylating reagent of the formula (XXXVIII)

R₃₅C(OR₃₆)₃ (XXXVIII)

in which
R₃₅ and R₃₆ independently of one another are substituted or unsubstituted C₁-C₂₄alkyl, C₅-C₁₂cycloalkyl, C₂-C₂₄alkenyl, C₆-C₂₄aryl, C₇-C₂₅aralkyl, or A₅-A₁₈heteroaryl,
and with a compound of the formula (IXLa) 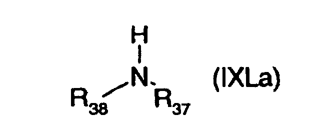 or
reacting benzofuran-2-ones (XXXa) and (XXXb), with a formylating reagent of the formula (XXXVIII)
and with a compound of the formula (IXLb)

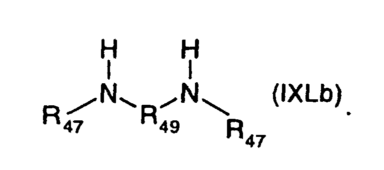

The compounds of the formula (la) or (Ic) are prepared in close analogy to a process descrybed by O.S. Wolfbeis, H. Junek, in Z. Naturforsch. 34b, 283-289, 1979 by one-pot coupling reaction of three compounds, a methylene-active compound with a formylating reagent and an amine.

The other process parameters of the preparation correspond to those above for the preparation of the compound (Ia) or (Ic) from benzofuran-2-one (XXXa) or (XXXa) and (XXXb) with a compound of the formula (XXXI a or b), (XXXII a or b), (XXXIII a or b), (XXXIV a or b), (XXXV a or b) or (XXXVII a or b).

Of course, many benzofuran-2-ones of the invention can also be prepared from other benzofuran-2-ones of the invention by chemically modifying their substituents as functional groups without altering the benzofuran-2-one parent structure. The person skilled in the art knows countless methods with which substituents can be converted into other substituents, examples being those methods disclosed in the series "Compendium of Organic Synthetic Methods" (Wiley & Sons, New York, from 1971). Owing to the known reactivity of benzofuran-2-one, judicious reaction conditions are those under which it is not anticipated that its lactone bonds will be cleaved or its double bond reduced. Depending on the nature of their substituents, the compounds of the formulae (la) or (Ic) can be used to prepare novel benzofuran-2-ones of the formulae (Ia) or (Ic). For example, novel ester derivatives or amide derivatives can be prepared in accordance with conventional synthesis methods for preparing esters or amides, as is described, for example, in Organic Syntheses, Collective Vol. I-VII. Preference is given in particular to esters prepared by transesterifying or esterifying compounds of the formula/formulae (Ia) or (Ic) using, for example, various alcohols under conventional synthesis conditions and catalysis conditions, such as, for example, at temperatures from 0°C to 200°C, with alcohol amounts of from 1 to 200 equivalents per equivalent of the compound of the formulae (la) or (Ic), in the absence or presence of a solvent.

The present invention further provides a composition of matter comprising a high molecular weight organic material a compound of the formula (la) in which
X₁ is X₁₀, where X₁₀ is a substituted or unsubstituted hydrazone or imine radical, or is a methylene radical 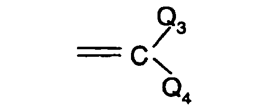 in which
Q₃ is a substituted or unsubstituted primary or secondary amine radical and Q₄ is hydrogen or a substituted or unsubstituted C₁-C₂₄alkyl, -CO-(C₁-C₂₄alkyl), -CO-O-(C₁-C₂₄alkyl), C₁-C₂₄alkoxy, C₁-C₂₄alkylthio, C₅-C₁₂cycloalkyl, C₅-C₁₂cycloalkoxy, C₅-C₁₂cycloalkylthio, C₂-C₂₄ alkenyl, C₆-C₂₄aryl, -CO-O-(C₆-C₂₄aryl), -CO-(C₆-C₂₄aryl), C₆-C₂₄aryloxy, C₆-C₁₂arylthio, C₇-C₂₅aralkyl, A₅-A₁₈heteroaryl, A₅-A₁₈heteroaryloxy or A₅-A₁₈heteroarylthio,
or
Q₃ and Q₄ together are a lactam, quinomethylene, hydantoin, acenaphthenequinone, azlactone, pyrazolonyl, barbituric acid, isoindolinone or isoindoline radical, with the proviso that
Q₄ is not hydrogen if R₃ is hydrogen, methoxy or hydroxyl and R₁, R₂ and R₄ are hydrogen,
or a composition as defined below, in a colouringly effective amount.

Dimeric benzofuran-2-ones are known in part from WO 92/08703, as is their use as antioxidants.

The present invention further provides compositions consisting of from 2 to 10, preferably 2 or 3, compounds of the formulae (la) and/or (Ic) and/or (XLIa) and/or (XLIb) and/or dimeric benzofuran-2-ones of the formula (XLIIb)
or 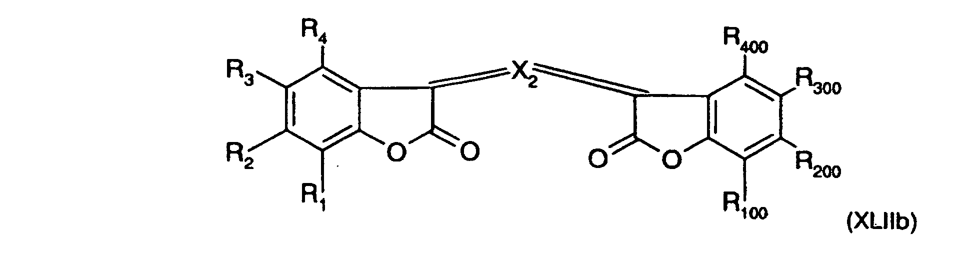 in which
X₂ is (C₆-C₂₄)arylene, (A₅-A₁₈)heteroarylene or a divalent polymethylidene, polyether, polyimine, polyamine radical, or bi(C₆-C₂₄)arylene or bi(A₅-A₁₈)heteroarylene, the bi(C₆-C₂₄)arylene or bi(A₅-A₁₈)heteroarylene radical being attached directly or via a substituted or unsubstituted carbon, nitrogen, oxygen or (-N=N-)-diradical, with the proviso that if R₂, R₄, R₂₀₀, R₄₀₀ are hydrogen and R₁, R₃, R₁₀₀ and R₃₀₀ are tert-butyl or hydrogen or R₃, R₄, R₃₀₀, R₄₀₀ are hydrogen and R₁ and R₂, R₁₀₀ and R₂₀₀ together are divalent, unsubstituted 1,3-butadien-1,4-ylene radicals, forming an additional fused 6-membered ring, X₂ is not CH-(C₆H₄)-CH.

The molar ratio of the composition consisting of two compounds of the formulae (la) (Ic), (XLIIb), (XLIa) and/or (XLIb) is usually within the range from 99:1 to 1:99.

The compositions containing 2 to 10 compounds can be prepared from the individual compounds by methods of mixing known per se.

One preferred embodiment of the present invention comprises a process for preparing compositions by reacting from 2 to 10, with particular preference 2 to 3, different benzofuran-2-ones (XXXa) with a compound of the formulae (XXXIa), (XXXIIa), (XXXIIIa), (XXXIVa) or (XXXVa),
or
by reacting from 2 to 10, with particular preference 2 to 3, different 3-oxobenzofuran-2-ones (XXXVIa) with a compound of the formula (XXXVIIa).

The compounds of the formulae (la), (Ic), (XLIIb), (XLIa) or (XLIb) are judiciously used in an amount of from 0.01 to 70% by weight, usually from 0.01 to 30% by weight, preferably from 0.01 to 10% by weight, based on the high molecular weight organic material to be coloured.

In a further embodiment of the process of the invention it is also possible if desired to admix two or more compounds, preferably from 2 to 10 and, with particular preference, 2 or 3 compounds of the formulae (Ia), (Ic), (XLIIb), (XLIa) or (XLIb) to the high molecular weight organic or inorganic material.

The invention therefore further provides a composition of matter comprising a high molecular weight organic material and at least one compound of the formulae (Ia), (Ic), (XLIIb), (XLIa) or (XLIb) or a composition consisting of compounds of the formulae (la), (Ic), (XLIIb), (XLIa) or (XLIb) in an amount effective for colouring, generally in the range from 0.01 to 70% by weight, in particular from 0.01 to 30% by weight, preferably from 0.01 to 10% by weight, based on the high molecular weight organic material.

Further, the present invention provides for the individual use of the compounds of the formulae (Ia), (Ic), (XLIIb), (XLIa) or (XLIb) as colorants, especially for colouring or pigmenting high or low molecular weight organic or inorganic material, especially the use of the compounds of the formulae (la), (Ic), (XLIIb), (XLIa) or (XLIb) and of the compositions of the invention, and also the compositions of matter, for preparing inks or colorants for coating materials, printing inks, mineral oils, lubricating greases or waxes, or dyed or pigmented plastics, non-impact-printing material, colour filters, cosmetics, toners.

It is, however, likewise possible to use the compositions of the invention comprising compounds of the formulae (la), (Ic), (XLIIIb), (XLIa) or (XLIb). Compounds of the formulae (la), (Ic), (XLIIb), (XLIa) or (XLIb) can also be combined with colorants from a different chemical class, for example with dyes or pigments as selected, for example, from the group consisting of the diketopyrrolopyrroles, quinacridones, perylenes, dioxazines, perinones, coumarins, anthraquinones, indanthrones, flavanthrones, indigos, thioindigos, quinophthalones, isoindolinones, isoindolines, phthalocyanines, metal complexes, azo pigments and azo dyes.

Depending on the nature of their substituents and of the polymer to be coloured, compounds of the formulae (la), (Ic), (XLIIb), (XLIa) or (XLIb) can be used as polymer-soluble dyes or as pigments. In the latter case it is advantageous to convert the as-synthesized products into a finely disperse form. This can be accomplished in a conventional manner. Depending on the compound and intended use, it is found advantageous to use the colorants as toners or in the form of preparations.

The high molecular weight material can be organic or inorganic and can comprise synthetic substances and/or natural substances. They can be, for example, natural resins or dry oils, rubber or caseine, or modified natural substances such as chloro rubber, oil-modified alkyd resins, viscose, or cellulose ethers or cellulose esters such as ethyl cellulose, cellulose acetate, cellulose propionate or cellulose butyrate, cellulose acetobutyrate and also nitrocellulose, but especially wholly synthetic organic polymers (thermosets and thermoplastics) as are obtainable by addition polymerization, for example by polycondensation or polyaddition. The class of the polymers includes, for example, polyolefins such as polyethylene, polypropylene, polyisobutylene, and also substituted polyolefins such as addition polymers of monomers such as vinyl chloride, vinyl acetate, styrene, acrylonitrile, acrylates, methacryaltes, fluoropolymers such as potyfluoroethylene, polytrifluorochloroethylene or tetrafluoroethylene-hexafluoropropylene copolymer, and also addition copolymers of the abovementioned monomers, especially ABS (acrylonitrile/butadiene/styrene) or EVA (ethylene/vinyl acetate). From the series of the polyaddition resins and polycondensation resins it is possible, for example, to use condensates of formaldehyde with phenols, known as phenolic resins, and condensates of formaldehyde and urea or thiourea, and also melamine, known as amino resins, and also the polyesters used as surface-coating resins, either saturated polyesters such as alkyd resins or unsaturated polyesters such as maleic resins, and also linear polyesters, polyamides, polyurethanes, polycarbonates, polyphenylene oxides or silicones, silicone resins.

The abovementioned high molecular weight compounds can be present individually or in mixtures as plastic compounds, as melts, or in the form of spinning solutions. They can also be present in the form of their monomers or in the polymerized state in dissolved form as film-formers or binders for coating materials or printing inks, such as, for example, linseed oil varnish, nitrocellulose, alkyd resins, melamine resins and urea-formaldehyde resins or acrylic resins.

The present invention therefore further provides for the use of the compositions of the invention comprising compounds of the formulae (la), (Ic), (XLIIb), (XLIa) or (XLIb) for preparing
inks, for printing inks in printing processes, for flexographic printing, screen printing, packaging printing, security ink printing, gravure printing or offset printing, for pre-press stages and for textile printing, for office applications, domestic applications or graphics applications, such as for paper goods, for ballpoint pens, felt tips, fibre tips, card, wood, (wood)stains, metal, inking pads or inks for impact printing processes (with impact-pressure ink ribbon), for the preparation of
colorants for coating materials, for industrial or commercial use, for textile decoration and industrial marking, for roller coatings or powder coatings or for automotive finishes, for high-solids (low-solvent), waterbome or metallic coating materials or for pigmented formulations for aqueous paints, for mineral oils, lubricating greases or waxes, for the preparation of
coloured plastics for coatings, fibres, platters or mould carriers, for the preparation of
non-impact-printing material for digital printing processes, for the thermal wax transfer printing process, the ink-jet printing process or for the thermal transfer printing process, and also for the preparation of
polymeric ink particles, toners, dry copy toners, liquid copy toners or electrophotographic toners.

The present invention additionally provides inks comprising high molecular weight organic material and a colouringly effective amount of the compound of the formulae (Ia), (Ic), (XLIIb), (XLIa) or (XLIb) or of the composition consisting of compounds of the formulae (Ia), (Ic), (XLIIb), (XLIa) or (XLIb).

Processes for preparing inks in particular for ink-jet printing are common knowledge.

The inks can be prepared, for example, by mixing the compounds of the invention with polymeric dispersants.

The mixing of the compounds of the invention with the polymeric dispersant takes place preferably in accordance with conventional methods of mixing, such as stirring or mechanical mixing; preferably it is advisable to use intensive mixers such as an Ultra-Turrax.

When mixing the compounds of the invention with polymeric dispersants it is judicious to use a water-dilutable organic solvent.

The weight ratio of the compounds of the invention to the ink is judiciously chosen in the range from 0.0001 to 75% by weight, preferably from 0.001 to 50% by weight, based on the overall weight of the ink.

The present invention therefore also provides a process for preparing inks by mixing with one another high molecular weight organic material and a colouringly effective amount of the compounds of the formulae (la), (Ic), (XLIIb), (XLIa) or (XLIb) or the compositions comprising compounds of the formulae (la), (Ic), (XLIIb), (XLIa) or (XLIb).

Further, the present invention provides colorants comprising high molecular weight organic material and compounds of the formula (I) and/or (XLI) and/or compounds of the formula (XLII) of the invention, or a composition of the invention, in a colouringly effective amount.

The present invention provides, moreover, a process for preparing colorant by mixing a high molecular weight organic material and a colouringly effective amount of the compounds of the formulae (Ia), (Ic), (XLIIb), (XLIa) or (XLIb) of the invention or compositions of the invention comprising compounds of the formulae (la), (Ic), (XLIIb), (XLIa) or (XLIb).

In addition, the present invention provides coloured plastics or polymeric ink particles comprising high molecular weight organic material and compounds of the formulae (Ia), (Ic), (XLIIb), (XLIa) or (XLIb), or compositions comprising compounds of the formulae (Ia), (Ic), (XLIIb), (XLIa) or (XLIb), in a colouringly effective amount.

Furthermore, the present invention provides a process for preparing coloured plastics or polymeric ink particles by mixing with one another a high molecular weight organic material and a colouringly effective amount of the compounds of the formulae (la), (Ic), (XLIIb), (XLIa) or (XLIb) or compositions comprising compounds of the formulae (la), (Ic), (XLIIb), (XLIa) or (XLIb).

The colouring of the high molecular weight organic substances with the colorants of the compounds of the formulae (la), (Ic), (XLIIb), (XLIa) or (XLIb), or with the compositions comprising compounds of the formulae (la), (Ic), (XLIIb), (XLIa) or (XLIb), takes place, for example, by mixing such a colorant in the form, if desired, of masterbatches into these substrates using roll mills, mixing or grinding apparatus to dissolve or finely disperse the colorant in the high molecular weight material. The high molecular weight material with the admixed colorant is subsequently processed by organic methods known per se, such as, for example, by calendering, compression, extrusion, coating, spinning, casting or injection moulding, whereby the coloured material acquires its ultimate shape. The admixing of the colorant can also be carried out directly prior to the actual processing step, for example by continuously metering in a pulverulent colorant of the invention and a granulated, high molecular weight organic material, and also, if desired, additional substances such as additives, simultaneously and directly into the inlet zone of an extruder where the mixing-in takes place just prior to processing. In general, however, prior mixing of the colorant into the high molecular weight organic material is preferable, since more uniform results can be obtained.

It is frequently desired to incorporate plasticizers into the high molecular weight compounds, prior to shaping in order to produce non-rigid mouldings or to reduce their brittleness. Examples of useful plasticizers are esters of phosphoric acid, phthalic acid or sebacic acid. In the process of the invention, the plasticizers can be incorporated into the polymers before or after the colorant has been incorporated. It is further possible, for the purpose of achieving different hues, to add to the high molecular weight organic materials not only the compounds of the formulae (Ia), (Ic), (XLIIb), (XLIa) or (XLIb), or the compositions of the invention, constituents such as white, colour or black pigments in any desired amounts.

To colour coating materials and printing inks, the high molecular weight organic materials and the compounds of the formulae (Ia), (Ic), (XLIIb), (XLIa) or (XLIb), or compositions of the invention, together if desired with additives such as fillers, dyes, pigments, siccatives or plasticizers, are finely dispersed or dissolved in a common organic solvent or solvent mixture. This can be accomplished by dispersing or dissolving the individual components by themselves or else more than one together and only then to combine all the components. Processing is effected by customary methods, for example by spraying, film-drawing or one of the many printing methods, after which the coating material or printing ink, after drying beforehand if desired, is subjected judiciously to thermal or radiative curing.

Where the high molecular weight material to be coloured is a coating material, it can be a standard paint or else a speciality paint, for example an automotive paint, preferably a metallic effect coating containing, for example, metal particles or mica particles.

Preference is given to the coloration of thermoplastics, including in particular those in the form of fibres, and of printing inks. Preferred high molecular weight organic materials which can be coloured in accordance with the invention are, very generally, polymers having a dielectric constant ≥ 2.5, especially polyesters, polycarbonate (PC), polystyrene (PS), polymethyl methacrylate (PMMA), polyamide, polyethylene, polypropylene, styrene/acrylonitrile (SAN) or acrylonitrile/butadiene/styrene (ABS). Particular preference is given to polyesters, polycarbonate, polystyrene, ABS and PMMA. Very particular preference is given to polyesters, ABS, polycarbonate or PMMA, especially aromatic polyesters obtainable by polycondensation of terephthalic acid, such as polyethylene terephthalate (PET) or polybutylene terephthalate (PBTP), for example.

Particular preference, furthermore, is given to the colouring of low molecular weight organic material such as mineral oils, lubricating greases and waxes using the compounds of the invention.

Moreover, the present invention provides non-impact-printing material comprising high molecular weight organic material and a compound of the formulae (Ia), (Ic), (XLIIb), (XLIa) or (XLIb), or compositions comprising compounds of the formulae (la), (Ic), (XLIIb), (XLIa) or (XLIb), in a colouringly effective amount.

Furthermore, the present invention provides a process for preparing non-impact-printing material by mixing with one another a high molecular weight organic material and a colouringly effective amount of the compounds of the formulae (la), (Ic), (XLIIb), (XLIa) or (XLIb), or compositions comprising compounds of the formulae (Ia), (Ic), (XLIIb), (XLIa) or (XLIb).

Furthermore, the present invention provides toners comprising high molecular weight organic material and a compound of the formulae (Ia), (Ic), (XLIIb), (XLIa) or (XLIb), or compositions comprising compounds of the formulae (Ia), (Ic), (XLIIb), (XLIa) or (XLIb), in a colouringly effective amount.

Moreover, the present invention provides a process for preparing toners by mixing with one another a high molecular weight organic material and a colouringly effective amount of the compounds of the formulae (la), (Ic), (XLIIb), (XLIa) or (XLIb), or compositions comprising compounds of the formulae (Ia), (Ic), (XLIIb), (XLIa) or (XLIb).

In one particular embodiment of the process of the invention, toners, coating materials, inks or coloured plastics are prepared by processing masterbatches of toners, coating materials, inks or coloured plastics in roll mills, mixing or grinding apparatus.

In the present invention, a colouringly effective amount of the compounds of the formulae (la), (Ic), (XLIIb), (XLIa) or (XLIb) or compositions comprising compounds of the formulae (la), (Ic), (XLIIb), (XLIa) or (XLIb) is generally from 0.0001 to 99.99% by weight, preferably from 0.001 to 50% by weight and, with particular preference, from 0.01 to 50% by weight, based on the overall weight of the material coloured or pigmented with them.

When the compounds of the formulae (la), (Ic), (XLIIb), (XUa) or (XLIb), or the compositions of the invention, are present dissolved in the polymers employed, they are notable for a clean hue, high colour strength, good light, heat and weather fastness, especially in PET, PMMA, PS and PC, and, if appropriate, for in some cases high fluorescence. The colorations obtained, for example, in thermoplastics or thermosets, fibres, coating materials or printing inks are notable for a clean hue, high colour strength, high saturation, high transparency, good fastness to overcoating, migration, rubbing, light, weathering and especially heat, and good gloss. The colorants possess good dispersibility and generally good solubilities in organic solvents. In mixtures containing the compounds of the invention, beautiful colour shades are obtained.

The compositions of the invention may exhibit advantages in terms of applications properties over individual compounds.

The examples which follow illustrate the invention without in any way restricting it:

The preparations of 5,7-di-tert-butyl-3H.-benzofuran-2-one, 5-methoxy-7-tert-butyl-3H-benzofuran-2-one and methyl 3-tert-butyl-3H-benzofuran-2-on-5-yl)propionate take place in analogy to H.-D. Becker, K. Gustafsson: J. Org. Chem. 42, 2966 (1977).

Example 1a: A 15 I multi-necked vessel with stirrer, dropping funnel, water separator, condenser and thermometer is charged with stirring and in succession with 300 ml of toluene, 212 g of 97% 2,4-di-tert-butylphenol (Aldrich 99%), 121.9 ml of 50% strength aqueous glyoxylic acid and 0.5 g of p-toluenesulfonic acid monohydrate. The reaction mixture is subsequently refluxed vigorously with thorough stirring. This is accompanied by the separation of the water present in the glyoxylic acid, and the water of reaction from the first stage. After a reflux time of about 3 h, the separation of water ends, to leave a homogeneous, pale yellow solution of 5,7-di-t-butyl-3-hydroxybenzofuran-2-one. Thereafter, the toluene is distilled off under atmospheric pressure and with a heating-bath temperature of up to 142°C. The crystalline solid is subsequently dried at 80°C/50 mbar.

Example 1b: 5,7-Di-tert-butyl-3-hydroxybenzofuran-2-one, 30 g (114 mmol), prepared in accordance with Example 1a, is dissolved in 200 ml of dimethyl sulfoxide. 54 ml (574 mmol) of acetic anhydride is added to the solution over 2 minutes with vigorous stirring, and stirring is continued for 15 hours at from 25 to 27°C. With stirring, the reaction mixture is poured into 2 litres of water and stirred for 2 hours. The precipitate is filtered off with suction and washed with 1 litre of water, then sodium chloride solution (25% by weight) and then with 1 litre of water. The precipitate is subsequently dried at 80°C/15 mbar. This gives 31.10 g of orange crystals of 5,7-di-tert-butyl-3-oxobenzofuran-2-one.

### Example 1c: Synthesis of 3-oxo-furan-2-one derivatives via oxallyl chloride, general instruction

0.5 mol of the hydroxyaromatic compound is dissolved in 200 ml of dichloromethane and that solution is then added over 2 hours to a solution of 0.55 mol of oxallyl chloride in 200 ml of dichloromethane. The solution is heated to 35°C, then 3 portions each of 0.5 g of aluminium chloride (Fluka) are added and the mixture is stirred for 17 hours. After cooling, the mixture is filtered with suction to isolate the residue, which is washed with 3 times 50 ml of hexane. A second fraction of the product can be obtained by concentrating the filtrates and then washing the residues with hexane. For purification, the product can be recrystallized from glacial acetic acid or used directly. Additional purification can be achieved by reaction with sodium hydrogen sulfide, recrystallization from water, and subsequent acidic hydrolysis of the adduct using mineral acids.

| Product | Yield | Properties |
|---|---|---|
| 5,7-Di-tert-butyl-3-oxobenzofuran-2-one | 65% | yellow crystals |
| 3-Oxo-2-naphthofuranone | 76% | orange crystals |
| 5-t-Butyl-7-methoxy-3-oxo-benzofuranone | 46% | orange crystals |
| 3-Oxo-1-naphthofuranone | 73% | orange crystals |
| 5-Hydroxy-3-oxo-β-naphthofuranone | 94% | orange crystals |
| 6-Hydroxy-3-oxobenzofuranone | 17% | yellow crystals |

Example 2: 5,7-Di-tert-butyl-3-oxobenzofuran-2-one, 4 g (15.4 mmol), prepared as in Example 1b, is dissolved together with barbituric acid, 2.14 g, (Fluka) in 100 ml of acetic acid and the solution is boiled under reflux for 17 hours. The solvent is subsequently distilled off with 60 mbar/60°C and then 250 ml of methanol are added to the residue. A bright yellow product is precipitated which is filtered off with suction and dried in vacuo at 50 mbar/40°C. This gives 0.17 g of a compound of the formula (XLIV)

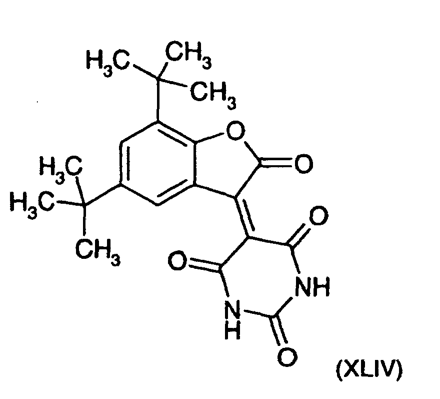

Example 3: 5,7-Di-t-butyl-3-oxobenzofuran-2-one, 4 g (15.4 mmol), prepared in accordance with Example 1b, and 2-methoxy-4-nitroaniline, 2.56 g (15.4 mmol) (Fluka) are dissolved in 100 ml of toluene. 50 mg of para-toluenesulfonic acid (Fluka) are added as catalyst to this solution, which is then boiled under reflux for 6 hours. The solvent is subsequently evaporated off under 60°C/75 mbar vacuum and the residue is recrystallized from 100 ml of methanol. This gives 2.08 g of dark yellow crystals of the formula (XLV)

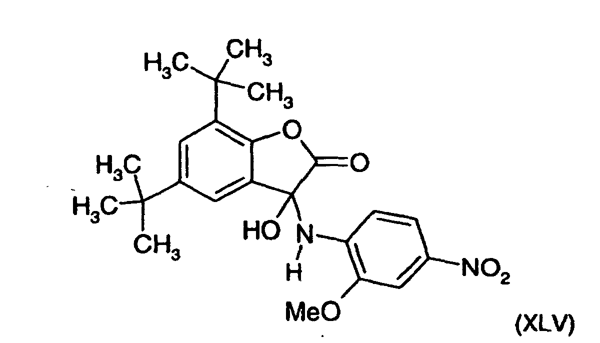

Example 4-6: 5,7-Di-tert-butyl-3-oxobenzofuran-2-one, 4 g (15.4 mmol), prepared in accordance with Example 1b, and 2-methoxy-4-nitroaniline, 2.56 g (15.4 mmol) (Fluka), are dissolved in 100 ml of cyclohexane. 4 g of a silicate is added as catalyst (see Table 1) to this solution, which is subsequently stirred at from 25 to 27°C for 24 h. The solvent is then evaporated off at 60°C under 100 mbar vacuum and the residue is washed with 100 ml of methanol and dried in vacuo (50 mbar) at 27°C. This gives 2.08 g of dark yellow crystals of the formula (IL).

**Table 1.**

| Catalyst | Yield | Example |
|---|---|---|
| Fulkat 40 from Pontecchio Marconi | 3.89 g | 4 |
| Catalyst K10 from Süd-Chemie | 3.88 g | 5 |
| Catalyst Rudex from Rudex Nebelova Bratislava | 3.57 g | 6 |

Example 7-9: Preparation as for Examples 4-6 but with the difference that silicate K10 is used as catalyst for Examples 7-9 and, instead of the 2-methoxy-4-nitroaniline, use is made in Example 7 of 5-aminobenzimidazolone (Clariant), 2.29 g (15.4 mmol), in Example 8 of 2,4-dimethoxyaniline (Aldrich), 2.36 g (15.4 mmol), under the same conditions, and
in Example 9 of 3-nitroaniline (Aldrich), 2.21 g (15.4 mmol), using xylene as solvent at 140°C.

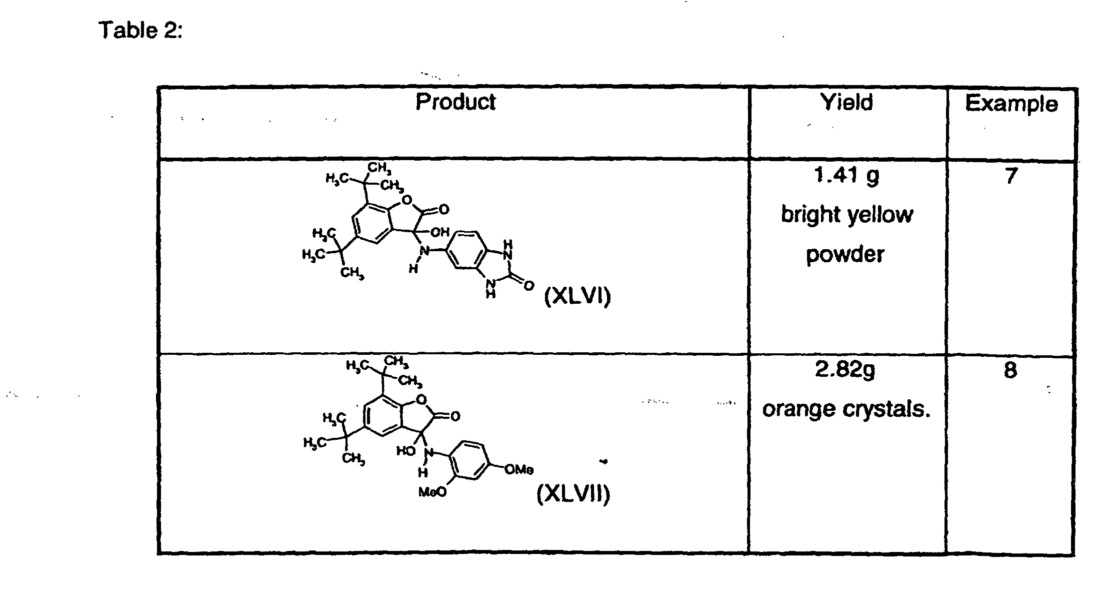

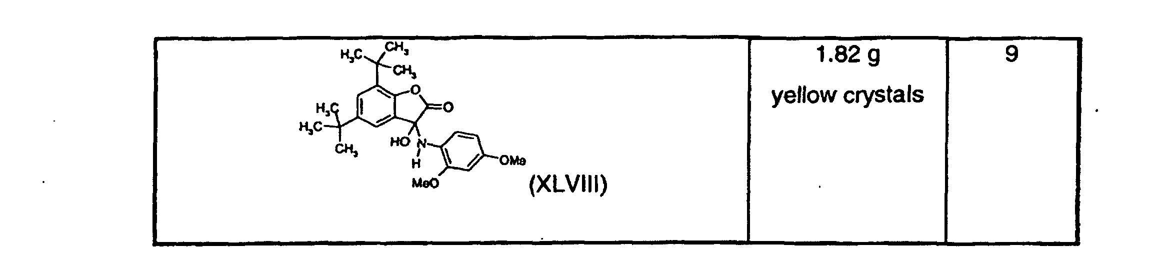

Example 13: To a boiling solution of **A**, 5,7-di-tert-butyl-3H-benzofuran-2-one, 1.5 g, prepared as in Example 1a, in 50 ml of acetic acid is added **B**, 2-cyano-N-(3,4-dichloro-phenyl)-2-(3-imino-2,3-dihydro-1 H-isoindol-1-yl)acetamide, 2.43 g, prepared in accordance with EP 657507 A2. The mixture is stirred further under reflux for 19 hours, then cooled to room temperature and subsequently filtered. The filter residue is washed with acetic acid and then with water. The moist filter residue is dried in a vacuum drying oven at 80°C. This gives 2.74 g of an orange powder of the formula (LIII), which when incorporated into PET gives an orange colour.

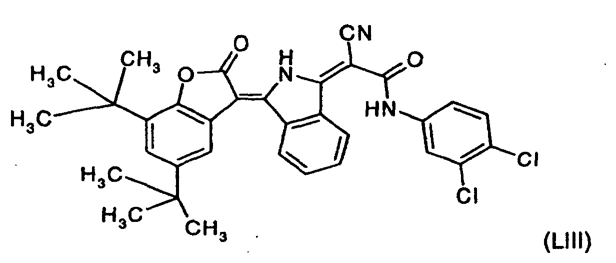

Example 14a-I: In analogy to the process in Example 13, the compounds of Table 3 are prepared:

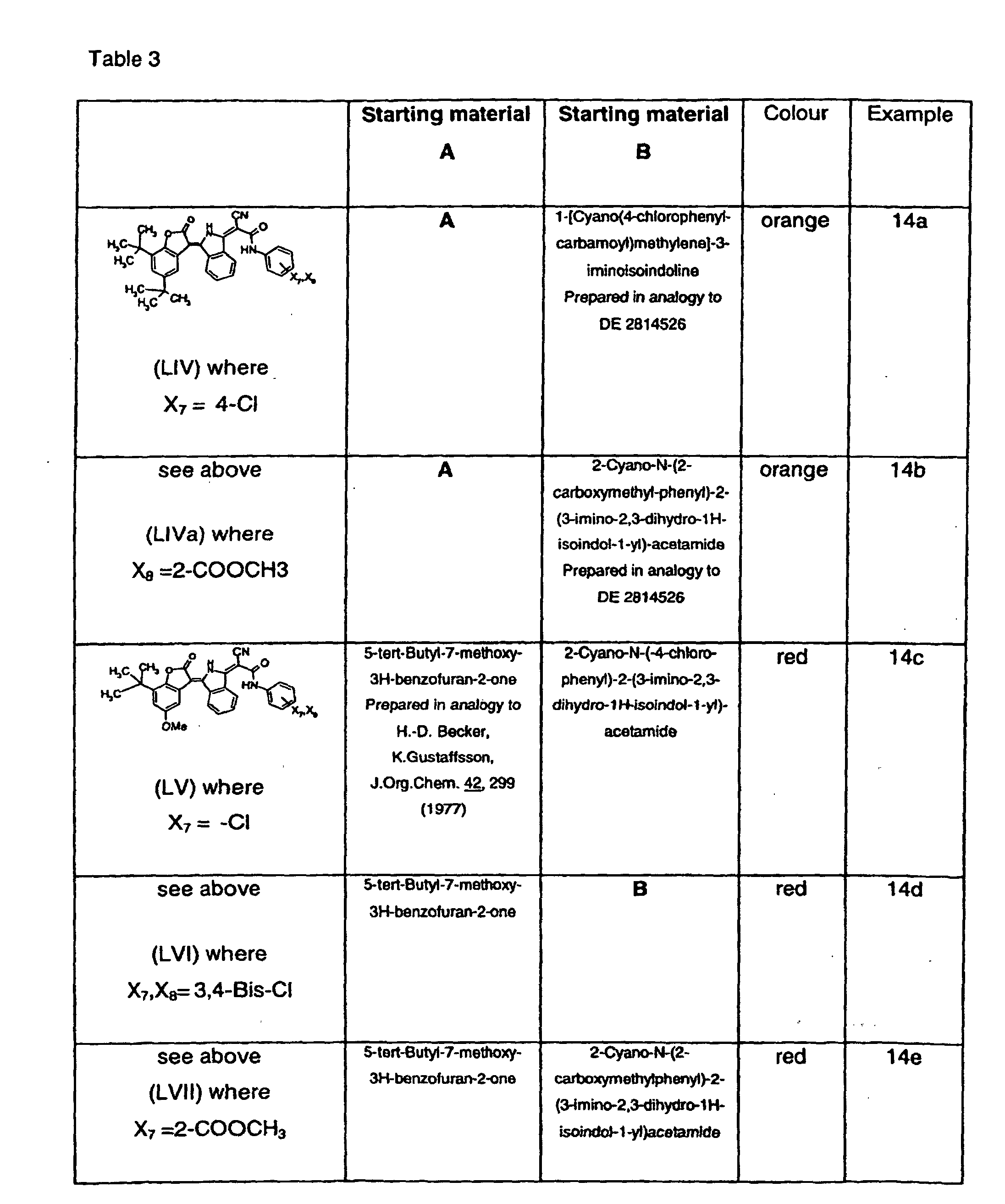

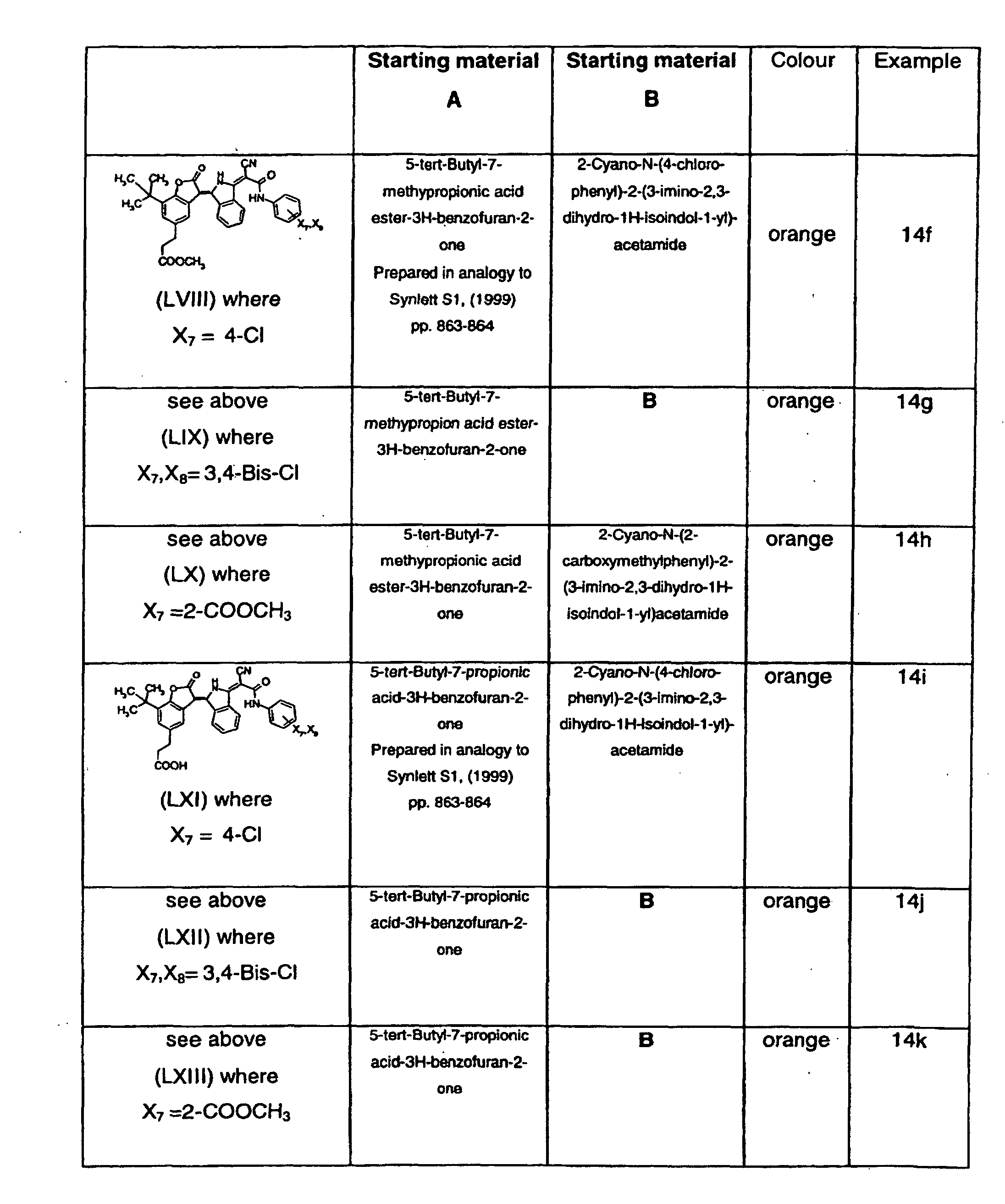

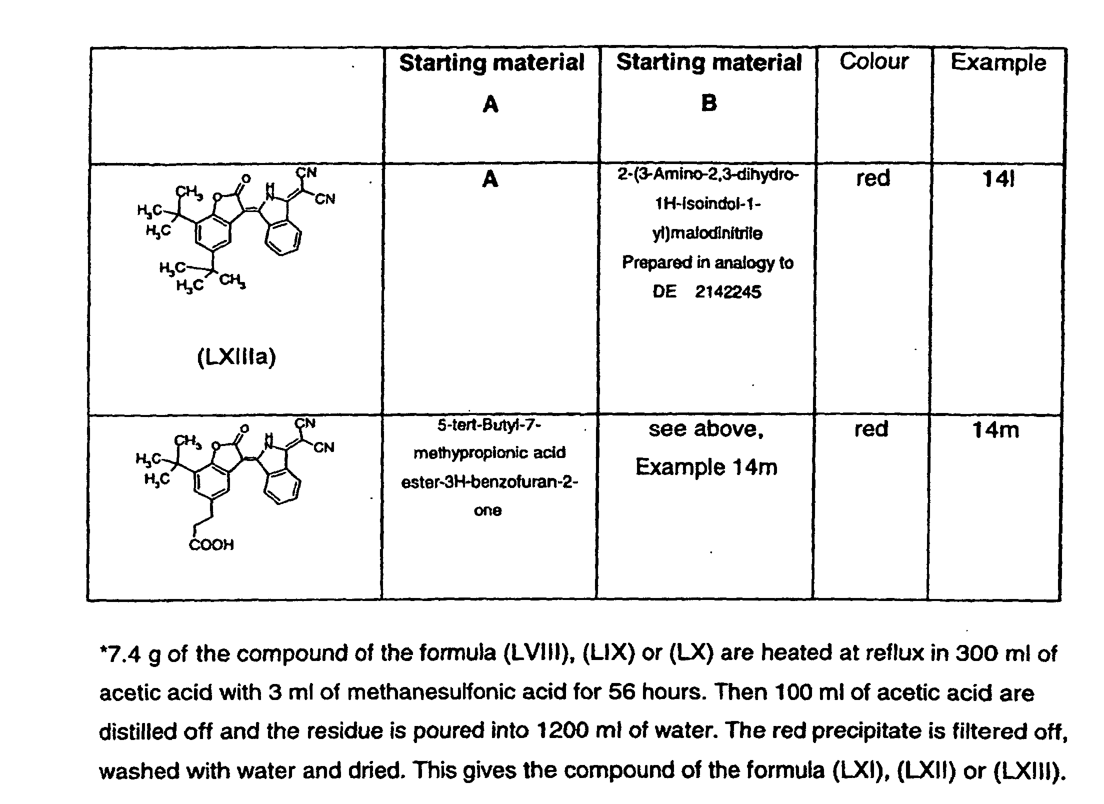

Example 16: Diiminoisoindolenine, 10g, (Fluka purum), and 6 ml of aniline (Fluka purum) in 60 ml of ethanol are stirred under reflux for 2.5 hours. The resulting mixture is cooled to 5-10°C and filtered. The filter cake is washed with 30 ml of ethanol and subsequently dried in a drying oven at 60°C and 200 mbar, to give 7.0 g of a yellow powder. The resulting yellow powder, 1.0 g, and 5,7-di-tert-butyl-3H-benzofuran-2-one, 1.2 g, in 20 ml of ethanol are heated to 65°C and then cooled to 20-25°C. The resulting mixture is then diluted with 20 ml of methanol and filtered. The filter residue is purified by column chromatography (Merck silica gel, eluent: hexane/ethyl acetate 4:1 to 1:4) and dried in a drying oven at 60°C and 200 mbar. This gives 0.9g of a red powder of the formula

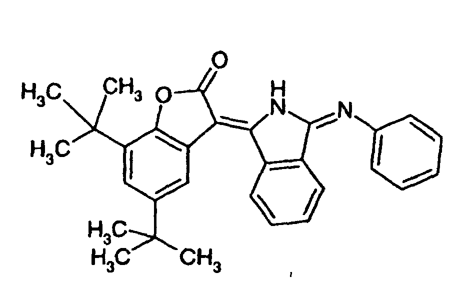

In analogy to the process in Example 16, the compound of Table 5 is prepared, aniline being replaced by 3-chloroaniline (Fluka):

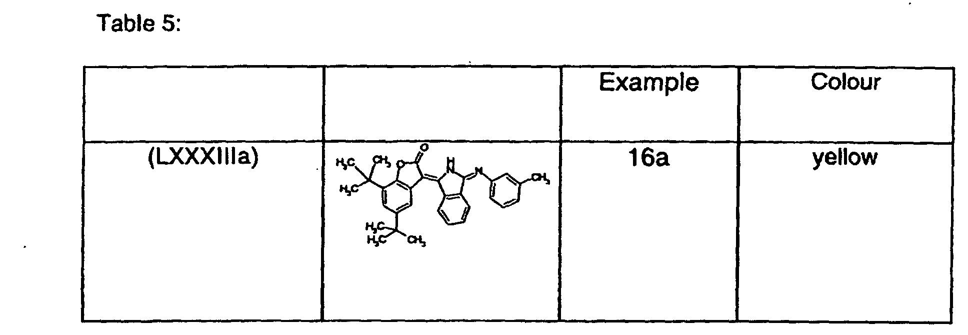

Example 17: Phthalodinitrile, 1.5 g, (Fluka purum), in 1.4 ml of a 30% sodium hydroxide solution and 30 ml of n-butanol are stirred for 2.5 hours. The pH is adjusted in the range of 6 to 7 using 37% hydrochloric acid solution, and then 5,7-di-t-butyl-3H-benzofuran-2-one, 3.1 g, is added. The mixture is stirred at 42°C for 4 hours and then cooled and filtered. The filter residue is dried in a drying oven at 60°C and 200 mbar. This gives 3.1 g of a yellow powder of the formula (LXXXIV)

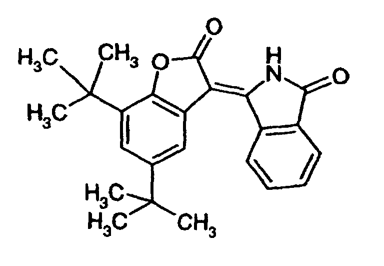

In analogy to the process in Example 17, the compounds of Table 6 are prepared, 5,7-di-t-butyl-3H-benzofuran-2-one being replaced in Example 17a by 5-t-butyl-7-methylpropionic acid ester-3H-benzofuran-2-one and in Example 17b by 5-t-butyl-7-propionic acid-3H-benzofuran-2-one and in Example 17c by 5-t-butyl-7-methoxy-3H-benzofuran-2-one:

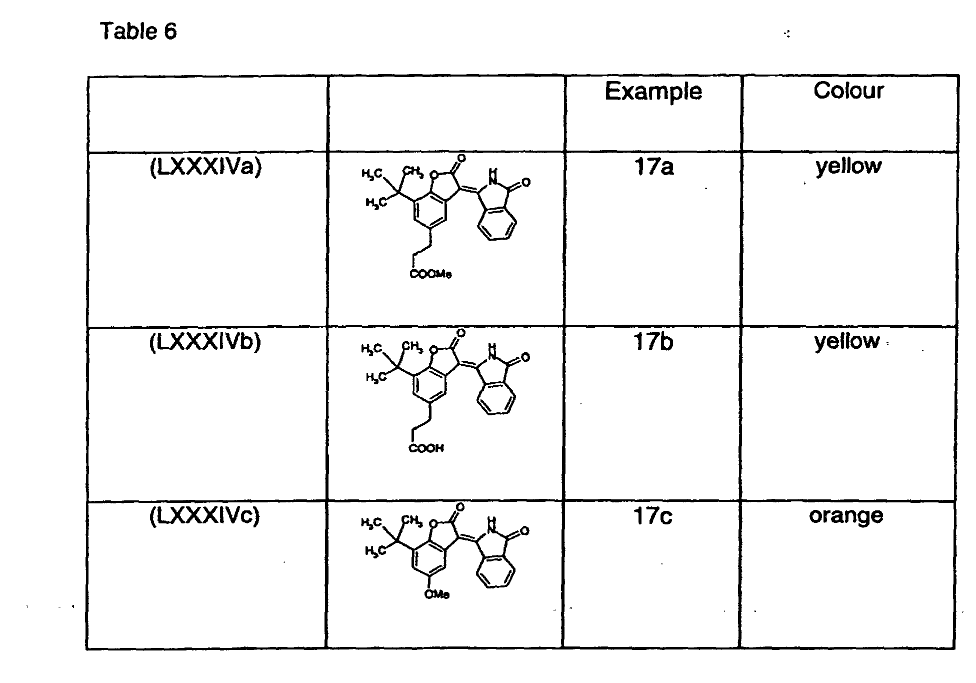

Example 18: 5,7-Di-tert-butyl-3H-benzofuran-2-one, 2.46 g, dissolved in 10 ml of acetic acid is added to 1-aminoanthraquinone (Fluka), 2.3 g, and trimethyl orthoformate (Fluka purum), 2.2 ml, dissolved in 10 ml of acetic acid. The mixture is stirred at 105°C for 2.5 hours. The resulting mixture is subsequently cooled to room temperature and filtered. The filter residue is washed with acetic acid and then with water, and subsequently dried in a drying oven at 60°C and 200 mbar. This gives 3.7 g of a red powder of the formula (LXIV)

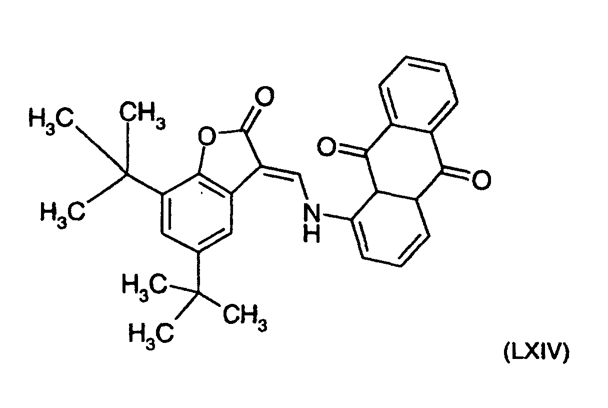

Examples 19-21a-d: In analogy to the process in Example 18, the compounds of Table 7 are prepared, 1-aminoanthraquinone being replaced in each case by the corresponding compound A:

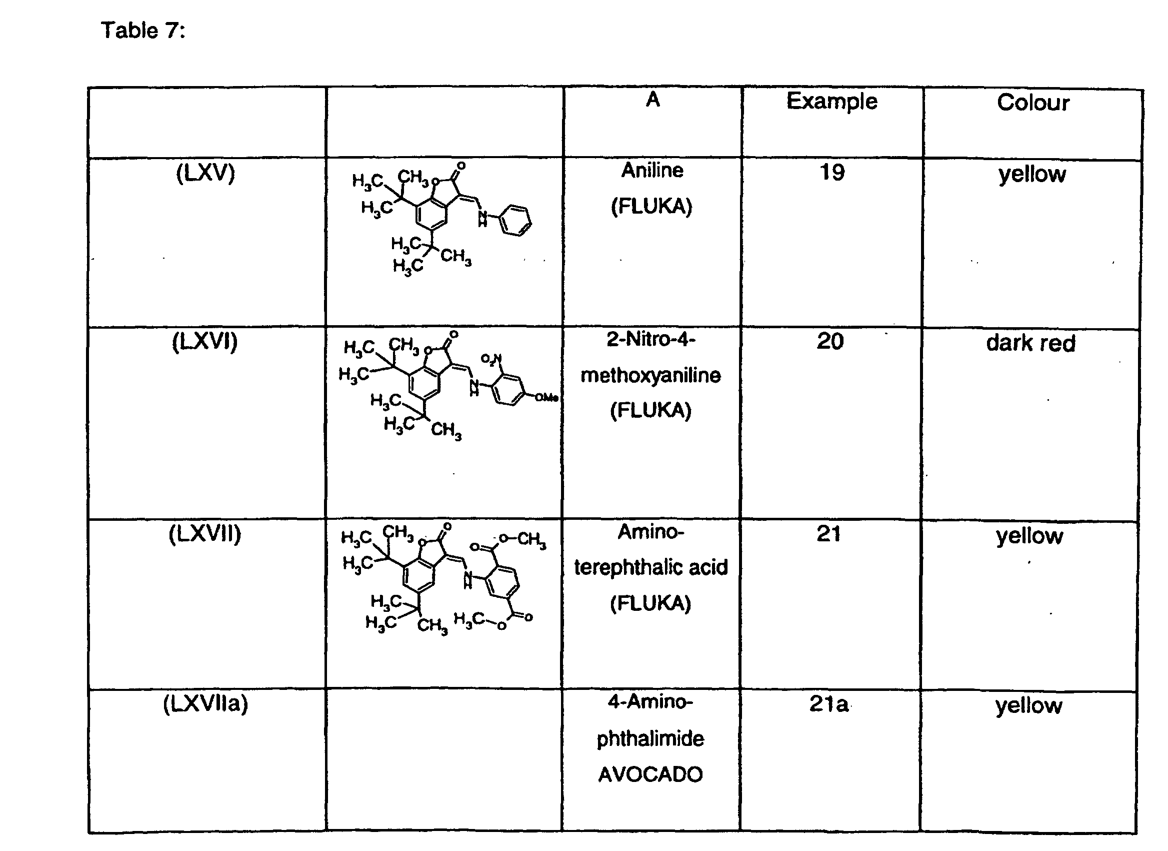

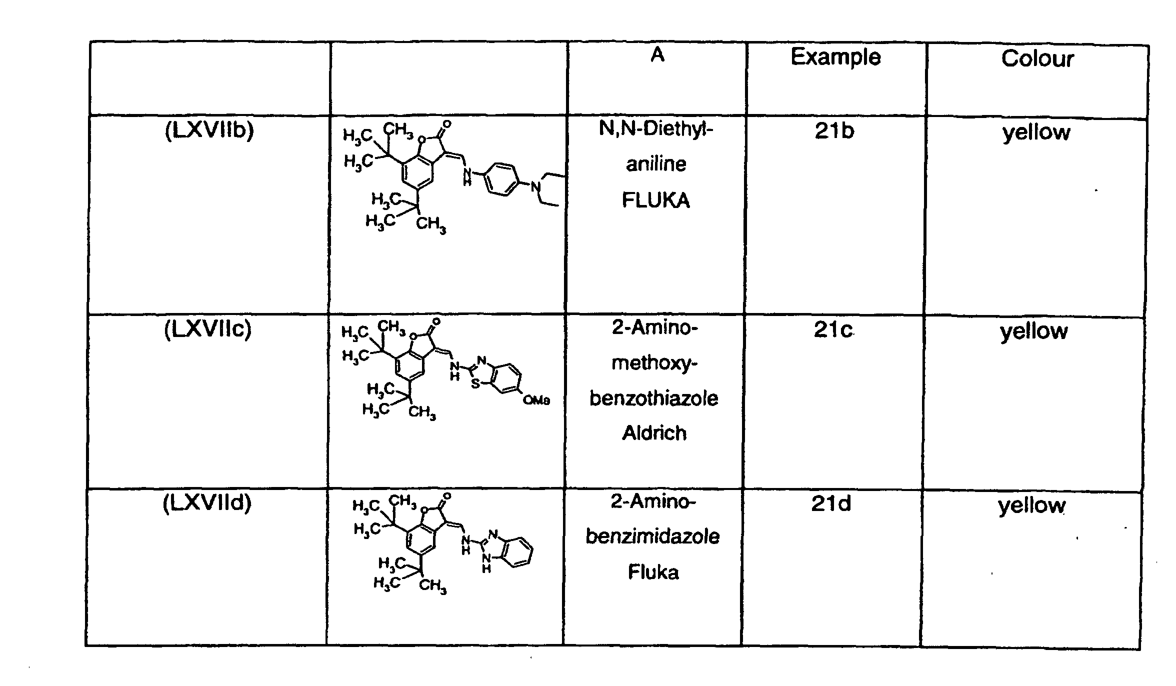

Example 21i: In analogy to the process in Example 18, the mixture of the compounds below is prepared, 5,7-di-tert-butyl-3H-benzofuran-2-one being replaced by a mixture of 5,7-di-tert-butyl-3H-benzofuran-2-one, 1.50 g, and 5-(methylpropionate)-7-tert-butyl-3H-benzofuran-2-one, 1.49 g, in a molar ratio of 1:1, and 1,4-phenylenediamine, 0.58 g (Fluka) being used instead of 1-aminoanthraquinone, the molar ratio of the mixture of 5,7-di-tert-butyl-3H-benzofuran-2-one and 5-(methylpropionate)-7-tert-butyl-3H-benzofuran-2-one to 1,4-phenylenediamine being 2:1.

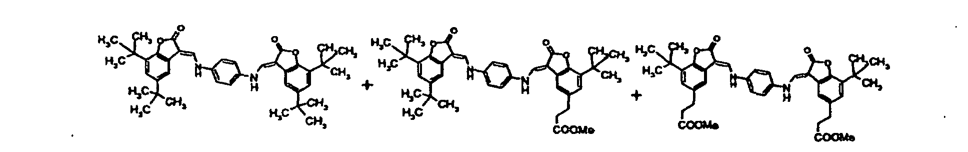

Example 21j: In analogy to the process in Example 21i, the mixture of the above compounds is prepared, first 5,7-di-tert-butyl-3H-benzofuran-2-one, 1.5 g, being reacted with trimethyl orthoformate, 1.2 ml, and then a mixture of 5-(methylpropionate)-7-tert-butyl-3H-benzofuran-2-one, 1.49 g, and trimethyl orthoformate, 1.2 ml, being added. The molar ratio of 5,7-di-tert-butyl-3H-benzofuran-2-one to 5-(methylpropionate)-7-tert-butyl-3H-benzofuran-2-one is 1:1, the molar ratio of the mixture of 5,7-di-tert-butyl-3H-benzofuran-2-one and 5-(methylpropionate)-7-tert-butyl-3H-benzofuran-2-one to 1,4-phenylenediamine being 2:1.

Example 22: A mixture of potassium carbonate. 1.78 g, phthalodinitrile (FLUKA), 1.5 g, and 4.7 ml of 7N ammonia solution in 30 ml of methanol is stirred at reflux for 12 hours. Thereafter, the resulting mixture is cooled to room temperature, and 5,7-di-t-butyl-3H-benzofuran-2-one, 2.88 g, dissolved in 4.7 ml of acetic acid is added. Subsequently, the resulting mixture is stirred at reflux for 4 hours and then cooled and subsequently filtered. The filter residue is purified by column chromatography (Merck Silica gel, eluent: hexane/ethyl acetate 4:1 to 1:4) and dried in a drying oven at 60°C and 200 mbar. This gives 3.1 g of a red powder of the formula (LXVIII)

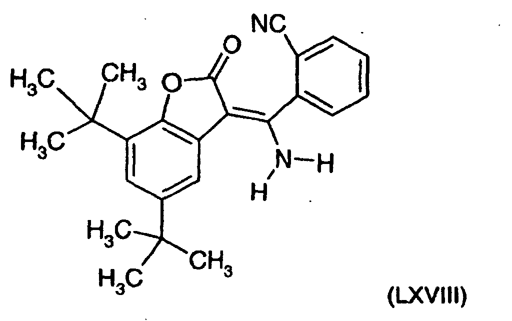

In analogy to the process in Example 22 the compounds of Table 8 are prepared, 5,7-di-t-butyl-3H-benzofuran-2-one being replaced by 5-t-butyl-7-propionic acid-3H-benzofuran-2one in Example 22a and by 5-t-butyl-7-methoxy-3H-benzofuran-2-one in Example 22b:

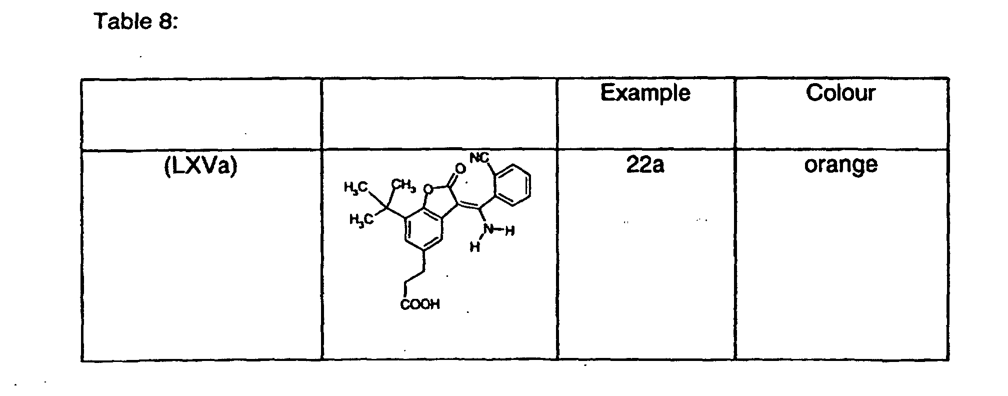

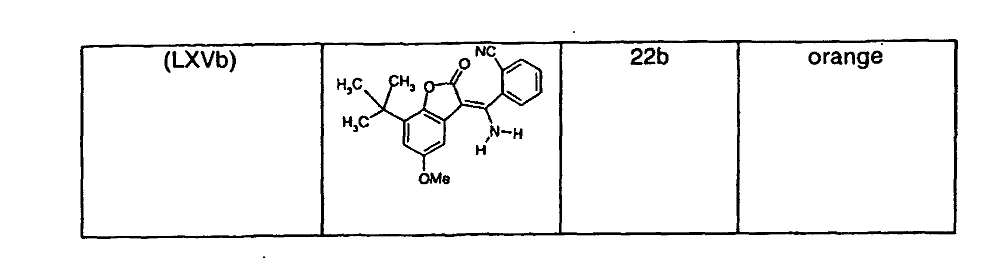

Example 23: 5-Aminobenzimidazolone (1.49 g, Aldrich) is dissolved at 40°C in 70 ml of water and 11 ml of acetic acid, and 2.7 ml of 32% hydrochloric acid are added. The solution is subsequently cooled to 5°C. Following the dropwise addition of 2.75 ml of 4N NaNO₂ solution and 0.5 hour of stirring at 5°C, the solution is filtered and then the excess nitrite is decomposed using sulfamic acid. Subsequently, 6 g of sodium acetate dissolved in 5 ml of acetic acid are added. 5,7-Di-t-butyl-3H-benzofuran-2-one, 2.34 g, dissolved in 35 ml of ethylcellosolve is added dropwise at 5°C to the resulting mixture, which is then stirred at room temperature for 3 hours and subsequently at 50°C for 2 h. Thereafter, the mixture obtained is filtered at 50°C and the filter residue is washed with water and subsequently dried at 80° in a vacuum drying oven. 3.3 g of a yellow powder (LIXX) are isolated, which when incorporated in PVC gives a yellow coloration.

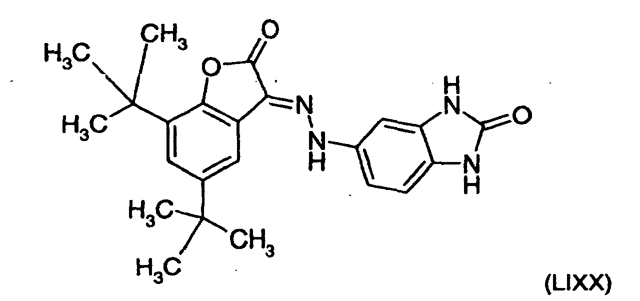

Example 25: A suspension of 5.75 g of aminoanthraquinone (Fluka purum) in 90 ml of acetic acid and 7.5 ml of concentrated hydrochloric acid (38%) is admixed dropwise at 0-5°C with 6.25 ml 4N sodium nitrite solution and then stirred at this temperature for 70 minutes. To the resultant mixture is added a solution of 6.16 g of 5,7-di-t-butyl-3H-benzofuran-2-one in 40 ml of 2-ethoxyethanol (Merck) at 5°C, followed by 30 g of sodium acetate, after which the mixture is stirred at room temperature for 3 h. The orange suspension obtained is diluted with 250 ml of water and filtered and the filter residue is washed with water and methanol. The filter residue is dried in a vacuum drying oven at 80°C. This gives 10.2 g of an orange powder of the formula (LXXI), which when incorporated into PET gives an orange colour.

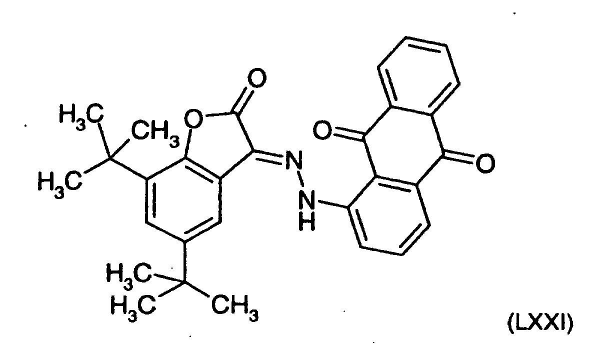

Example 26: Process analogous to Example 25, but differing in that aminoanthraquinone is replaced with 2,5-dichloroaniline (Fluka purum). This gives 0.35 g of a yellow powder of the formula (LXXII), which when incorporated into PVC gives a yellow colour.

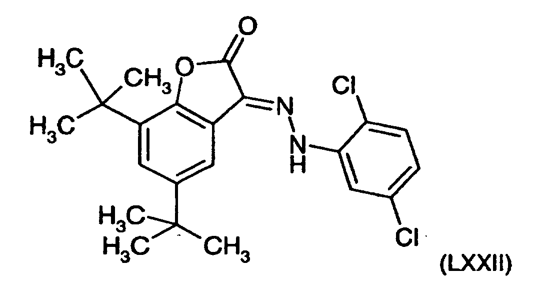

Example 26a: Process analogous to Example 25, but differing in that aminoanthraquinone is replaced with 2-nitro-4-methoxyaniline (Fluka purum). This gives 5.7 g of an orange powder of the formula (LXXIII), which when incorporated into PVC gives a yellow colour.

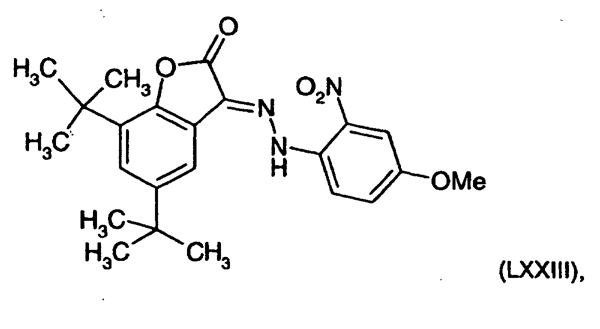

Example 27: 3-Oxofuranone, 0.01 mol, and arylhydrazine, 0.01 mol, are dissolved in 50 ml of glacial acetic acid. With hydrazines which are very slow to react, a little sulfuric acid is added. The reaction mixture is heated under reflux until the starting compounds can no longer be detected. It is then cooled to room temperature and the precipitated product is filtered off with suction. If no product, or too little product, is precipitated, the solution is concentrated and cooled to 6°C. The product is washed with methanol and dried in vacuo.

The compounds of Examples 27a-m, 27/1 a-d, 27/2a-f, 27/3a-f, 27/4a are prepared in analogy to the above instructions of Example 27, with 3-oxofuranone being replaced respectively by 3-oxo-5,7-di-t-butylbenzofuranone and 3-oxo-1-naphthofuranone and 3-oxo-2-naphthofuranone and 5-hydroxy-3-oxo-β-naphthofuranone and 6-hydroxy-3-oxo-benzofuranone, and arylhydrazine by the respective hydrazine indicated in the examples.

In Examples 27a-m, 3-oxo-5,7-di-t-butylbenzofuranone is used instead of 3-oxofuranone:

| Hydrazine (FLUKA) | m/w | λₘₐₓ | Colour |
|---|---|---|---|
| Example 27a : 2,4-Dinitrophenylhydrazine | 440 | 434 nm | yellow |
| Example 27b : 4-Methoxy-phenylhydrazine | 380 | 436 nm | yellow |
| Example 27c : Phenylhydrazine | 350 | 420 nm | yellow |
| Example 27d : 4-Dimethylaminobenzoic hydrazide | 421 | 412 nm | orange |
| Example 27e : Phenylsulfonic hydrazide | 414 | 340 nm | yellow |
| Example 27f: 2,4,6-Trichlorophenylhydrazine | 453 | 394 nm | yellow |
| Example 27g : N-Methyl-N-phenyl-hydrazine | 364 | 424 nm | yellow |
| Example 27h : 4-Chlorophenylhydrazine | 384 | 417 nm | yellow |
| Example 27i: Pyridin-4-carboxylic hydrazide | 379 | | yellow |
| Example 27i: Benzoic acid-2-hydrazine | 394 | | yellow |
| Example 27k: 1-Naphthylhydrazine | 400 | 445 nm | orange |
| Example 27l: 2-Trifluoromethylphenylhydrazine | 418 | 409 nm | yellow |
| Example 27m: N,N-Diphenylhydrazine | 426 | 414 nm | yellow |

In Examples 27/1a-d, 3-Oxo-1-naphthofuranone is used instead of 3-oxofuranone:

| Hydrazine (FLUKA) | m/w | λₘₐₓ | Colour |
|---|---|---|---|
| Example 27/1a : 2,4-Dinitrophenylhydrazine | 378 | 459 nm | orange |
| Example 27/1b : 2-Hydrazinobenzoic acid | 332 | 428 nm | yellow |
| Example 27/1c : 2,4,6-Trichlorophenylhydrazine | 391 | 420 nm | orange |
| Example 27/1d : 2-Trifluoromethylphenylhydrazine | 356 | 426 nm | yellow |

In Examples 27/2a-f, 3-oxo-2-naphthofuranone is used instead of 3-oxofuranone:

| Hydrazine (FLUKA) | m/w | λₘₐₓ | Colour |
|---|---|---|---|
| Example 27/2a : 2,4-Dinitrophenylhydrazine | 378 | 468 nm | red |
| Example 27/2b : 2-Hydrazinobenzoic acid | 332 | 445 nm | orange |
| Example 27/2c : 2,4,6-Trichlorophenylhydrazine | 391 | 424 nm | yellow |
| Example 27/2d : 2-Trifluoromethylphenylhydrazine | 356 | 432 nm | yellow |
| Example 27/2e : 4-Pyridinecarboxylic hydrazide | 317 | 411 nm | yellow |
| Example 27/2f : 1-Naphthylhydrazine | 338 | 469 nm | orange |

In Examples 27/3a-f, 5-hydroxy-3-oxo-β-naphthofuranone is used instead of 3-oxofuranone:

| Hydrazine (FLUKA) | m/w | λₘₐₓ | Colour |
|---|---|---|---|
| Example 27/3a : 2-Hydrazinobenzoic acid | 348 | | yellow |
| Example 27/3b : 2,4,6-Trichlorophenylhydrazine | 407 | 429 nm | yellow |
| Example 27/3c: 1-Naphthylhydrazine | 374 | 452 nm | orange |
| Example 27/3d: 4-Methoxyphenylhydrazine | 354 | 440 nm | orange |
| Examples 27/3f: 4-Pyridinecarboxylic hydrazide | 353 | 351 nm; 420 nm | yellow |

In Example 27/4a, 6-hydroxy-3-oxo-benzofuranone is used instead of 3-oxofuranone:

| Hydrazine | m/w | λₘₐₓ | Colour |
|---|---|---|---|
| Example 27/4 a:2-Hydrazinobenzoic acid | 298 | | yellow |

Example 28: General synthesis instructions for preparing azines, the 3-oxofuranone being replaced in Examples 28/1-3 in each case in accordance with the table below:

Hydrazine hydrate, 0.02 mol, is dissolved with 3-oxofuranone, 0.01 mol, in 20 ml of acetic acid and the solution is boiled under reflux for 5 h. If reaction is slow, 0.2 ml of conc. HCI can be added. At the end of reaction, the solution is either filtered cold or the solvent is evaporated off. The residue is washed with hexane until starting material can no longer be detected.

| | Azine | | |
|---|---|---|---|
| 3-Oxofuranone | m/w | λₘₐₓ | Colour |
| Example 28/1 3-Oxo-5,7-di-t-butyl-benzofuranone | 516 | 379 nm | orange |
| Example 28/2 3-Oxo-5-butyl-7-methoxybenzofuranone | 464 | 463 nm | red |
| Example 28/3 3-Oxo-α-naphthofuranone | 392 | 426 nm | red |

General synthesis instructions A for preparing the compounds of Examples 28/4-7,3-oxofuranone being replaced in each case in accordance with the table below:
N,N-Diacetylpiperazine-2,5-dione, 0.01 mol, is dissolved with 3-oxofuranone, 0.01 mol, in 50 ml of dimethylacetamide, triethylamine, 0.5 ml, is added, and the mixture is stirred at 40°C. The reaction mixture is cooled and added to 150 ml of 0.5M hydrochloric acid, and the precipitate is filtered off with suction, washed with water and methanol, and then dried in vacuo.

General synthesis instructions B for preparing the compounds of Examples 28/8-9, 3-oxofuranone being replaced in each case in accordance with the table below:
Piperazine-2,5-dione, 0.01 mol, is dissolved together with 3-oxofuranone, 0.01 mol, in 50 ml of acetic anhydride, 0.5 g of sodium acetate is added, and the mixture is boiled under reflux. The precipitate is filtered off with suction at room temperature and washed with methanol and the product is dried in vacuo.

With both syntheses, the product is obtained in very good yields.

| 3-Oxofuranone | Synthesis method | m/w | λₘₐₓ | Colour |
|---|---|---|---|---|
| Example 28/4: 5,7-Di-t-butyl-3-oxobenzofuranone | A | 598 | 498 nm | red |
| Example 28/5: 5,7-Di-t-butyl-3-oxobenzofuranone | B | | | |
| Example 28/6: β-Naphtho-3-oxofuranone | A | 474 | 537 nm | violet |
| Example 28/7: 5-t-Butyl-7-methoxy-3-oxobenzofuranone | A | 546 | 445 nm; 517 nm | violet |
| Example 28/8: Isatin | B | 372 | | brown-red |
| Example 28/9: 6-Hydroxy-3-oxobenzofuranone | B | 406 | | red |

Example 29: General preparation of lactams of Examples 29/1-6,3-oxofuranone being replaced in each case in accordance with the table below.: 3-Oxofuranone, 0.01 mol, is dissolved together with 4-ethoxycarbonyl-5-(4-chloro)phenylpyrrolin-2-one (A), 0.01 mol, or 4-ethoxycarbonyl-5-phenylpyrrolin-2-one (B), 0.01 mol, in 50 ml of glacial acetic acid and the solution is boiled under reflux until starting material can no longer be detected. The solvent is evaporated off in vacuo and the residue is washed with a little hexane. The filtrate is left to stand in a refrigerator overnight and then the residue is filtered off with suction and dried in vacuo.

| 3-Oxofuranone | Product | m/w | Colour. | λₘₐₓ |
|---|---|---|---|---|
| Example 29/1: 5,7-Di-t-butyl-3-oxobenzofuranone | A | 508 | claret | 517 nm |
| Example 29/2: 5,7-Di-t-butyl-3-oxobenzofuranone | B | 473 | violet | |
| Example 29/3: 5-t-Butyl-7-methoxy-3oxobenzofuranone | B | 447 | claret | |
| Example 29/4: α-Naphtho-3oxofuranone | B | 411 | blue | 546 nm |
| Example 29/5: α-Naphtho-3oxofuranone | A | 445 | brown | 546 nm |
| Example 29/6: 6-Hydroxy-3-oxobenzofuranone | A | 411 | violet | 520 nm |

Example 29/7: A solution of 5,7-di-tert-butyl-3-oxobenzofuran-2-one, 4 g (15.4 mmol), prepared in accordance with Example 1b, and 1,2-dihydro-4-(4-chlorophenyl)pyrrolone-3-carboxylic acid ethyl ester, 4.46 g (obtainable in accordance with Bull. Soc. Chem. Belg., 97, 8-9, 615, 1988) in 100 ml of acetic acid is boiled under reflux for 17 h. The solvent is subsequently distilled off at 60°C/60 mbar and the product is chromatographed with 3l of toluene over silica gel (0.025-0.064 mm). This gives 2.96 g of reddish brown powder comprising a compound of the formula (XLIII)

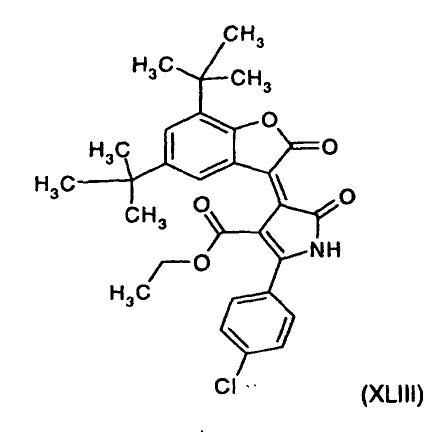

Example 30: General preparation of pyrazolinones of Examples 30/1-2, 3-oxofuranone being replaced in each case in accordance with the table below:

3-Oxo-furanon, 0.01 mol, and 1-phenyl-3-methyl-5-pyrazolone (ALDRICH), 0.01 mol, are dissolved in 50 ml of glacial acetic acid (synthesis A) or in 50 ml of tetrahydrofuran with the addition of 0.1 ml of conc. hydrochloric acid (synthesis B) and the solution is boiled under reflux until starting materials can no longer be detected. The solvent is evaporated off in vacuo and the residue is recrystallized from methanol and then dried in vacuo.

| 3-Oxofuranone | Product | m/w | Colour | λₘₐₓ |
|---|---|---|---|---|
| Example 30/1: 5,7-Di-t-butyl-3-oxobenzofuranone | B | 416 | violet | 517 nm |
| Example 30/2:α-Naphtho-3-oxofuranone | A | 354 | red | 399 nm |

Example 31/4: Hydantoin (FLUKA), 0.02 mol, and 5,7-di-t-butyl-3-oxobenzofuranone, 0.02 mol, are dissolved in 25 ml of acetic anhydride, 2 mmol of sodium acetate are added and the mixture is boiled under reflux until starting material can no longer be detected. The product is filtered off with suction, washed with methanol and water and dried in vacuo. This gives 1.3 g of monoacetylated product (yellow, m/w= 384; λₘₐₓ= 391 nm) of the formula

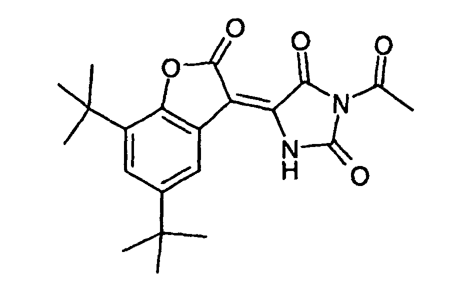

Example 33/1-4: General preparation of 2-hydroxyimines, the 3-oxofuranone and the 2-hydroxyaniline being replaced in each case in accordance with the table below:

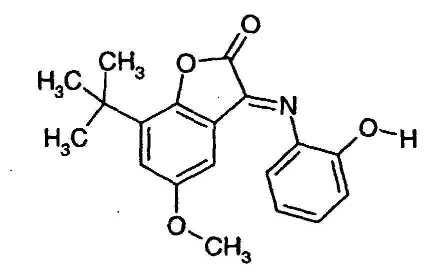

The hydroxy aniline (Fluka), 0.02 mol, is dissolved together with 3-oxofuranone (0.02 mol) in 50 ml of glacial acetic acid (Fluka) and the solution is heated at 100°C until starting material can no longer be detected. The precipitate is filtered off with suction or, if appropriate, the solvent is concentrated by evaporation and the residue is recrystallized from glacial acetic acid, if appropriate with the addition of active carbon, and dried in vacuo.

| 3-Oxo furanone | 2-Hydroxy aniline | m/w | Colour | λₘₐₓ |
|---|---|---|---|---|
| Example 33/1: 5-t-Butyl-7-methoxy-3-oxobenzofuranone | 2-Hydroxyaniline | 325 | orange | 463 nm |
| Example 33/2: 5-t-Butyl-7-methoxy-3-oxobenzofuranone | 2-Hydroxy-1-aminonaphthalene | 375 | red | 463 nm |
| Example 33/3: 5,7-Di-t-butyl-3-oxo-benzofuranone | 2-Hydroxy-1-aminonaphthalene | 401 | orange | 412 nm |
| Example 33/4: β-Naphtho-3-oxofuranone | 2-Hydroxyaniline | 289 | orange | 452 nm |

Example 34: Synthesis of a quinomethide 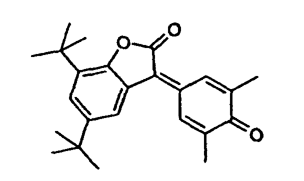 3-(2,5-Dimethyl-4-hydroxyphenyl)-3H-5,7-di-t-butylbenzofuranone, 13.6 mmol, prepared, for example, in accordance with Synlett 1999, S1, pp. 863-864, dissolved in 100 ml of toluene (Fluka) is admixed with 123.5 mmol of potassium hexacyanoferrate(III) (Fluka), 307 mmol of sodium hydroxide (Fluka) and 100 ml of water and stirred at room temperature. When starting material can no longer be detected, the organic phase is separated off, shaken with water and dried over sodium sulfate, and the solvent is removed by evaporation. The residue is recrystallized from isopropanol and dried at 60°C in vacuo. Red crystals are obtained.

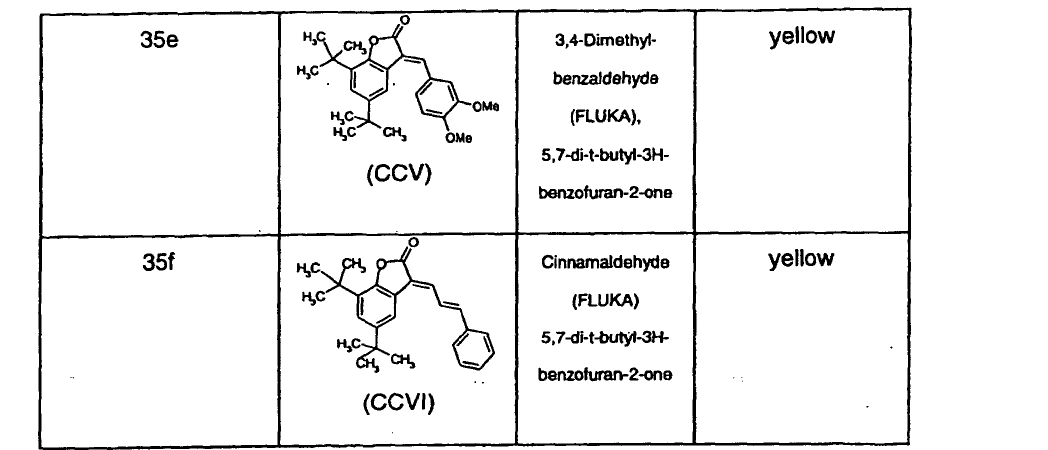

Example 36: In analogy to the process in Example 1 from EP 0 632 102 the compounds of Table 9 are prepared, or in accordance with the procedure below:

A mixture of 5,7-di-tert-butyl-3H-benzofuran-2-one, 2.0 g, 4-dimethylaminobenzaldehyde, 1.25 g (Fluka puriss) and piperidine, 0.1 ml (Fluka puriss) in 25 ml of toluene is heated at 112°C, the water of reaction being removed simultaneously using a water separator. The reaction mixture is stirred further overnight. The mixture is stirred at reflux for 7 hours. Thereafter, the mixture obtained is concentrated and the residue is treated with 30 ml of methanol. The red solid obtained is filtered, and the filter residue is washed with methanol and then with water and subsequently dried in a drying oven at 60°C and 200 mbar. This gives 1.7 g of a red powder of the formula (CCVII)

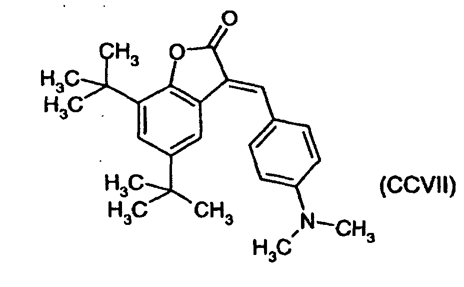

The compounds of Table 9 are prepared in analogy to the above process in Example 36:

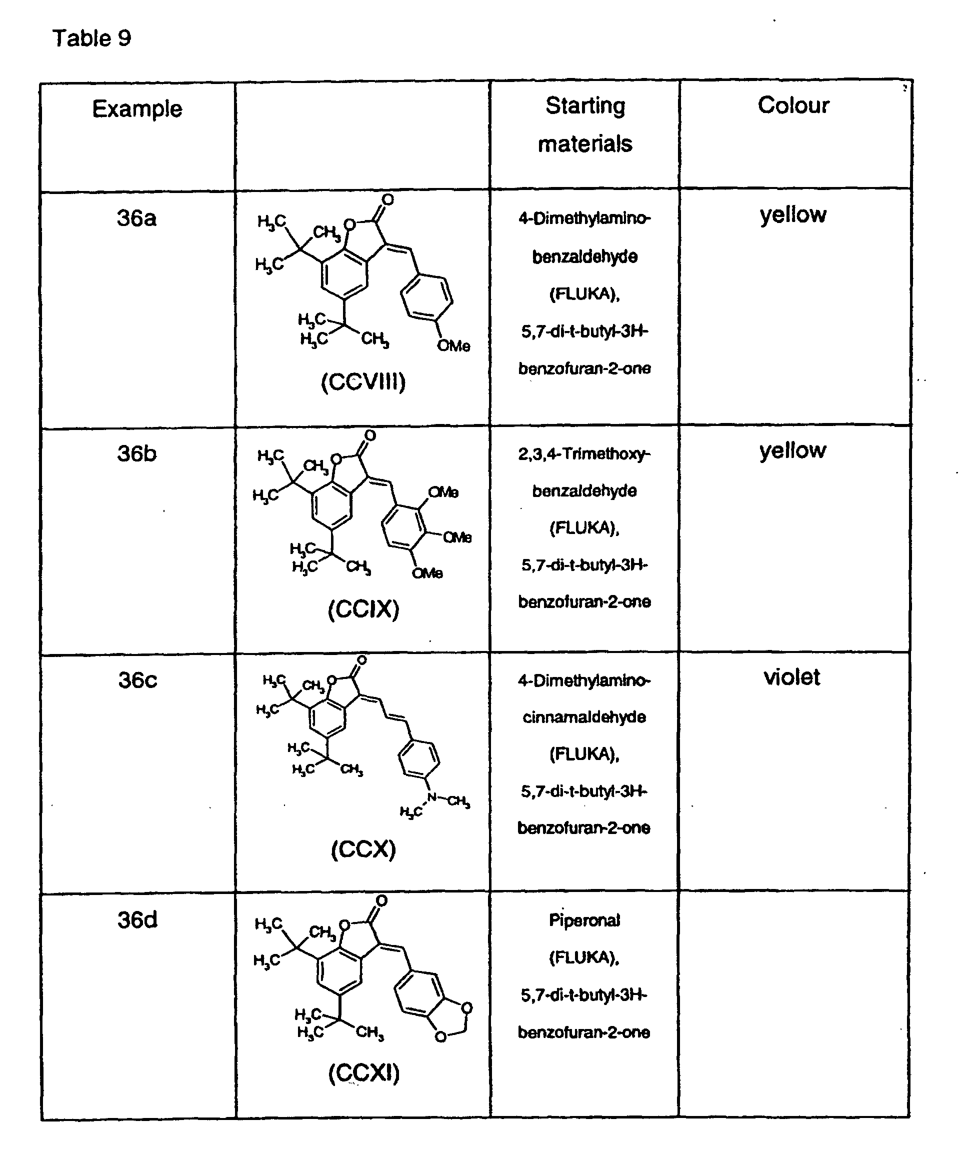

Example 37: 15 g of vinyl copolymer (with 13% acetate, 86% chlorine and 1% copolymerized maleic acid, e.g. VINYLITE VMCH from UCC) is stirred into 30 g of toluene and 50 g of methyl ethyl ketone and brought into solution completely (dissolver for about 20 minutes or propeller stirred for about 1 hour). Subsequently, 5 g of a lactone dye of the formulae (XLIII-LXXIII) is incorporated by stirring for 5-15 minutes. The printing ink prepared in this way is applied to aluminium or to metallized polymer films or used as the basis for a hot stamp onto a polyester film.

Example 38: Preparation of injection-moulded plaques in polyethylene terephthalate (PET) 0.3 g of compound of the formulae (XLIII-LXXIII) is mixed with 1500 g of polyethylene terephthalate (PET) [™MELINAR PURA, ICI, predried at 120°C) briefly by hand, then on a tumble mixer at 50 rpm for 5 min. This mixture is subsequently preextruded at 270°C on a 25 mm single-screw extruder (Collin).

The compound is subsequently processed on a microprocessor-controlled injection moulding machine (™Ferromatik FM 40, Klöckner). The residence time of the polymer (dependent on cycle time, screw volume and plastification volume) is 5 min, during which backpressure and screw speed are kept low. This is beneficial to the homogeneity of the plastic and prevents the generation of frictional heat. The first mouldings (plaques 65 × 25 × 1.5 mm in size) are discarded.

The mouldings obtained at 270°C, 280°C, 290°C and 300°C are notable for very high heat stability, high light fastness, good migration resistance and high colour strength.

## Claims

1. A compound of the formula (Ia) or (Ic) ${\text{Q}}_{\text{1}} {\text{=X}}_{\text{1}} \text{(la)}$${\text{Q}}_{\text{1}} {\text{=X}}_{\text{2}} {\text{=Q}}_{\text{2}} \text{(Ic)}$in which
Q₁ is a benzofuran-2-one of the formula (IIa),
and
Q₂ is a benzofuran-2-one of the formula (IIb) 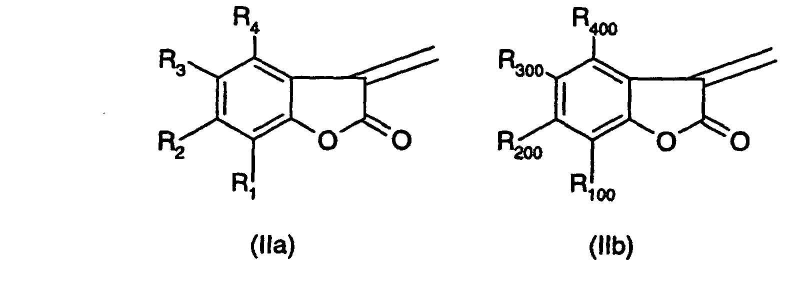 in which
R₁, R₂, R₃, R₄, R₁₀₀, R₂₀₀, R₃₀₀ or R₄₀₀ independently of one another are hydrogen, halogen, hydroxyl, cyano, ether, nitro, an amine, amide, imine, urethane, sulfonamide, ester, carboxylic acid or sulfonic acid radical or carboxylic salt, sulfonic salt or substituted or unsubstituted C₁-C₂₄alkyl, C₁-C₂₄alkoxy, C₁-C₂₄alkylthio, C₅-C₁₂cycloalkyl, C₅-C₁₂cycloalkoxy, C₅-C₁₂cycloalkylthio, C₂-C₂₄alkenyl, C₆-C₂₄aryl, C₇-C₂₅aralkyl, C₆-C₂₄aryloxy, C₆-C₂₄arylthio, A₅-A₁₈heteroaryl, A₅-A₁₈heteroaryloxy or A₅-A₁₈heteroarylthio,
or
R₁ and R₂, R₂ and R₃, R₃ and R₄ or R₁₀₀ and R₂₀₀, or R₂₀₀ and R₃₀₀, R₃₀₀ and R₄₀₀, independently of one another in each case together are divalent, substituted or unsubstituted radicals, such as polycyclic radicals or 1,3-butadien-1,4-ylene or -CH=CH-NH-, the two last radicals forming an additional fused-on 5- or 6-membered ring,
with the proviso that Q₁ und Q₂ are different
and
X₁ is a hydrazone or imine radical, with the proviso that, if R₁, R₂, R₃ and R₄ are hydrogen, or at least one R₁, R₂, R₃ or R₄ is methyl, the hydrazone radical is excluded, or, if R₁, R₂, R₃ or
R₄ is hydrogen, X₁ is not phenylimine- or 4-dimethylamine-phenylimine,
or
X₁ is a methylene radical, 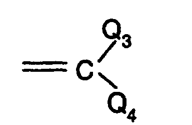 in which
Q₃ is a substituted or unsubstituted primary or secondary amine radical and Q₄ is hydrogen or a substituted or unsubstituted C₁-C₂₄alkyl, -CO-(C₁-C₂₄alkyl), -CO-O-(C₁-C₂₄alkyl), C₁-C₂₄alkoxy, C₁-C₂₄alkylthio, C₅-C₁₂cycloalkyl, C₅-C₁₂cycloalkoxy, C₅-C₁₂cycloalkylthio, C₂-C₂₄ alkenyl, C₆-C₂₄aryl, -CO-O-(C₆-C₂₄aryl), -CO-(C₆-C₂₄aryl), C₆-C₂₄aryloxy, C₆-C₁₂arylthio, C₇-C₂₅aralkyl, A₅-A₁₈heteroaryl, A₅-A₁₈heteroaryloxy or A₅-A₁₈heteroarylthio,
or
Q₃ and Q₄ together are a lactam, quinomethylene, hydantoin, acenaphthenequinone, azlactone, pyrazolonyl, barbituric acid, isoindolinone or isoindoline radical,
with the proviso that
Q₄ is not hydrogen if R₃ is hydrogen, methoxy or hydroxyl and R₁, R₂ and R₄ are hydrogen, and
X₂ is a tetravalent 5- or 6-membered heterocyclic ring,
or is 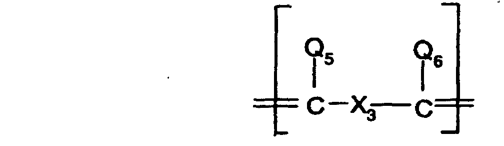 in which
X₃ is a single bond, unsubstituted or substituted C₆-C₂₄arylene, A₅-A₁₈heteroarylene, 1,2-phenylene, 1,3-phenylene, 1,4-phenylene or naphthylene, or a tetravalent polyether, polyimine, polyamine radical, or bi(C₆-C₂₄)arylene, bi(A₅-A₁₈)heteroarylene, C₂-C₂₄alkenylene, in which bi(C₆-C₂₄)arylene, bi(A₅-A₁₈)heteroarylene or C₂-C₂₄alkenylene can be interrupted by one or more intermediate units such as -CH=CH-, -CH=N-, -N=N-, -CR₄₄R₄₂-, -CO-, -COO-, -OCO-, -NR₄₂CO-, -CONR₄₂-, -O-, -S-, -SO-, -SO₂- or -NR₄₂-,
in which
R₄₂ and R₄₄ independently of one another are hydrogen, substituted or unsubstituted C₁-C₂₄alkyl, C₅-C₁₂cycloalkyl, C₂-C₂₄ alkenyl, C₆-C₂₄aryl, C₇-C₂₅aralkyl or A₅-A₁₈heteroaryl, and
Q₅ and Q₆ independently of one another are hydrogen, C₆-C₂₄aryl, C₆-C₂₄aryloxy, C₁-C₂₄alkyl, C₁-C₂₄ alkoxy, C₁-C₂₄ alkylthio, C₅-C₁₂cycloalkyl, C₅-C₁₂cycloalkoxy, C₅-C₁₂cycloalkylthio, C₂-C₂₄alkenyl, C₆-C₂₄aryl, C₆-C₂₄aryloxy, C₆-C₂₄arylthio or A₅-A₁₈heteroaryl, A₅-A₁₈heteroaryloxy, A₅-A₁₈heteroarylthio,
with the proviso that if R₂, R₄, R₂₀₀, R₄₀₀ are hydrogen and R₁, R₃, R₁₀₀ and R₃₀₀ are tert-butyl or hydrogen or R₃, R₄, R₃₀₀, R₄₀₀ are hydrogen and R₁ and R₂, R₁₀₀ and R₂₀₀ together are divalent, unsubstituted 1,3-butadien-1,4-ylene radicals, forming an additional fused 6-membered ring, and Q₅ and Q₆ are hydrogen, X₃ is not 1,4-phenylene,
or
X₂ is 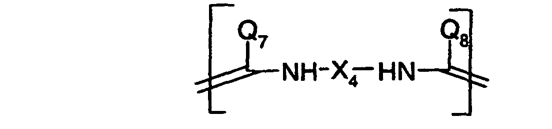 in which
Q₇ and Q₈ independently of one another are hydrogen, C₈-C₂₄aryl, C₆-C₂₄aryloxy, C₁-C₂₄alkyl, C₁-C₂₄ alkoxy, C₁-C₂₄alkylthio, C₅-C₁₂cycloalkyl, C₅-C₁₂cycloalkoxy, C₅-C₁₂cycloalkylthio, C₂-C₂₄alkenyl, C₆-C₂₄aryl, C₆-C₂₄aryloxy, C₆-C₂₄arylthio or A₅-A₁₈heteroaryl, A₅-A₁₈heteroaryloxy, A₅-A₁₈heteroarylthio,
and
X₄ is C₆-C₂₄arylene, A₅-A₁₈heteroarylene, a polymethylidene or divalent polyether, polyimine, polyamine radical, or bi(C₆-C₂₄)arylene, bi(A₅-A₁₈)heteroarylene, C₂-C₂₄alkenylene, in which bi(C₆-C₂₄)arylene, bi(A₅-A₁₈)heteroarylene or C₂-C₂₄alkenylene can be interrupted by one or more intermediate units such as -CH=CH-, -CH=N-, -N=N-, -CR₄₄R₄₂-, -CO-, -COO-, -OCO-, -NR₄₂CO-, -CONR₄₂-, -O-, -S-, -SO-, -SO₂- or -NR₄₂-,
or
X₂ is

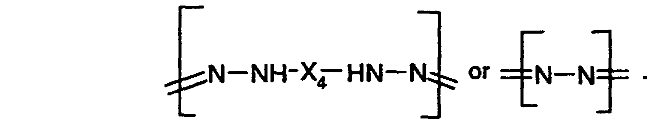

2. A compound according to claim 1 of the formula (XVI) 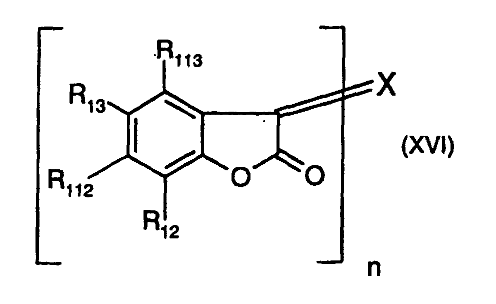 in which
n is 1
X is X₁ as defined in claim 1, and
R₁₂, R₁₁₂, R₁₃ and R₁₁₃ independently of one another are hydrogen, halogen, OH, NO₂, R₁₄, OR₁₄, OC₉-C₁₈alkyl or SC₉-C₁₈alkyl, in which
R₁₄ is C₁-C₂₄alkyl which is unsubstituted or substituted one or more times by oxo or by COO⁻ X₅⁺ and which can be uninterrupted or interrupted one or more times by O, N and/or S, or is C₇-C₁₈aralkyl or C₆-C₁₂aryl unsubstituted or substituted one or more times by halogen, OR₁₆, NR₁₆R₁₇, COOR₁₆, CONR₁₆R₁₇, NR₁₈COR₁₆ or NR₁₈COOR₁₆,
X₅⁺ is a cation H⁺, Na⁺, K⁺, Mg⁺⁺_{½}, Ca⁺⁺_{½}, Zn⁺⁺_{½}, Al⁺⁺⁺_{1/3}, or [NR₁₆R₁₇R₁₈R₁₉]⁺,
and
R₁₆ and R₁₇ independently of one another are hydrogen, C₆-C₁₂aryl, C₇-C₁₀aralkyl, or C₁-C₈alkyl which is unsubstituted or substituted one or more times by halogen, hydroxyl or C₁-C₄alkoxy,
or
R₁₆ and R₁₇ in NR₁₆R₁₇ or CONR₁₆R₁₇, together with the nitrogen atom connecting them, are pyrrolidine, piperidine, piperazine or morpholine each of which is unsubstituted or substituted from one to four times by C₁-C₄alkyl,
and
R₁₈ and R₁₉ independently of one another are hydrogen, C₁-C₈alkyl, C₆-C₁₀aryl or C₆-C₁₂aralkyl,
R₁₂ and R₁₁₂, R₁₁₂ and R₁₃, R₁₃ and R₁₁₃ can also independently of one another each together be divalent substituted or unsubstituted radicals, such as polycyclic radicals,
with the proviso that, if R₁₂, R₁₁₂, R₁₃ and R₁₁₃ are hydrogen, or at least one R₁₂, R₁₁₂, R₁₃ and
R₁₁₃ is methyl, X₁ is not a hydrazone radical, or if R₁₂, R₁₁₂, R₁₃ and R₁₁₃ are hydrogen, X₁ is not phenylimine- or 4-dimethylamine-phenylimine,
or
if X₁ is a methylene radical, 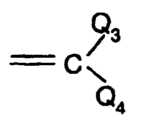 Q₄ is not hydrogen if R₁₃ is hydrogen, methoxy or hydroxyl, and R₁₂, R₁₁₂ and R₁₁₃ are hydrogen.

3. A compound according to claim 1, of the formula (XVII) 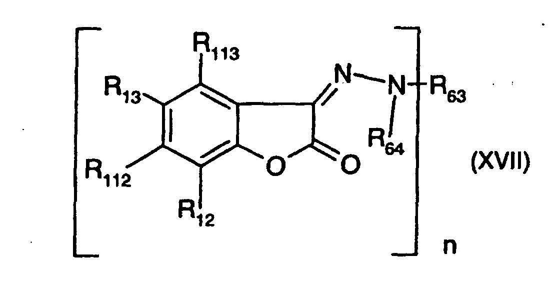 in which,
n is 1
R₆₄ independently of R₆₃ is a radical as defined under R₆₃ or is hydrogen, and
R₆₃ is substituted or unsubstituted C₁-C₁₂alkyl, C₅-C₁₂cycloalkyl, C₂-C₆alkenyl, C₆-C₁₂aryl, C₇-C₁₃aralkyl, or A₅-A₁₂heteroaryl,
or
a compound of the formula (XXIV) 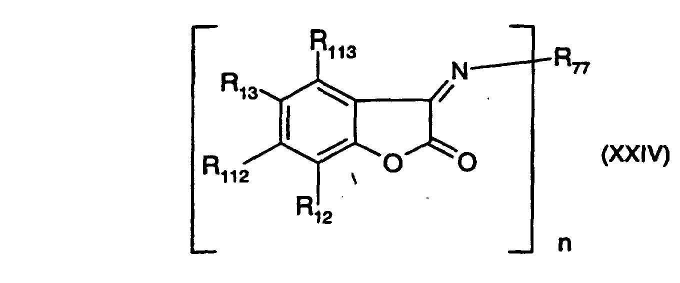 in which,
n is 1,
R₇₇ is substituted or unsubstituted C₁-C₁₂alkyl, C₅-C₆cycloalkyl, C₂-C₆alkenyl, C₆-C₁₂aryl, C₇-C₁₃aralkyl or A₅-A₁₂heteroaryl, with the proviso that in formula (XXIV), if R₁₂, R₁₁₂, R₁₃ or R₁₁₃ are hydrogen, R₇₇ is not unsubstituted phenylimine or 4-dimethylaminephenylimine,
or
a compound of the formula (XXV) 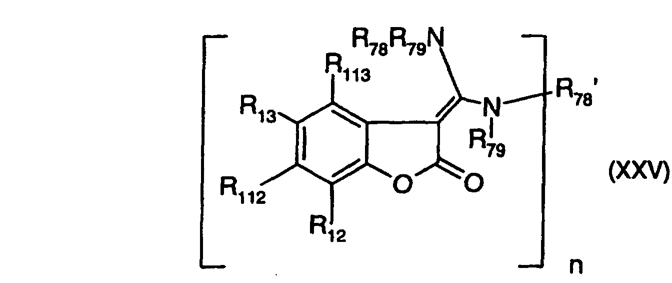 in which
n is 1
R₇₈, R₇₈' and R₇₉ independently of one another are hydrogen or substituted or unsubstituted C₁-C₁₂alkyl, C₁-C₁₂alkoxy, C₁-C₁₂alkylthio, C₅-C₆cycloalkoxy, C₅-C₆-cycloalkylthio, C₆-C₂₄aryloxy, C₆-C₂₄arylthio or A₅-A₁₂heteroaryloxy, A₅-A₁₂heteroarylthio, C₅-C₆cycloalkyl, C₂-C₁₂alkenyl, C₆-C₁₂aryl, C₇-C₁₃aralkyl, or A₅-A₁₂heteroaryl, or dependently of one another are hydrogen,
or
a compound of the formula (XXVI) 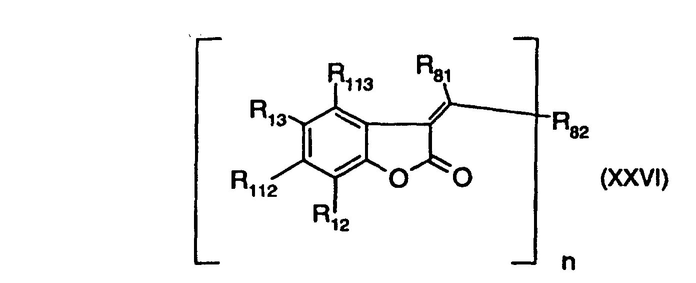 in which
n is 1
R₈₁ is a primary or secondary amine radical, and R₈₂ is hydrogen or unsubstituted or substituted C₁-C₁₂alkyl, -CO-(C₁-C₂₄alkyl), -CO-O-(C₁-C₂₄alkyl), C₆-C₁₂aryloxy, C₁-C₁₂alkoxy, C₁-C₁₂alkylthio, C₅-C₁₂cycloalkyl, C₅-C₁₂cycloalkoxy, C₂-C₁₂alkenyl, a primary or secondary amine radical, C₆-C₁₈aryl, -CO-O-(C₆-C₂₄aryl), -CO-(C₆-C₂₄aryl), C₆-C₁₈aryloxy, C₆-C₁₈arylthio or A₅-A₁₂heteroaryl, A₅-A₁₂heteroaryloxy, A₅-A₁₂heteroarylthio, or R₈₁ and R₈₂ together are a lactam, quinomethylene, hydantoin, acenaphthenequinone, azlactone, pyrazolonyl, barbituric acid, isoindolinone or isoindoline radical,
with the proviso that
R₈₂ is not hydrogen if R₁₃ is hydrogen, methoxy or hydroxyl and R₁₂, R₁₁₂ and R₁₁₃ are hydrogen.

4. A process for preparing a benzofuran-2-one (la) according to claim 1, which comprises reacting benzofuran-2-one (XXXa) 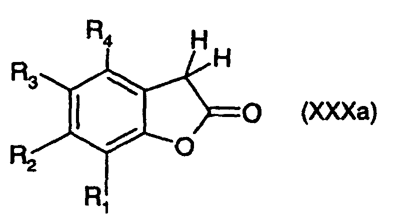 with a compound of the formula (XXXIa), (XXXIIa), (XXXIIIa), (XXXIVa) or (XXXVa) 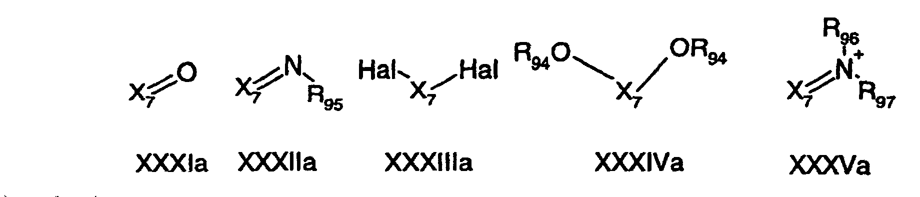 in which
Hal is halogen,
and
R ₉₄ is substituted or unsubstituted C₁-C₂₄alkyl, C₅-C₁₂cycloalkyl, C₂-C₂₄alkenyl, C₆-C₂₄aryl, C₇-C₂₅aralkyl or A₅-A₁₈heteroaryl,
and
R ₉₅ is hydrogen or hydroxyl,
R ₉₆ and R ₉₇ independently of one another are C₆-C₁₂aryl, C₁-C₅acyl, C₆-C₁₂aralkyl, or C₁-C₄alkyl,
and
X₇ is a methylene radical, 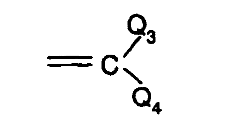 in which
Q₃ is a substituted or unsubstituted primary or secondary amine radical and Q₄ is hydrogen or a substituted or unsubstituted C₁-C₂₄alkyl, -CO-(C₁-C₂₄alkyl), -CO-O-(C₁-C₂₄alkyl), C₁-C₂₄alkoxy, C₁-C₂₄alkylthio, C₅-C₁₂cloalkyl, C₅-C₁₂cycloalkoxy, C₅-C₁₂cycloalkylthio, C₂-C₂₄alkenyl, C₆-C₂₄aryl, -CO-O-(C₆-C₂₄aryl), -CO-(C₆-C₂₄aryl), C₆-C₂₄aryloxy, C₆-C₁₂arylthio, C₇-C₂₅aralkyl, A₅-A₁₈heteroaryl, A₅-A₁₈heteroaryloxy or A₅-A₁₈heteroarylthio,
or
Q₃ and Q₄ together are a lactam, quinomethylene, hydantoin, acenaphthenequinone, azlactone, pyrazolonyl, barbituric acid, isoindolinone or isoindoline radical,
with the proviso that
Q₄ is not hydrogen if R₃ is hydrogen, methoxy or hydroxyl and R₁, R₂ and R₄ are hydrogen.

5. A process for preparing a benzofuran-2-one (Ic) according to claim 1, which comprises reacting benzofuran-2-one (XXXa) and a compound of the formula (XXXb) 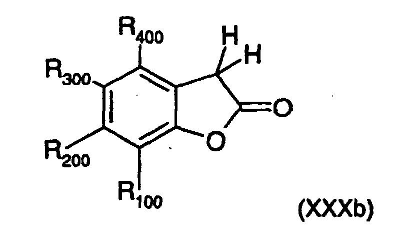 with a compound of the formulae (XXXIb), (XXXIIb), (XXXIIIb), (XXXIVb) or (XXXVb) 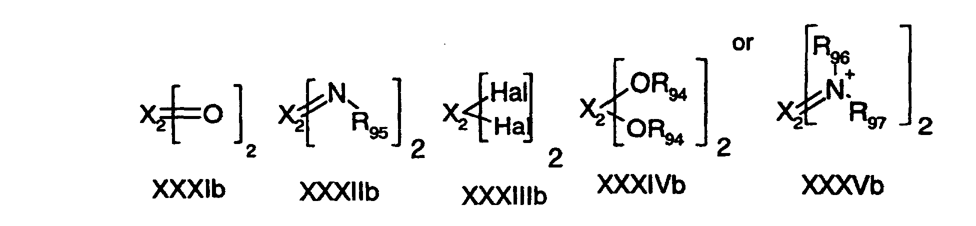 in which
X₂ is X₈ and corresponds to the definition in claim for X₂, with the proviso that X₈ is not 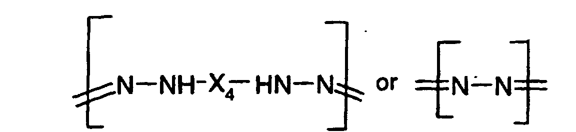 with the proviso that the compounds of formula (XXXa) and (XXXb) are different.

6. A process for preparing a benzofuran-2-one (la) according to claim 1, which comprises reacting 3-oxobenzofuran-2-one (XXXVla) 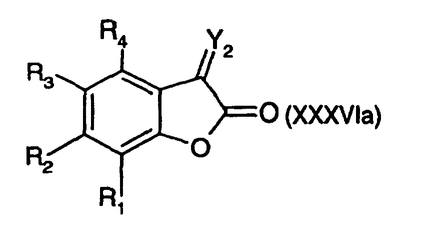 with a compound of the formula (XXXVIIa) 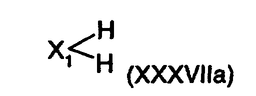 in which,
Y₂ is O, NR₉₅ or N⁺(R₉₆R₉₇), NO or two chlorine atoms, the chlorine atoms each forming a single bond with the benzofuran-2-one (la).

7. A process for preparing a benzofuran-2-one (Ic) according to claim 1, which comprises reacting 3-oxobenzofuran-2-one (XXXVIa) and a compound of the formula (XXXVIb) 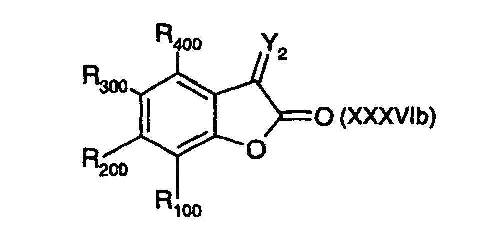 with a compound of the formula (XXXVIIb) 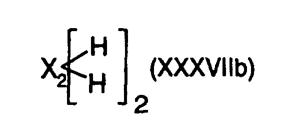 with the proviso that the compounds of formula (XXXVIa) and (XXXVIb) are different.

8. An aminohydroxy compound of the formula (XLIa) or (XLIb) 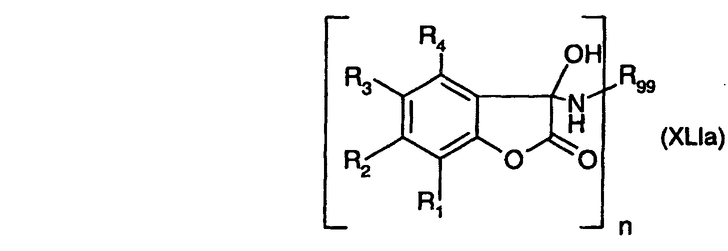 or 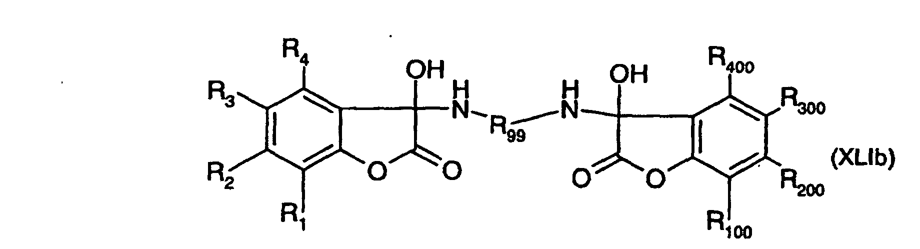 in which
n is 1
R₉₉ is hydrogen or substituted or unsubstituted C₁-C₂₄alkyl, C₅-C₁₂cycloalkyl, C₂-C₂₄alkenyl, C₆-C₂₄aryl, C₇-C₂₅aralkyl or A₅-A₁₈heteroaryl.

9. A process for preparing an amine hydroxy compound of the formula (XLIa) or (XLIb) according to claim 8, which comprises reacting 3-oxobenzofuran-2-one (XXXVIa) according to claim 6 with a compound of the formula (XXXVIIa)
H―X₁―H (XXXVIIa)
or
reacting 3-oxobenzofuran-2-one (XXXVIa) and (XXXVIb) of claim 7 with a compound of the formula (XXXVIIb)
H―X₂―H (XXXVIIb).

10. A process for preparing a benzofuran-2-one (Ia) or (Ic) according to claim 1 in which X₁ is a compound of the formula (IV) 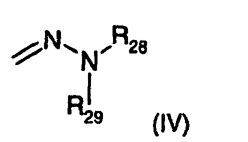 in which
R₂₈ and R₂₉ independently of one another are substituted or unsubstituted C₁-C₂₄alkyl, C₅-C₁₂cycloalkyl, C₂-C₂₄alkenyl, C₆-C₂₄aryl, C₇-C₂₅aralkyl, A₅-A₁₈heteroaryl or dependently of one another are hydrogen,
and
X₂ is a compound of the formula (X) 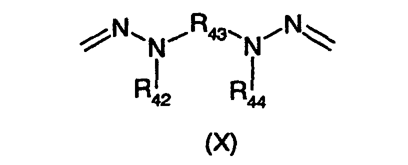 in which
R₄₂ and R₄₄ independently of one another are substituted or unsubstituted C₁-C₂₄alkyl, C₅-C₁₂cycloalkyl, C₂-C₂₄ alkenyl, C₆-C₂₄aryl, C₇-C₂₅aralkyl, A₅-A₁₈heteroaryl or dependently of one another are hydrogen,
and
R₄₃ is a direct bond, C₆-C₂₄arylene, A₅-A₁₈heteroarylene, C₅-C₁₂cloalkyl or bi(C₆-C₂₄)arylene, bi(A₅-A₁₈)heteroarylene, C₂-C₂₄alkenylene, in which bi(C₆-C₂₄)arylene, bi(A₅-A₁₈)heteroarylene, C₂-C₂₄alkenylene can be connected to one another and/or interrupted by a direct bond or by one or more intermediate units such as -CH=CH-, -CH=N-, -N=N-, -CR₄₄R₄₂-, -CO-, -COO-, -OCO-, -NR₄₂CO-, -CONR₄₂-, -O-, -S-, -SO-, -SO₂- or -NR₄₂-,
in which
R₄₂ and R₄₄ independently of one another are hydrogen, substituted or unsubstituted C₁-C₂₄alkyl, C₅-C₁₂cycloalkyl, C₂-C₂₄ alkenyl, C₆-C₂₄aryl, C₇-C₂₅aralkyl, or A₅-A₁₈heteroaryl, by coupling diazotized amines with coupling components in an aqueous medium, which comprises reacting benzofuran-2-one (XXXa) of claim 4 with a diazonium salt of the formula (XXXVIIIa) 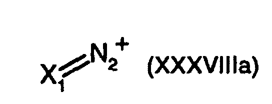 or
reacting benzofuran-2-one (XXXa) and (XXXb) of claim 5 with a diazonium salt of the formula (XXXVIIIb)

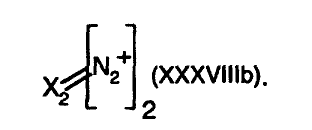

11. A composition consisting of from 2 to 10, preferably 2 or 3, compounds of the formulae (la) and/or (Ic) according to claim 1, and/or (XLIa) and/or (XUb) according to claim 8, and/or dimeric benzofuran-2-ones of the formula (XLIIb) 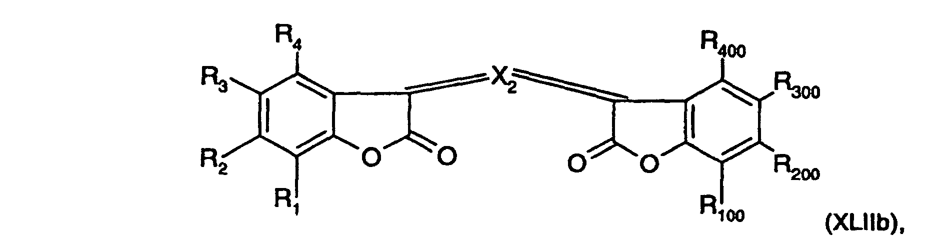 in which X₂ is (C₆-C₂₄)arylene, (A₅-A₁₈)heteroarylene or a divalent polymethylidene, polyether, polyimine, polyamine radical, or bi(C₆-C₂₄)arylene or bi(A₅-A₁₈)heteroarylene, the bi(C₆-C₂₄)arylene or bi(A₅-A₁₈)heteroarylene radical being attached directly or via a substituted or unsubstituted carbon, nitrogen, oxygen or (-N=N-)-diradical, with the proviso that if R₂, R₄, R₂₀₀, R₄₀₀ are hydrogen and R₁, R₃, R₁₀₀ and R₃₀₀ are tert-butyl or hydrogen or R₃, R₄, R₃₀₀, R₄₀₀ are hydrogen and R₁ and R₂, R₁₀₀ and R₂₀₀ together are divalent, unsubstituted 1,3-butadien-1,4-ylene radicals, forming an additional fused 6-membered ring, X₂ is not CH-(C₆H₄)-CH.

12. A composition of matter comprising a high molecular weight organic material and a compound of the formula (Ia) according to claim 1
in which
X₁ is X₁₀, where X₁₀ is a substituted or unsubstituted hydrazone or imine radical, or is a methylene radical 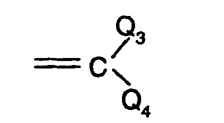 in which
Q₃ is a substituted or unsubstituted primary or secondary amine radical and Q₄ is hydrogen or a substituted or unsubstituted C₁-C₂₄alkyl, -CO-(C₁-C₂₄alkyl), -CO-O-(C₁-C₂₄alkyl),
C₁-C₂₄alkoxy, C₁-C₂₄alkylthio, C₅-C₁₂cycloalkyl, C₅-C₁₂cycloalkoxy, C₅-C₁₂cycloalkylthio, C₂-C₂₄ alkenyl, C₆-C₂₄aryl, -CO-O-(C₆-C₂₄aryl), -CO-(C₆-C₂₄aryl), C₆-C₂₄aryloxy, C₆-C₁₂arylthio, C₇-C₂₅aralkyl, A₅-A₁₈heteroaryl, A₅-A₁₈heteroaryloxy or A₅-A₁₈heteroarylthio,
or
Q₃ and Q₄ together are a lactam, quinomethylene, hydantoin, acenaphthenequinone, azlactone, pyrazolonyl, barbituric acid, isoindolinone or isoindoline radical,
with the proviso that
Q₄ is not hydrogen if R₃ is hydrogen, methoxy or hydroxyl and R₁, R₂ and R₄ are hydrogen, or
a composition according to claim 11, (XLIa) or (XLIb) according to claim 8, in a colouringly effective amount.

13. A process for preparing a benzofuran-2-one (Ia) or (Ic) in which X₁ is X₁₀ according to claim 12, and X₁ is a compound of the formula (V) 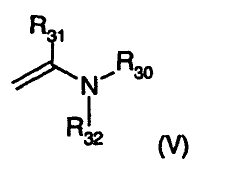 in which
R₃₁ is hydrogen or -NR₈₉R₉₀,
in which
R₃₀, R₃₂, R₈₉ and R₉₀ independently of one another are hydrogen, C₁-C₂₄ alkoxy, C₁-C₂₄alkylthio, C₅-C₁₂cycloalkoxy, C₅-C₁₂cycloalkylthio, C₅-C₂₄aryloxy, -thio or A₅-A₁₈heteroaryloxy, -thio,
or
are C₆-C₂₄aryl-substituted secondary or tertiary amine or C₆-C₂₄aryl,
and
where X₂ is of the formula (XI) 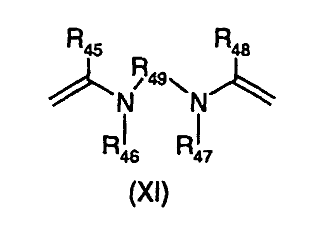 in which
R₄₆ and R₄₇ independently of one another are substituted or unsubstituted C₁-C₂₄alkyl, C₅-C₁₂cycloalkyl, C₂-C₂₄alkenyl, C₆-C₂₄aryl, C₇-C₂₅aralkyl, or A₅-A₁₈heteroaryl,
and
R₄₅ and R₄₈ independently of one another are hydrogen, C₁-C₂₄alkyl, C₁-C₂₄alkoxy, C₁-C₂₄alkylthio, C₅-C₁₂cycloalkyl, C₅-C₁₂cycloalkoxy, C₅-C₁₂cycloalkylthio, C₂-C₂₄alkenyl, C₅-C₂₄aryl, C₇-C₂₅aralkyl, C₅-C₂₄aryloxy, -thio or A₅-A₁₈heteroaryl, A₅-A₁₈heteroaryloxy, -thio,
and R₄₉ is a direct bond, C₆-C₂₄arylene, A₅-A₁₈heteroarylene, C₅-C₁₂cycloalkyl or bi(C₆-C₂₄)arylene, bi(A₅-A₁₈)heteroarylene, C₂-C₂₄alkenylene, in which bi(C₆-C₂₄)arylene, bi(A₅-A₁₈)heteroarylene, C₂-C₂₄alkenylene can be connected to one another and/or interrupted by a direct bond or by one or more intermediate units such as -CH=CH-, -CH=N-, -N=N-, -CR ₄₄R₄₂-, -CO-, -COO-, -OCO-, -NR₄₂CO-, -CONR₄₂-, -O-, -S-, -SO-, -SO₂- or -NR₄₂-,
in which
R₄₂ and R₄₄ independently of one another are hydrogen, substituted or unsubstituted C₁-C₂₄alkyl, C₅-C₁₂cycloalkyl, C₂-C₂₄alkenyl, C₆-C₂₄aryl, C₇-C₂₅aralkyl, or A₅-A₁₈heteroaryl, by formylation and subsequent reaction with an amine, which comprises reacting benzofuran-2-one (XXXa) of claim 4 with a formylating reagent of the formula (XXXVIII)
R₃₅C(OR₃₆)₃ (XXXVIII)
in which
R₃₅ and R₃₆ independently of one another are substituted or unsubstituted C₁-C₂₄alkyl, C₅-C₁₂cycloalkyl, C₂-C₂₄alkenyl, C₆-C₂₄aryl, C₇-C₂₅aralkyl, or A₅-A₁₈heteoaryl
and a compound of the formula (IXLa) 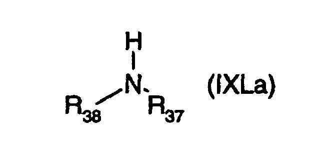 in which
R₃₇ and R₃₈ independently of one another are hydrogen or substituted or unsubstituted C₁-C₂₄alkyl, C₁-C₂₄alkoxy, C₅-C₁₂cycloalkoxy, C₅-C₁₂cycloalkylthio, C₅-C₆cycloalkyl, C₂-C₂₄alkenyl, C₆-C₂₄aryl, C₅-C₂₄aryloxy, C₅-C₂₄arylthio, C₇-C₂₅aralkyl, a primary or secondary amine radical, A₅-A₁₈heteroaryl, A₅-A₁₈heteroaryloxy or A₅-A₁₈heteroarylthio, benzofuran-2-one (XXXa) or (XXXa) and (XXXb) of claim 5 with a formylating reagent of the formula (XXXVIII) and a compound of the formula (IXLb) 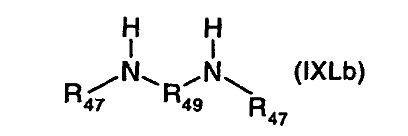 in which
R₄₆ and R₄₇ independently of one another are hydrogen or substituted or unsubstituted C₁-C₂₄alkyl, C₅-C₁₂cycloalkyl, C₂-C₂₄alkenyl, C₆-C₂₄aryl, C₇-C₂₅aralkyl, or A₅-A₁₈heteroaryl, divalent polyether, polyimine, polyamine radical, bi(C₆-C₂₄)arylene, bi(A₅-A₁₈)heteroarylene, C₂-C₂₄alkenylene, in which bi(C₆-C₂₄)arylene, bi(A₅-A₁₈)heteroarylene or C₂-C₂₄alkenylene can be interrupted by one or more intermediate units such as -CH=CH-, -CH=N-, -N=N-, -CR ₄₄R₄₂-, -CO-, -COO-, -OCO-, -NR₄₂CO-, -CONR₄₂-, -O-, -S-, -SO-, -SO₂- or -NR₄₂-,
in which
R₄₂ and R₄₄ independently of one another are hydrogen, substituted or unsubstituted C₁-C₂₄alkyl, C₅-C₁₂cycloalkyl, C₂-C₂₄alkenyl, C₆-C₂₄aryl, C₇-C₂₅aralkyl or A₅-A₁₈heteroaryl, and
R₄₉ is a direct bond or substituted or unsubstituted C₆-C₂₄arylene, A₅-A₁₈heteroarylene, C₅-C₁₂cycloalkylene or bi(C₆-C₂₄)arylene, bi(A₅-A₁₈)heteroarylene, C₂-C₂₄alkenylene, in which bi(C₆-C₂₄)arylene, bi(A₅-A₁₈)heteroarylene or C₂-C₂₄alkenylene can be interrupted by one or more intermediate units such as -CH=CH-, -CH=N-, -N=N-, -CR ₄₄R₄₂-, -CO-, -COO-, -OCO-, -NR₄₂CO-, -CONR₄₂-, -O-, -S-, -SO-, -SO₂- or -NR₄₂-.

14. A method of preparing inks or for coating materials, printing inks, mineral oils, lubricating greases, waxes or dyed or pigmented plastics, non-impact printing material or toners which comprises incorporating a colouring effective amount of compound according to claim 1 or composition according to claim 11 or composition of matter according to claim 12 therein.

## Patentansprüche

1. Verbindung der Formel (Ia) oder (Ic) ${\text{Q}}_{\text{1}} {\text{=X}}_{\text{1}} \text{(Ia)}$${\text{Q}}_{\text{1}} {\text{=X}}_{\text{2}} {\text{=Q}}_{\text{2}} \text{(Ic)}$worin
Q₁ ein Benzofuran-2-on der Formel (IIa) darstellt,
und
Q₂ ein Benzofuran-2-on der Formel (IIb) darstellt 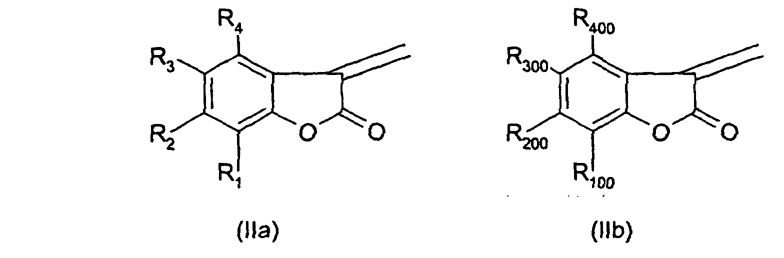 worin
R₁, R₂, R₃, R₄, R₁₀₀, R₂₀₀, R₃₀₀ oder R₄₀₀ unabhängig voneinander Wasserstoff, Halogen, Hydroxyl, Cyano, Ether, Nitro, einen Amin-, Amid-, Imin-, Urethan-, Sulfonamid-, Ester-, Carbonsäure- oder Sulfonsäure-Rest oder Carbonsäuresalz, Sulfonsäuresalz oder substituiertes oder unsubstituiertes C₁-C₂₄Alkyl, C₁-C₂₄Alkoxy, C₁-C₂₄Alkylthio, C₅-C₁₂Cycloalkyl, C₅-C₁₂Cycloalkoxy, C₅-C₁₂Cycloalkylthio, C₂-C₂₄Alkenyl, C₆-C₂₄Aryl, C₇-C₂₅Aralkyl, C₆-C₂₄Aryloxy, C₆-C₂₄Arylthio, A₅-A₁₈Heteroaryl, A₅-A₁₈Heteroaryloxy oder A₅-A₁₈Heteroarylthio bedeuten,
oder
R₁ und R₂, R₂ und R₃, R₃ und R₄ oder R₁₀₀ und R₂₀₀, oder R₂₀₀ und R₃₀₀, R₃₀₀ und R₄₀₀, unabhängig voneinander jeweils zusammen zweiwertige, unsubstituierte oder substituierte Reste bedeuten, wie polycyclische Reste oder l,3-Butadien-1,4-ylen oder -CH=CH-NH-, wobei die beiden letzten Reste einen zusätzlich ankondensierten 5- oder 6-gliedrigen Ring bilden,
mit der Maßgabe, dass Q₁ und Q₂ verschieden sind, und
X₁ einen Hydrazon- oder Imin-Rest bedeutet, mit der Maßgabe, dass, wenn R₁, R₂, R₃ und R₄ Wasserstoff bedeuten oder mindestens ein R₁, R₂, R₃ oder R₄ Methyl bedeutet, der Hydrazon-Rest ausgeschlossen ist, oder, wenn R₁, R₂, R₃ oder R₄ Wasserstoff bedeuten, X₁ nicht Phenylimin- oder 4-Dimethylamin-phenylimin bedeutet,
oder
X₁ einen Methylen-Rest bedeutet, 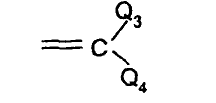 worin
Q₃ einen substituierten oder unsubstituierten primären oder sekundären Amin-Rest bedeutet und Q₄ Wasserstoff oder substituiertes oder unsubstituiertes C₁-C₂₄Alkyl, -CO-(C₁-C₂₄Alkyl), -CO-O-(C₁-C₂₄Alkyl), C₁-C₂₄Alkoxy, C₁-C₂₄Alkylthio, C₅-C₁₂Cycloalkyl, C₅-C₁₂Cycloalkoxy, C₅-C₁₂Cycloalkylthio, C₂-C₂₄Alkenyl, C₆-C₂₄Aryl, -CO-O-(C₆-C₂₄Aryl), -CO- (C₆-C₂₄Aryl), C₆-C₂₄Aryloxy, C₆-C₁₂Arylthio, C₇-C₂₅Aralkyl, A₅-A₁₈Heteroaryl, A₅-A₁₈Heteroaryloxy oder A₅-A₁₈Heteroarylthio bedeutet,
oder
Q₃ und Q₄ zusammen einen Lactam-, Chinomethylen-, Hydantoin-, Acenaphthenchinon- Azlacton-, Pyrazolonyl-, Barbitursäure-[ Isoindolinon- oder Isoindolin-Rest bedeuten,
mit der Maßgabe, dass
Q₄ nicht Wasserstoff bedeutet, wenn R₃ Wasserstoff, Methoxy oder Hydroxyl bedeutet und R₁, R₂ und R₄ Wasserstoff bedeuten, und
X₂ einen vierwertigen 5- oder 6-gliedrigen heterocyclischen
Ring bedeutet, oder 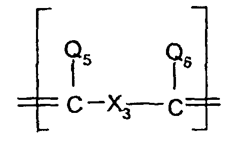 bedeutet,
worin
X₃ eine Einfachbindung, unsubstituiertes oder substituiertes C₆-C₂₄Arylen, A₅-A₁₈Heteroarylen, 1,2-Phenylen, 1,3-Phenylen, 1,4-Phenylen oder Naphthylen, oder einen vierwertigen Polyether-, Polyimin-, Polyamin-Rest oder Bi-(C₆-C₂₄)arylen, Bi(A₅-A₁₈)heteroarylen, C₂-C₂₄Alkenylen, worin Bi-(C₆-C₂₄)arylen, Bi-(A₅-A₁₈)heteroarylen oder C₂-C₂₄Alkenylen durch eine oder mehrere Zwischeneinheiten, wie -CH=CH-, -CH=N-, -N=N-, -CR₄₄R₄₂-, -CO-, -COO-, -OCO-, -NR₄₂CO-, -CONR₄₂-, -O-, -S-, -SO-, -SO₂- oder -NR₄₂-, unterbrochen sein können, bedeutet, worin
R₄₂ und R₄₄ unabhängig voneinander Wasserstoff, substituiertes oder unsubstituiertes C₁-C₂₄Alkyl, C₅-C₁₂Cycloalkyl, C₂-C₂₄Alkenyl, C₆-C₂₄Aryl, C₇-C₂₅Aralkyl oder A₅-A₁₈Heteroaryl bedeuten, und
Q₅ und Q₆ unabhängig voneinander Wasserstoff, C₆-C₂₄Aryl, C₆-C₂₄Aryloxy, C₁-C₂₄Alkyl, C₁-C₂₄Alkoxy, C₁-C₂₄Alkylthio, C₅-C₁₂Cycloalkyl, C₅-C₁₂Cycloalkoxy, C₅-C₁₂Cycloalkylthio, C₂-C₂₄Alkenyl, C₆-C₂₄Aryl, C₆-C₂₄Aryloxy, C₆-C₂₄Arylthio oder A₅-A₁₈Heteroaryl, A₅-A₁₈Heteroaryloxy, A₅-A₁₈Heteroarylthio bedeuten,
mit der Maßgabe, dass, wenn R₂, R₄, R₂₀₀, R₄₀₀ Wasserstoff darstellen und R₁, R₃, R₁₀₀ und R₃₀₀ tert-Butyl oder Wasserstoff darstellen oder R₃, R₄, R₃₀₀, R₄₀₀ Wasserstoff darstellen und R₁ und R₂, R₁₀₀ und R₂₀₀ zusammen zweiwertige, unsubstituierte 1,3-Butadien-1,4-ylen-Reste bedeuten, die einen zusätzlich ankondensierten 6-gliedrigen Ring bilden, und Q₅ und Q₆ Wasserstoff darstellen, X₃ nicht 1,4-Phenylen bedeutet,
oder
X₂ 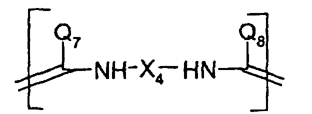 bedeutet,
worin
Q₇ und Q₈ unabhängig voneinander Wasserstoff, C₆-C₂₄Aryl, C₆-C₂₄Aryloxy, C₁-C₂₄Alkyl, C₁-C₂₄Alkoxy, C₁-C₂₄Alkylthio, C₅-C₁₂Cycloalkyl, C₅-C₁₂Cycloalkoxy, C₅-C₁₂Cycloalkylthio, C₂-C₂₄Alkenyl, C₆-C₂₄Aryl, C₆-C₂₄Aryloxy, C₆-C₂₄Arylthio oder A₅-A₁₈Heteroaryl, A₅-A₁₈Heteroaryloxy, A₅-A₁₈Heteroarylthio bedeuten,
und
X₄ C₆-C₂₄Arylen, A₅-A₁₈Heteroarylen, einen Polymethyliden- oder zweiwertigen Polyether-, Polyimin-, Polyamin-Rest, oder Bi(C₆-C₂₄)arylen, Bi-(A₅-A₁₈)heteroarylen, C₂-C₂₄Alkenylen, worin Bi-(C₆-C₂₄)arylen, Bi-(A₅-A₁₈)heteroarylen oder C₂-C₂₄Alkenylen durch eine oder mehrere Zwischeneinheiten, wie -CH=CH-, -CH=N-, -N=N-, -CR₄₄R₄₂-, -CO-, -COO-, -OCO-, -NR₄₂CO-, -CONR₄₂-, -O-, -S-, -SO-, -SO₂- oder -NR₄₂-, unterbrochen sein können, bedeutet,
oder
X₂ 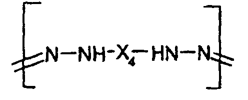 oder 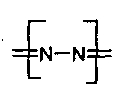 bedeutet.

2. Verbindung nach Anspruch 1 der Formel (XVI) 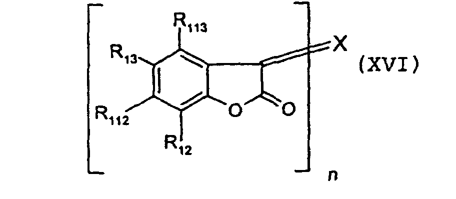 worin
n 1 ist,
X X₁, wie in Anspruch 1 definiert, darstellt, und
R₁₂, R₁₁₂, R₁₃ und R₁₁₃ unabhängig voneinander Wasserstoff, Halogen, OH, NO₂, R₁₄, OR₁₄, OC₉-C₁₈Alkyl oder SC₉-C₁₈Alkyl bedeuten, worin
R₁₄ unsubstituiertes oder einfach oder mehrfach mit Oxo oder COO⁻X₅⁺ substituiertes C₁-C₂₄Alkyl und welches nicht unterbrochen oder einfach oder mehrfach durch O, N und/oder S unterbrochen sein kann, bedeutet oder unsubstituiertes oder einfach oder mehrfach mit Halogen, OR₁₆, NR₁₆R₁₇, COOR₁₆, CONR₁₆R₁₇, NR₁₈COR₁₆ oder NR₁₈COOR₁₆ substituiertes C₇-C₁₈Aralkyl oder C₆-C₁₂Aryl bedeutet,
X₅⁺ ein Kation H⁺, Na⁺, K⁺, Mg⁺⁺_{1/2}, Ca⁺⁺_{1/2}, Zn⁺⁺_{1/2}, Al⁺⁺⁺_{1/3} oder [NR₁₆R₁₇R₁₈R₁₉]⁺ darstellt,
und
R₁₆ und R₁₇ unabhängig voneinander Wasserstoff, C₆-C₁₂Aryl, C₇-C₁₀Aralkyl, oder unsubstituiertes oder einfach oder mehrfach mit Halogen, Hydroxyl oder C₁-C₄Alkoxy substituiertes C₁-C₈Alkyl bedeuten,
oder
R₁₆ und R₁₇ in NR₁₆R₁₇ oder CONR₁₆R₁₇ zusammen mit dem sie verbindenden Stickstoffatom unsubstituiertes oder mit C₁-C₄Alkyl einfach bis vierfach substituiertes Pyrrolidin, Piperidin, Piperazin oder Morpholin bedeuten,
und
R₁₈ und R₁₉ unabhängig voneinander Wasserstoff, C₁-C₈Alkyl, C₆-C₁₀Aryl oder C₆-C₁₂Aralkyl bedeuten,
R₁₂ und R₁₁₂, R₁₁₂ und R₁₃, R₁₃ und R₁₁₃ auch unabhängig voneinander jeweils zusammen zweiwertige substituierte oder unsubstituierte Reste, wie polycyclische Reste, bedeuten können,
mit der Maßgabe, dass wenn R₁₂, R₁₁₂, R₁₃ und R₁₁₃ Wasserstoff darstellen oder mindestens einer von R₁₂, R₁₁₂, R₁₃ und R₁₁₃ Methyl bedeutet, X₁ keinen Hydrazon-Rest bedeutet, oder wenn R₁₂, R₁₁₂, R_{13 und} R₁₁₃ Wasserstoff bedeuten, X₁ nicht Phenyliminoder 4-Dimethylamin-phenylimin bedeutet,
oder
wenn X₁ einen Methylen-Rest bedeutet, 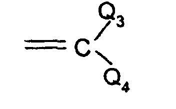 Q₄ nicht Wasserstoff bedeutet, wenn R₁₃ Wasserstoff, Methoxy oder Hydroxyl bedeutet, und R₁₂, R₁₁₂ und R₁₁₃ Wasserstoff bedeuten.

3. Verbindung nach Anspruch 1 der Formel (XVII) 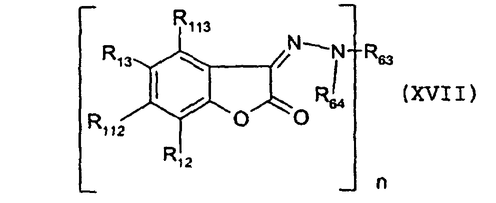 worin
n 1 ist,
R₆₄ unabhängig von R₆₃ einen Rest wie unter R₆₃ definiert bedeutet oder Wasserstoff bedeutet, und
R₆₃ substituiertes oder unsubstituiertes C₁-C₁₂Alkyl, C₅-C₁₂-Cycloalkyl, C₂-C₆Alkenyl, C₆-C₁₂Aryl, C₇-C₁₃Aralkyl oder A₅-A₁₂Heteroaryl bedeutet,
oder
eine Verbindung der Formel (XXIV) 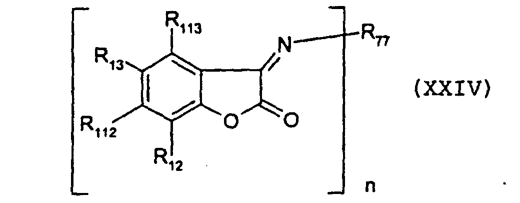 worin
n 1 ist,
R₇₇ substituiertes oder unsubstituiertes C₁-C₁₂Alkyl, C₅-C₆-Cycloalkyl, C₂-C₆Alkenyl, C₆-C₁₂Aryl, C₇-C₁₃Aralkyl oder A₅-A₁₂Heteroaryl bedeutet, mit der Maßgabe, dass in Formel (XXIV), wenn R₁₂, R₁₁₂, R₁₃ oder R₁₁₃ Wasserstoff bedeuten, R₇₇ nicht unsubstituiertes Phenylimin- oder 4-Dimethylamin-phenylimin bedeutet,
oder
eine Verbindung der Formel (XXV) 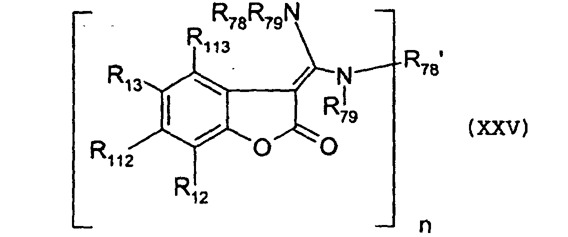 worin,
n 1 ist,
R_{78,} R_{78'} und R₇₉ unabhängig voneinander Wasserstoff oder substituiertes oder unsubstituiertes C₁-C₁₂Alkyl, C₁-C₁₂Alkoxy, C₁-C₁₂Alkylthio, C₅-C₆Cycloalkoxy, C₅-C₆Cycloalkylthio, C₆-C₂₄Aryloxy, C₆-C₂₄Arylthio oder A₅-A₁₂Heteroaryloxy, A₅-A₁₂Heteroarylthio, C₅-C₆Cycloalkyl, C₂-C₁₂Alkenyl, C₆-C₁₂Aryl, C₇-C₁₃Aralkyl, oder A₅-A₁₂Heteroaryl bedeuten, oder abhängig voneinander Wasserstoff bedeuten,
oder
eine Verbindung der Formel (XXVI) 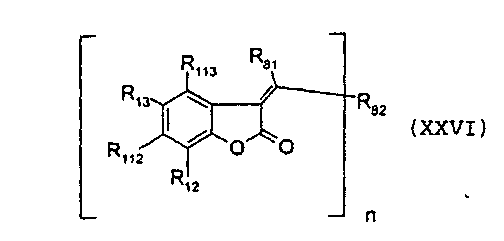 worin
n 1 ist,
R₈₁ einen primären oder sekundären Aminrest bedeutet, und R₈₂ Wasserstoff oder unsubstituiertes oder substituiertes C₁-C₁₂Alkyl, -CO-(C₁-C₂₄Alkyl), -CO-O-(C₁-C₂₄Alkyl), C₆-C₁₂Aryloxy, C₁-C₁₂Alkoxy, C₁-C₁₂Alkylthio, C₅-C₁₂Cycloalkyl, C₅-C₁₂Cycloalkoxy, C₂-C₁₂Alkenyl, einen primären oder sekundären Aminrest, C₆-C₁₈Aryl, -CO-O-(C₆-C₂₄Aryl), -CO-(C₆-C₂₄Aryl), C₆-C₁₈Aryloxy, C₆-C₁₈Arylthio oder A₅-A₁₂Heteroaryl, A₅-A₁₂Heteroaryloxy, A₅-A₁₂Heteroarylthio bedeutet, oder R₈₁ und R₈₂ zusammen einen Lactam-, Chinomethylen-, Hydantoin-, Acenaphthenchinon-, Azlacton-, Pyrazolonyl-, Barbitursäure-, Isoindolinon- oder Isoindolin-Rest bedeuten,
mit der Maßgabe, dass
R₈₂ nicht Wasserstoff bedeutet, wenn R₁₃ Wasserstoff, Methoxy oder Hydroxyl darstellt und R_{12,} R₁₁₂ und R₁₁₃ Wasserstoff darstellen.

4. Verfahren zur Herstellung eines Benzofuran-2-ons (Ia) nach Anspruch 1, das Umsetzen von Benzofuran-2-on (XXXa) 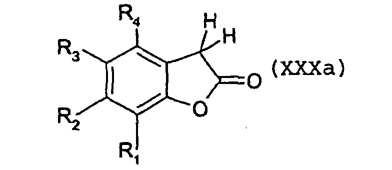 mit einer Verbindung der Formel (XXXIa), (XXXIIa), (XXXIIIa), (XXXIVa) oder (XXXVa) 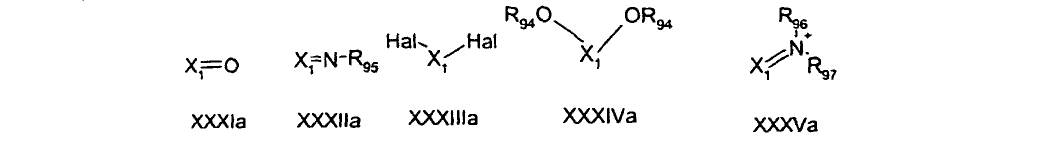 worin
Hal Halogen bedeutet,
und
R₉₄ substituiertes oder unsubstituiertes C₁-C₂₄Alkyl, C₅-C₁₂Cycloalkyl, C₂-C₂₄Alkenyl, C₆-C₂₄Aryl, C₇-C₂₅Aralkyl, oder A₅-A₁₈Heteroaryl bedeutet,
und
R₉₅ Wasserstoff oder Hydroxyl bedeutet,
R₉₆ und R₉₇ unabhängig voneinander C₆-C₁₂Aryl, C₁-C₅Acyl, C₆-C₁₂Aralkyl, oder C₁-C₄Alkyl bedeuten,
und
X₇ einen Methylen-Rest bedeutet, 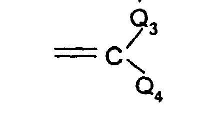 worin
Q₃ einen substituierten oder unsubstituierten primären oder sekundären Amin-Rest bedeutet und Q₄ Wasserstoff oder ein substituiertes oder unsubstituiertes C₁-C₂₄Alkyl, -CO-(C₁-C₂₄Alkyl), -CO-O-(C₁-C₂₄Alkyl), C₁-C₂₄Alkoxy, C₁-C₂₄Alkylthio, C₅-C₁₂Cycloalkyl, C₅-C₁₂Cycloalkoxy, C₅-C₁₂Cycloalkylthio, C₂-C₂₄Alkenyl, C₆-C₂₄Aryl, -CO-O-(C₆-C₂₄Aryl), -CO-(C₆-C₂₄Aryl), C₆-C₂₄Aryloxy, C₆-C₁₂Arylthio, C₇-C₂₅Aralkyl, A₅-A₁₈Heteroaryl, A₅-A₁₈Heteroaryloxy oder A₅-A₁₈Heteroarylthio bedeutet,
oder
Q₃ und Q₄ zusammen einen Lactam-, Chinomethylen-, Hydantoin-, Acenaphthenchinon-, Azlacton-, Pyrazolonyl-, Barbitursäure-, Isoindolinon- oder Isoindolin-Rest bedeuten,
mit der Maßgabe, dass
Q₄ nicht Wasserstoff bedeutet, wenn R₃ Wasserstoff, Methoxy oder Hydroxyl bedeutet und R₁, R₂ und R₄ Wasserstoff bedeuten, umfasst.

5. Verfahren zur Herstellung eines Benzofuran-2-ons (Ic) nach Anspruch 1, das Umsetzen von Benzofuran-2-on (XXXa) und einer Verbindung der Formel (XXXb) 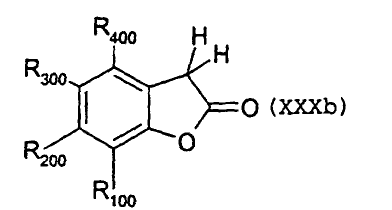 mit einer Verbindung der Formeln (XXXIb), (XxXIIb), (XXXIIIb), (XXXIVb) oder (XXXVb) 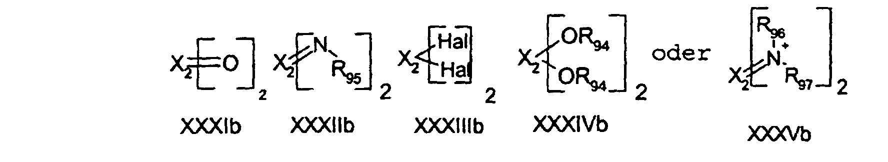 worin
X₂ X₈ bedeutet und der Definition im Anspruch für X₂ entspricht, mit der Maßgabe, dass X₈ nicht 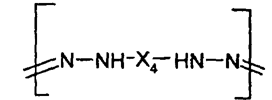 oder 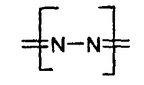 bedeutet,
mit der Maßgabe, dass die Verbindungen (XXXa) und (XXXb) verschieden sind, umfasst.

6. Verfahren zur Herstellung eines Benzofuran-2-ons (Ia) nach Anspruch 1, das Umsetzen von 3-Oxo-benzofuran-2-on (XXXVIa) 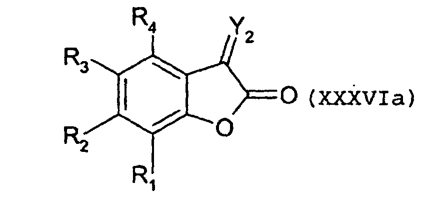 mit einer Verbindung der Formel (XXXVIIa) 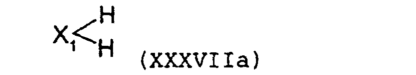 worin
Y₂ O, NR₉₅ oder N⁺(R₉₆R₉₇), NO oder zwei Chloratome bedeutet, wobei die Chloratome jeweils eine Einfachbindung mit dem Benzofuran-2-on (Ia) bilden, umfasst.

7. Verfahren zur Herstellung eines Benzofuran-2-ons (Ic) nach Anspruch 1, das Umsetzen von 3-Oxo-benzofuran-2-on (XXXVIa) und einer Verbindung der Formel (XXXVIb) 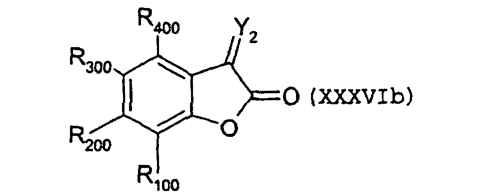 mit einer Verbindung der Formel (XXXVIIb) 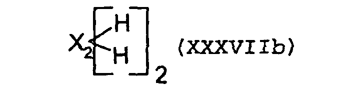 mit der Maßgabe, dass die Verbindungen der Formel (XXXVIa) und (XXXVIb) verschieden sind,
umfasst.

8. Amino-Hydroxy-Verbindungen der Formel (XLIa) oder (XLIb) 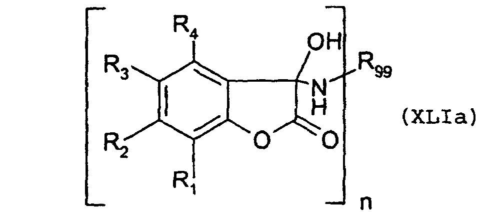 oder 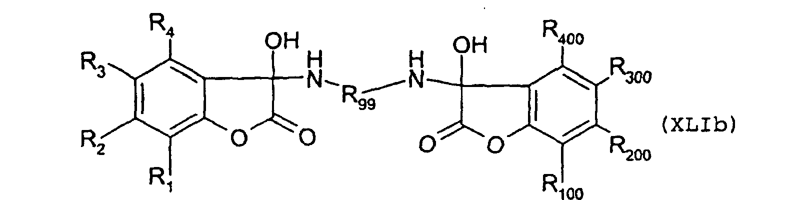 worin
n 1 ist,
R₉₉ Wasserstoff oder substituiertes oder unsubstituiertes C₁-C₂₄Alkyl, C₅-C₁₂Cycloalkyl, C₂-C₂₄Alkenyl, C₆-C₂₄Aryl, C₇-C₂₅Aralkyl oder A₅-A₁₈Heteroaryl bedeutet.

9. Verfahren zur Herstellung einer Amin-Hydroxy-Verbindung der Formel (XLIa) oder (XLIb) nach Anspruch 8, das Umsetzen von 3-Oxo-benzofuran-2-on (XXXVIa) nach Anspruch 6 mit einer Verbindung der Formel (XXXVIIa)
H―X₁―H (XXXVIIa)
oder
Umsetzen von 3-Oxo-benzofuran-2-on (XXXVIa) und (XXXVIb) von Anspruch 7 mit einer Verbindung der Formel (XXXVIIb)
H―X₂―H (XXXVIIb)
umfasst.

10. Verfahren zur Herstellung eines Benzofuran-2-ons (Ia) oder (Ic) nach Anspruch 1, worin X₁ eine Verbindung der Formel (IV) 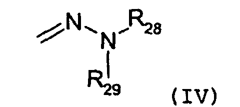 bedeutet,
worin
R₂₈ und R₂₉ unabhängig voneinander substituiertes oder unsubstituiertes C₁-C₂₄Alkyl, C₅-C₁₂Cycloalkyl, C₂-C₂₄Alkenyl, C₆-C₂₄Aryl, C₇-C₂₅Aralkyl, A₅-A₁₈Heteroaryl oder abhängig voneinander Wasserstoff bedeuten,
und
X₂ eine Verbindung der Formel (X) 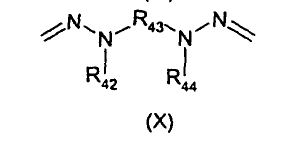 bedeutet,
worin
R₄₂ und R₄₄ unabhängig voneinander substituiertes oder unsubstituiertes C₁-C₂₄Alkyl, C₅-C₁₂Cycloalkyl, C₂-C₂₄Alkenyl, C₆-C₂₄Aryl, C₇-C₂₅Aralkyl, A₅-A₁₈Heteroaryl oder abhängig voneinander Wasserstoff bedeuten,
und
R₄₃ eine direkte Bindung, C₆-C₂₄Arylen, A₅-A₁₈Heteroarylen, C₅-C₁₂Cycloalkyl oder Bi-(C₆-C₂₄)arylen, Bi-(A₅-A₁₈)heteroarylen, C₂-C₂₄Alkenylen, worin Bi- (C₆-C₂₄)arylen, Bi-(A₅-A₁₈)heteroarylen, C₂-C₂₄Alkenylen durch eine direkte Bindung oder eine oder mehrere Zwischeneinheiten, wie -CH=CH-, -CH=N-, -N=N-, -CR₄₄R₄₂-, -CO-, -COO-, -OCO-, -NR₄₂CO-, -CONR₄₂-, -O-, -S-, -SO-, -SO₂- oder -NR₄₂-, miteinander verbunden und/oder unterbrochen sein können,
worin
R₄₂ und R₄₄ unabhängig voneinander Wasserstoff, substituiertes oder unsubstituiertes C₁-C₂₄Alkyl, C₅-C₁₂Cycloalkyl, C₂-C₂₄Alkenyl, C₆-C₂₄Aryl, C₇-C₂₅Aralkyl oder A₅-A₁₈Heteroaryl bedeuten, durch Kupplung diazotierter Amine mit Kupplungskomponenten im wässerigen Medium, das Umsetzen von Benzofuran-2-on (XXXa) von Anspruch 4 mit einem Diazoniumsalz der Formel (XXXVIIIa) 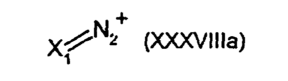 oder
Umsetzen von Benzofuran-2-on (XXXa) und (XXXb) von Anspruch 5 mit einem Diazoniumsalz der Formel (XXXVIIIb) 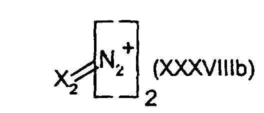 umfasst.

11. Zusammensetzung, bestehend aus 2 bis 10, bevorzugt aus 2 oder 3, Verbindungen der Formeln (Ia) und/oder (Ic) nach Anspruch 1, und/oder (XLIa) und/oder (XLIb) nach Anspruch 8 und/oder dimeren Benzofuran-2-onen der Formel (XLIIb) 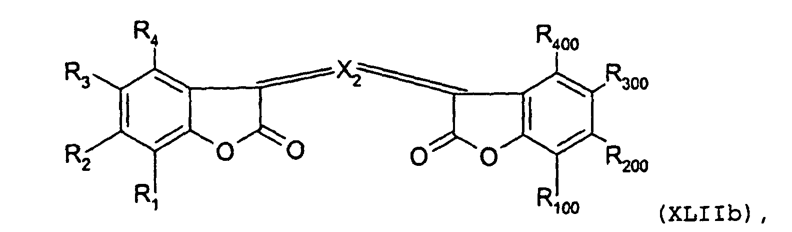 worin X₂ (C₆-C₂₄)Arylen, (A₅-A₁₈)Heteroarylen oder einen zweiwertigen Polymethyliden-, Polyether-, Polyimin-, Polyamin-Rest, oder Bi-(C₆-C₂₄)arylen oder Bi-(A₅-A₁₈)heteroarylen bedeutet, wobei der Bi-(C₆-C₂₄)arylen- oder Bi-(A₅-A₁₈)heteroarylen-Rest direkt oder über einen substituierten oder unsubsituierten Kohlenstoff-, Stickstoff-, Sauerstoff- oder (-N=N-)-Direst verbunden sind,
mit der Maßgabe, dass wenn R₂, R₄, R₂₀₀, R₄₀₀ Wasserstoff darstellen und R₁, R₃, R₁₀₀ und R₃₀₀ tert-Butyl oder Wasserstoff bedeuten oder R₃, R₄, R₃₀₀, R₄₀₀ Wasserstoff darstellen und R₁ und R₂, R₁₀₀ und R₂₀₀ zusammen zweiwertige, unsubstituierte 1,3-Butadien-1,4-ylen-Reste bedeuten, die einen zusätzlich ankondensierten 6-gliedrigen Ring bilden, X₂ kein CH-(C₆H₄)-CH bedeutet.

12. Stoffzusammensetzung, umfassend ein hochmolekulares organisches Material und eine Verbindung der Formel (Ia) nach Anspruch 1,
worin
X₁ X₁₀ bedeutet, worin X₁₀ einen substituierten oder unsubstituierten Hydrazon- oder Imin-Rest bedeutet, oder einen Methylen-Rest 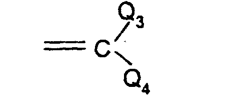 bedeutet, worin
Q₃ einen substituierten oder unsubstituierten primären oder sekundären Aminrest bedeutet und Q₄ Wasserstoff oder ein substituiertes oder unsubstituiertes C₁-C₂₄Alkyl, -CO-(C₁-C₂₄Alkyl), -CO-O-(C₁-C₂₄Alkyl), C₁-C₂₄Alkoxy, C₁-C₂₄Alkylthio, C₅-C₁₂Cycloalkyl, C₅-C₁₂Cycloalkoxy, C₅-C₁₂Cycloalkylthio, C₂-C₂₄-Alkenyl, C₆-C₂₄Aryl, -CO-O- (C₆-C₂₄Aryl), -CO-(C₆-C₂₄Aryl), C6-C₂₄Aryloxy, C₆-C₁₂Arylthio, C₇-C₂₅Aralkyl, A₅-A₁₈Heteroaryl, A₅-A₁₈Heteroaryloxy oder A₅-A₁₈Heteroarylthio bedeutet,
oder
Q₃ und Q₄ zusammen einen Lactam-, Chinomethylen-, Hydantoin-, Acenaphthenchinon, Azlacton-, Pyrazolonyl-, Barbitursäure-, Isoindolinon- oder Isoindolin-Rest bedeuten,
mit der Maßgabe, dass
Q₄ nicht Wasserstoff bedeutet, wenn R₃ Wasserstoff, Methoxy oder Hydroxyl darstellt und R₁, R₂ und R₄ Wasserstoff bedeuten,
oder
eine Zusammensetzung nach Anspruch 11, (XLIa) oder (XLIb) nach Anspruch 8, in einer färberisch wirksamen Menge.

13. Verfahren zur Herstellung von Benzofuran-2-on (Ia) oder (Ic), worin X₁ X₁₀ bedeutet, nach Anspruch 12, und X₁ eine Verbindung der Formel (V) 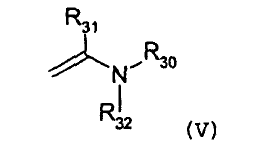 bedeutet,
worin
R₃₁ Wasserstoff oder -NR₈₉R₉₀ bedeutet,
worin
R₃₀, R₃₂, R₈₉ und R₉₀ unabhängig voneinander Wasserstoff, C₁-C₂₄-Alkoxy, C₁-C₂₄Alkylthio, C₅-C₁₂Cycloalkoxy, C₅-C₁₂-Cycloalkylthio, C₅-C₂₄Aryloxy, -thio oder A₅-A₁₈Heteroaryloxy, -thio bedeuten,
oder
mit C₆-C₂₄Aryl substituiertes sekundäres oder tertiäres Amin oder C₆-C₂₄Aryl bedeuten,
und
worin
X₂ die Formel (XI) 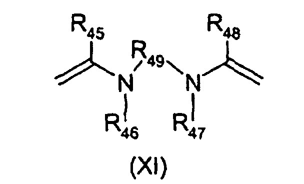 bedeutet,
worin
R₄₆ und R₄₇ unabhängig voneinander substituiertes oder unsubstituiertes C₁-C₂₄Alkyl, C₅-C₁₂Cycloalkyl, C₂-C₂₄Alkenyl, C₆-C₂₄Aryl, C₇-C₂₅Aralkyl oder A₅-A₁₈Heteroaryl bedeuten,
und
R₄₅ und R₄₈ unabhängig voneinander Wasserstoff, C₁-C₂₄Alkyl, C₁-C₂₄Alkoxy, C₁-C₂₄Alkylthio, C₅-C₁₂-Cycloalkyl, C₅-C₁₂Cycloalkoxy, C₅-C₁₂Cycloalkylthio, C₂-C₂₄Alkenyl, C₅-C₂₄Aryl, C₇-C₂₅Aralkyl, C₅-C₂₄Aryloxy, -thio oder A₅-A₁₈Heteroaryl, A₅-A₁₈Heteroaryloxy, -thio bedeuten,
und
R₄₉ eine direkte Bindung, C₆-C₂₄Arylen, A₅-A₁₈Heteroarylen, C₅-C₁₂Cycloalkyl oder Bi- (C₆-C₂₄)arylen, Bi- (A₅-A₁₈)heteroarylen, C₂-C₂₄Alkenylen, worin Bi-(C₆-C₂₄)arylen, Bi-(A₅-A₁₈)heteroarylen, C₂-C₂₄Alkenylen durch eine direkte Bindung oder eine oder mehrere Zwischeneinheiten, wie -CH=CH-, -CH=N-, -N=N-, -CR₄₄R₄₂-, -CO-, -COO-, -OCO-, -NR₄₂CO-, -CONR₄₂-, -O-, -S-, -SO-, -SO₂- oder -NR₄₂-, miteinander verbunden und/oder unterbrochen sein können,
worin
R₄₂ und R₄₄ unabhängig voneinander Wasserstoff, substituiertes oder unsubstituiertes C₁-C₂₄Alkyl, C₅-C₁₂Cycloalkyl, C₂-C₂₄Alkenyl, C₆-C₂₄Aryl, C₇-C₂₅Aralkyl, oder A₅-A₁₈Heteroaryl bedeuten,
durch Formylierung und anschließende Umsetzung mit einem Amin, das Umsetzen von Benzofuran-2-on (XXXa) von Anspruch 4 mit einem Formylierungsreagenz der Formel (XXXVIII)
R₃₅C(OR₃₆)₃ (XXXVIII)
worin
R₃₅ und R₃₆ unabhängig voneinander substituiertes oder unsubstituiertes C₁-C₂₄Alkyl, C₅-C₁₂Cycloalkyl, C₂-C₂₄Alkenyl, C₆-C₂₄Aryl, C₇-C₂₅Aralkyl, oder A₅-A₁₈Heteroaryl bedeuten,
und einer Verbindung der Formel (IXLa) 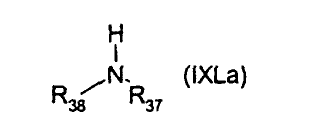 worin
R₃₇ und R₃₈ unabhängig voneinander Wasserstoff oder substituiertes oder unsubstituiertes C₁-C₂₄Alkyl, C₁-C₂₄Alkoxy, C₅-C₁₂Cycloalkoxy, C₅-C₁₂-Cycloalkylthio, C₅-C₆Cycloalkyl, C₂-C₂₄₋ Alkenyl, C₆-C₂₄Aryl, C₅-C₂₄Aryloxy, C₅-C₂₄Arylthio, C₇-C₂₅Aralkyl, einen primären oder sekundären Aminrest, A₅-A₁₈Heteroaryl, A₅-A₁₈Heteroaryloxy oder A₅-A₁₈Heteroarylthio bedeuten, Benzofuran-2-on (XXXa) oder (XXXa) und (XXXb) von Anspruch 5 mit einem Formylierungsreagenz der Formel (XXXVIII)
und einer Verbindung der Formel (IXLb) 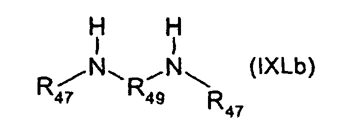 worin
R₄₆ und R₄₇ unabhängig voneinander Wasserstoff oder substituiertes oder unsubstituiertes C₁-C₂₄Alkyl, C₅-C₁₂Cycloalkyl, C₂-C₂₄Alkenyl, C₆-C₂₄Aryl, C₇-C₂₅Aralkyl, oder A₅-A₁₈Heteroaryl bedeuten, zweiwertigen Polyether-, Polyimin-, Polyamin-Rest, Bi-(C₆-C₂₄)arylen, Bi-(A₅-A₁₈)heteroarylen, C₂-C₂₄Alkenylen,
worin Bi-(C₆-C₂₄)arylen, Bi-(A₅-A₁₈)heteroarylen oder C₂-C₂₄Alkenylen durch eine oder mehrere Zwischeneinheiten, wie -CH=CH-, -CH=N-, -N=N-, -CR₄₄R₄₂-, -CO-, -COO-, -OCO-, -NR₄₂CO-, -CONR₄₂-, -O-, -S-, -SO-, -SO₂- oder -NR₄₂-, unterbrochen sein können,
worin
R₄₂ und R₄₄ unabhängig voneinander Wasserstoff, substituiertes oder unsubstituiertes C₁-C₂₄Alkyl, C₅-C₁₂Cycloalkyl, C₂-C₂₄Alkenyl, C₆-C₂₄Aryl, C₇-C₂₅Aralkyl oder A₅-A₁₈Heteroaryl bedeuten, und
R₄₉ eine direkte Bindung oder substituiertes oder unsubstituiertes C₆-C₂₄Arylen, A₅-A₁₈Heteroarylen, C₅-C₁₂Cycloalkylen oder Bi- (C₆-C₂₄)arylen, Bi-(A₅-A₁₈)heteroarylen, C₂-C₂₄Alkenylen, worin Bi-(C₆-C₂₄) arylen, Bi-(A₅-A₁₈)heteroarylen oder C₂-C₂₄Alkenylen durch eine oder mehrere Zwischeneinheiten, wie -CH=CH-, -CH=N-, -N=N-, -CR₄₄R₄₂-, -CO-, -COO-, -OCO-, -NR₄₂CO-, -CONR₄₂-, -O-, -S-, -SO-, -SO₂- oder -NR₄₂-, unterbrochen sein können, bedeutet, umfasst.

14. Verfahren zur Herstellung von Tinten oder für Beschichtungsmaterialien, Druckfarben, Mineralöle, Schmierfette, Wachse oder gefärbte oder pigmentierte Kunststoffe, Nonimpact-printing-Material oder Tonern, das Einarbeiten einer wirksam färbenden Menge der Verbindung nach Anspruch 1 oder der Zusammensetzung nach Anspruch 11 oder Stoffzusammensetzung nach Anspruch 12 darin umfasst.

## Revendications

1. Composé de formule (Ia) ou (Ic) ${\text{Q}}_{\text{1}} {\text{̿X}}_{\text{1}} \text{(Ia)}$${\text{Q}}_{\text{1}} {\text{̿X}}_{\text{2}} {\text{̿Q}}_{\text{2}} \text{(Ic)}$dans lesquelles
Q₁ est une benzofuran-2-one de formule (IIa),
et
Q₂ est une benzofuran-2-one de formule (IIb), 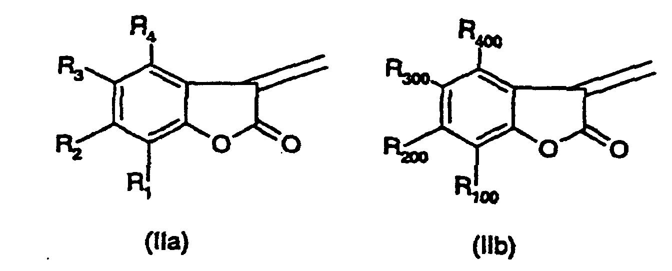 dans lesquelles R₁, R₂, R₃, R₄, R₁₀₀, R₂₀₀, R₃₀₀ ou R₄₀₀ indépendamment les uns des autres représentent un atome d'hydrogène, un atome d'halogène, un groupe hydroxyle, cyano, éther, nitro, une amine, amide, imine, uréthanne, sulfonamide, ester, radical acide carboxylique ou acide sulfonique ou sel carboxylique, sel sulfonique ou un groupe substitué ou non substitué alkyle en C₁-C₂₄, alkoxy en C₁-C₂₄, (alkyl en C₁-C₂₄)thio, cycloalkyle en C₅-C₁₂, cycloalkoxy en C₅-C₁₂, cyclo(alkyl en C₅-C₁)thio, alcényle en C₂-C₂₄, aryle en C₆-C₂₄, aralkyle en C₇-C₂₅, aryloxy en C₆-C₂₄, (aryl en C₆-C₂₄)thio, hétéroaryle en A₅-A₁₈, hétéroaryloxy en A₅-A₁₈ ou (hétéroaryl en A₅-A₁₈)-thio,
ou
R₁ et R₂, R₂ et R₃, R₃ et R₄ ou R₁₀₀ et R₂₀₀, ou R₂₀₀ et R₃₀₀, R₃₀₀ et R₄₀₀ indépendamment les uns des autres sont dans chaque cas conjointement des radicaux bivalents, substitués ou non substitués, tels que des radicaux polycycliques ou 1,3-butadién-l,4-ylène ou -CH=CH-NH-, les deux derniers radicaux formant un cycle supplémentaire condensé de 5 ou 6 chaînons, à condition que Q₁ et Q₂ soient différents et
X₁ représente un radical hydrazone ou imine, à condition que, si R₁, R₂, R₃ et R₄ représentent un atome d'hydrogène, ou au moins un R₁, R₂, R₃ ou R₄ représente un groupe méthyle, le radical hydrazone soit exclu, ou, si R₁, R₂, R₃ ou R₄ représentent un atome d'hydrogène, X₁ ne représente pas un groupe phénylimine- ou 4-diméthylamine-phénylimine,
ou
X₁ représente un radical méthylène 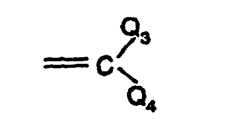 dans lequel
Q₃ est un radical amine primaire ou secondaire non substitué ou substitué et Q₄ représente un atome d'hydrogène ou un groupe substitué ou non substitué alkyle en C₁-C₂₄, -CO-(alkyle en C₁-C₂₄), -CO-O-(alkyle en C₁-C₂₄), alkoxy en C₁-C₂₄, (alkyl en C₁-C₂₄)thio, cycloalkyle en C₅-C₁₂, cycloalkoxy en C₅-C₁₂, cyclo(alkyl en C₅-C₁₂)thio, alcényle en C₂-C₂₄, aryle en C₆-C₂₄, -CO-O-(aryle en C₆-C₂₄), -CO-(aryle en C₆-C₂₄), aryloxy en C₆-C₂₄, (aryl en C₆-C₂₄)thio, aralkyle en C₇-C₂₅, hétéroaryle en A₅-A₁₈, hétéroaryloxy en A₅-A₁₈ ou (hétéroaryl en A₅-A₁₈)thio,
ou
Q₃ et Q₄ représentent conjointement un radical lactame, quinométhylène, hydantoïne, acénaphtènequinone, azlactone, pyrazolonyle, acide barbiturique, isoindolinone ou isoindoline,
à condition que
Q₄ ne représentera pas un atome d'hydrogène si R₃ représente un atome d'hydrogène, un groupe méthoxy ou hydroxyle et R₁, R₂ et R₄ représentent un atome d'hydrogène,
et
X₂ représente un cycle hétérocyclique tétravalent de 5 ou 6 chaînons,
ou représente 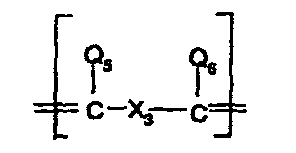 où
X₃ est une simple liaison, un groupe substitué ou non substitué arylène en C₆-C₂₄, hétéroarylène en A₅-A₁₈, 1,2-phénylène, 1,3-phénylène, 1,4-phénylène ou nahtylène, ou un radical polyéther tétravalent, polyimine, polyamine, ou bi(arylène en C₆-C₂₄), bi(hétéroarylène en A₅-A₁₈), alcénylène en C₂-C₂₄, où bi(arylène en C₆-C₂₄), bi(hétéroarylène en A₅-A₁₈) ou alcénylène en C₂-C₂₄ peut être interrompu par un ou plusieurs unités intermédiaires telles que -CH=CH-, -CH=N-, -N=N-, -CR₄₄R₄₂-, -CO-, -COO-, -OCO-, -NR₄₂CO-, -CONR₄₂-, -O-, -S-, -SO-, -SO₂ ou -NR₄₂-,
dans lesquels
R₄₂ et R₄₄ indépendamment l'un de l'autre représentent un atome d'hydrogène, un groupe substitué ou non substitué alkyle en C₁-C₂₄, cycloalkyle en C₅-C₁₂, alcényle en C₂-C₂₄, aryle en C₆-C₂₄, aralkyle en C₇-C₂₅ ou hétéroaryle en A₅-A₁₈, et
Q₅ et Q₆ indépendamment l'un de l'autre représentent un atome d'hydrogène, un groupe aryle en C₆-C₂₄, aryloxy en C₆-C₂₄, alkyle en C₁-C₂₄, alkoxy en C₁-C₂₄, (alkyl en C₁-C₂₄)thio, cycloalkyle en C₅-C₁₂, cycloalkoxy en C₅-C₁₂, cyclo(alkyl en C₅-C₁)thio, alcényle en C₂-C₂₄, aryle en C₆-C₂₄, aryloxy en C₆-C₂₅, (aryl en C₆-C₂₄)thio, ou hétéroaryle en A₅-A₁₈, hétéroaryloxy en A₅-A₁₈, (hétéroaryl en A₅-A₁₈)thio,
à condition que si R₂, R₄, R₂₀₀, R₄₀₀ représentent un atome d'hydrogène et R₁, R₃, R₁₀₀ et R₃₀₀ représentent un groupe tert-butyle ou un atome d'hydrogène, ou R₃, R₄, R₃₀₀, R₄₀₀ représentent un atome d'hydrogène et R₁ et R₂, R₁₀₀ et R₂₀₀ sont conjointement des radicaux 1,3-butadién-1,4-ylène bivalents, non substitués, formant un cycle condensé supplémentaire de 6 chaînons, et Q₅ et Q₆ représentent un atome d'hydrogène, X₃ ne représente pas un groupe 1,4-phénylène,
ou
X₂ réprésente 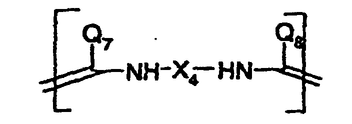 dans laquelle
Q₇ et Q₈ indépendamment l'un de l'autre représentent un atome d'hydrogène, un groupe aryle en C₆-C₂₄, aryloxy en C₆-C₂₄, alkyle en C₁-C₂₄, alkoxy en C₁-C₂₄, (alkyl en C₁-C₂₄)thio, cycloalkyle en C₅-C₁₂, cycloalkoxy en C₅-C₁₂, cyclo(alkyl en C₅-C₁₂)thio, alcényle en C₂-C₂₄, aryle en C₆-C₂₄, aryloxy en C₆-C₂₅, (aryl en C₆-C₂₄)thio ou hétéroaryle en A₅-A₁₈, hétéroaryloxy en A₅-A₁₈, (hétéroaryl en A₅-A₁₈)thio,
et
X₄ représente un groupe arylène en C₆-C₂₄, hétéroarylène en A₅-A₁₈, un radical polyméthylidène ou polyéther bivalent, polyimine, polyamine, ou bi(arylène en C₆-C₂₄), bi(hétéroarylène en A₅-A₁₈), alcénylène en C₂-C₂₄, où bi(arylène en C₆-C₂₄), bi(hétéroarylène en A₅-A₁₈) ou alcénylène en C₂-C₂₄ peuvent être interrompus par une ou plusieurs unités intermédiaires telles que -CH=CH-, -CH=N-, -N=N-, -CR₄₄R₄₂-, -CO-, -COO-, -OCO-, -NR₄₂CO-, -CONR₄₂-, -O-, -S-, -SO-, -SO₂ ou -NR₄₂-,
ou X₂ représente

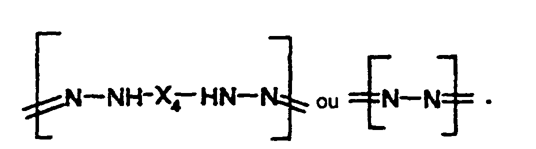

2. Composé selon la revendication 1 de formule (XVI) 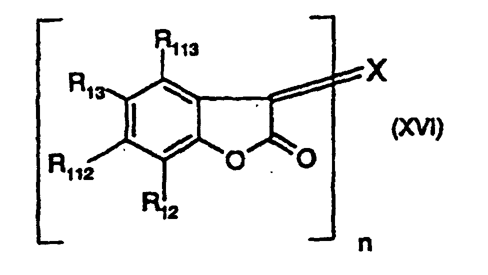 dans laquelle
n vaut 1
X est X₁ tel que défini à la revendication 1, et
R₁₂, R₁₁₂, R₁₃ et R₁₁₃ indépendamment les uns des autres représentent un atome d'hydrogène, un atome d'halogène, un groupe OH, NO₂, R₁₄, OR₁₄, O-alkyle en C₉-C₁₈ ou S-alkyle en C₉-C₁₈, dans laquelle
R₁₄ représente un groupe alkyle en C₁-C₂₄, qui est non substitué ou substitué une ou plusieurs fois par des substituants oxo ou COO-X₅⁺ et qui peut être non interrompu ou interrompu une ou plusieurs fois par des atomes pris parmi N, O et/ou S, ou représente un groupe aralkyle en C₁-C₁₈ ou aryle en C₆-C₁₂ non substitué ou substitué une ou plusieurs fois par des substituants halogène, OR₁₆, NR₁₆R₁₇, COOR₁₆, CONR₁₆R₁₇, NR₁₈COR₁₆ ou NR₁₈COOR₁₆,
X₅⁺ représente un cation H⁺, Na⁺, K⁺, Mg⁺⁺_{1/2}, Ca⁺⁺_{1/2}, Zn⁺⁺_{1/2}, Al⁺⁺⁺_{1/3} ou [NR₁₆R₁₇R₁₈R₁₉]⁺,
et
R₁₅ et R₁₇ indépendamment l'un de l'autre représentent un atome d'hydrogène, un groupe aryle en C₆-C₁₂, aralkyle en C₇-C₁₀ ou alkyle en C₁-C₈, qui est non substitué ou substitué une ou plusieurs fois par des substituants halogène, hydroxyle ou alkoxy en C₁-C₄,
ou
R₁₆ et R₁₇ dans les groupes NR₁₆R₁₇ ou CONR₁₆R₁₇, conjointement avec l'atome d'azote qui les relie, représentent un pyrrolidine, pipéridine, pipérazine ou morpholine, dont chacun est non substitué ou substitué une à quatre fois par un substituant alkyle en C₁-C₄,
et
R₁₈ et R₁₉ indépendamment l'un de l'autre représentent un atome d'hydrogène, un groupe alkyle en C₁-C₈, aryle en C₆-C₁₀ ou aralkyle en C₆-C₁₂,
R₁₂ et R₁₁₂, R₁₁₂ et R₁₃, R₁₃ et R₁₁₃ peuvent aussi représenter, indépendamment les uns des autres, des radicaux bivalents substitués ou non substitués, tels que des radicaux polycycliques,
à condition que, si R₁₂, R₁₁₂, R₁₃ et R₁₁₃ représentent un atome d'hydrogène, ou au moins un R₁₂, R₁₁₂, R₁₃ et R₁₁₃ représente un groupe méthyle, X₁ ne représente pas un radical hydrazone, ou si R₁₂, R₁₁₂, R₁₃ et R₁₁₃ représentent un atome d'hydrogène, X₁ ne représente pas un groupe phénylimine- ou 4-diméthylamine-phénylimine,
ou
si X₁ représente un radical méthylène, 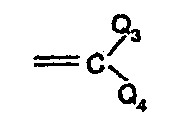 Q₄ ne représentera pas un atome d'hydrogène si R₁₃ représente un atome d'hydrogène, un groupe méthoxy ou hydroxyle, et R₁₂, R₁₁₂ et R₁₁₃ représentent un atome d'hydrogène.

3. Composé selon la revendication 1, de formule (XVII) 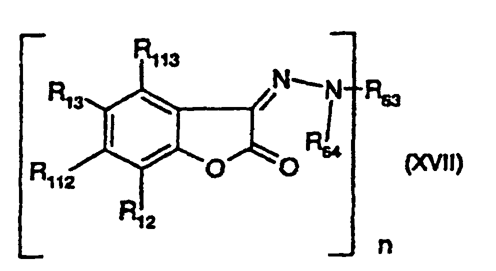 dans laquelle
n vaut 1
R₆₄ indépendamment de R₆₃ représente un radical comme défini pour R₆₃ ou un atome d'hydrogène, et
R₆₃ représente un groupe substitué ou non substitué alkyle en C₁-C₁₂, cycloalkyle en C₅-C₁₂, alcényle en C₂-C₆, aryle en C₆-C₁₂, aralkyle en C₇-C₁₃ ou hétéroaryle en A₅-A₁₂,
ou
un composé de formule (XXIV) 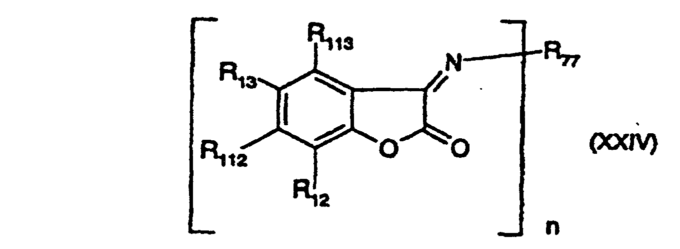 dans laquelle
n vaut 1,
R₇₇ représente un groupe substitué ou non substitué alkyle en C₁-C₁₂, cycloalkyle en C₅-C₆, alcényle en C₂-C₆, aryle en C₆-C₁₂, aralkyle en C₇-C₁₃ ou hétéroaryle en A₅-A₁₂, à condition qu'à la formule (XXIV), si R₁₂, R₁₁₂, R₁₃ ou R₁₁₃ représentent un atome d'hydrogène, R₇₇ ne représente pas un groupe phénylimine- ou 4-diméthylamine-phénylimine non substitué,
ou
un composé de formule (XXV) 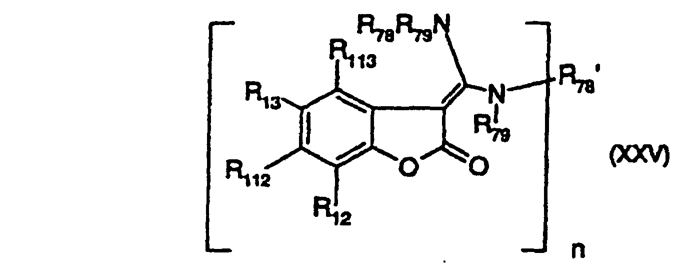 dans laquelle
n vaut 1,
R₇₈, R₇₈, et R₇₉ indépendamment les uns des autres représentent un atome d'hydrogène, un groupe non substitué ou substitué alkyle en C₁-C₁₂, alkoxy en C₁-C₁₂, (alkyl en C₁-C₁₂)thio, cycloalkoxy en C₅-C₆, (cycloalkyl en C₅-C₆)thio, aryloxy en C₆-C₂₄, (aryl en C₆-C₂₄)thio ou hétéroaryloxy en A₅-A₁₂, hétéroarylthio en A₅-A₁₂, cycloalkyle en C₅-C₆, alcényle en C₂-C₁₂, aryle en C₆-C₁₂, aralkyle en C₇-C₁₃ ou hétéroaryle en A₅-A₁₂, ou en dépendant les uns des autres représentent un atome d'hydrogène,
ou
un composé de formule (XXVI) 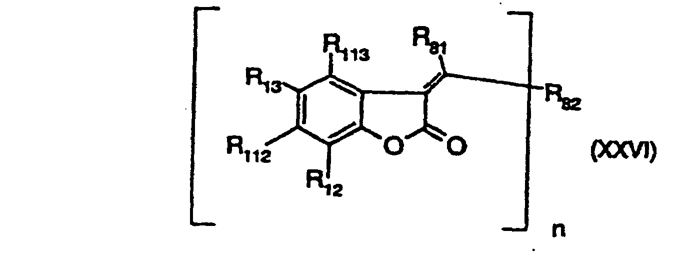 dans laquelle
n vaut 1,
R₈₁ représente un radical amine primaire ou secondaire, et R₈₂ représente un atome d'hydrogène ou un groupe substitué ou non substitué alkyle en C₁-C₁₂, -CO-(alkyle en C₁-C₂₄), -CO-O-(alkyle en C₁-C₂₄), aryloxy en C₆-C₁₂, alkoxy en C₁-C₁₂, (alkyl en C₁-C₁₂)thio, cycloalkyle en C₅-C₁₂, cycloalkoxy en C₅-C₁₂, alcényle en C₂-C₁₂, un radical amine primaire ou secondaire, aryle en C₆-C₁₈, -CO-O-(aryle en C₆-C₂₄), -CO-(aryle en C₆-C₂₄), aryloxy en C₆-C₁₈, (aryl en C₆-C₁₈)thio ou hétéroaryle en A₅-A₁₂, hétéroaryloxy en A₅-A₁₂ ou (hétéroaryl en A₅-A₁₂)thio, ou R₈₁ et R₈₂ représentent conjointement un radical lactame, quinométhylène, hydantoïne, acénaphtènequinone, aziactone, pyrazolonyle, acide barbiturique, isoindolinone ou isoindoline,
à condition que
R₈₂ ne représente pas un atome d'hydrogène si R₁₃ représente un atome d'hydrogène, un groupe méthoxy ou hydroxyle, et R₁₂, R₁₁₂ et R₁₁₃ représentent un atome d'hydrogène.

4. Procédé pour la préparation d'une benzofuran-2-one (Ia) selon la revendication 1, qui comprend la réaction de la benzofuran-2-one (XXXa) 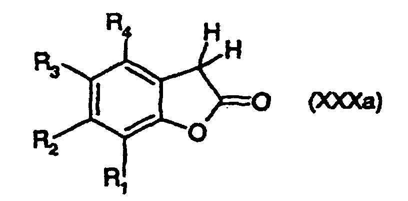 sur un composé de formules (XXXIa), (XXXIIa), (XXXIIIa), (XXXXIVa) ou (XXXVa) 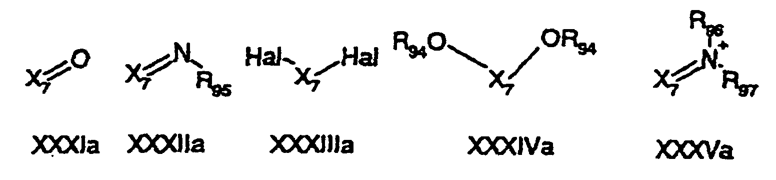 où
Hal représente un atome d'halogène
et
R₉₄ représente un groupe substitué ou non substitué alkyle en C₁-C₂₄, cycloalkyle en C₅-C₁₂, alcényle en C₂-C₂₄, aryle en C₆-C₂₄, aralkyle en C₇-C₂₅ ou hétéroaryle en A₅-A₁₈,
et
R₉₅ représente un atome d'hydrogène ou un groupe hydroxyle,
R₉₆ et R₉₇ indépendamment l'un de l'autre représentent un groupe aryle en C₆-C₁₂, acyle en C₁-C₅, aralkyle en C₆-C₁₂ ou alkyle en C₁-C₄,
et
X₇ représente un radical méthylène, 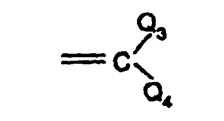 dans laquelle
Q₃ est un radical amine primaire ou secondaire non substitué ou substitué et Q₄ représente un atome d'hydrogène ou un groupe substitué ou non substitué alkyle en C₁-C₂₄, -CO-(alkyle en C₁-C₂₄), -CO-O-(alkyle en C₁-C₂₄), alkoxy en C₁-C₂₄, (alkyl en C₁-C₂₄)thio, cycloalkyle en C₅-C₁₂, cycloalkoxy en C₅-C₁₂, cyclo(alkyl en C₅-C₁₂)thio, alcényle en C₂-C₂₄, aryle en C₆-C₂₄, -CO-O-(aryle en C₆-C₂₄), -CO-(aryle en C₆-C₂₄), aryloxy en C₆-C₂₄, (aryl en C₆-C₂₄)thio, aralkyle en C₇-C₂₅, hétéroaryle en A₅-A₁₈, hétéroaryloxy en A₅-A₁₈ ou (hétéroaryl en A₅-A₁₈)thio,
ou
Q₃ et Q₄ représentent conjointement un radical lactame, quinométhylène, hydantoïne, acénaphtènequinone, azlactone, pyrazolonyle, acide barbiturique, isoindolinone ou isoindoline,
à condition que
Q₄ ne représente pas un atome d'hydrogène si R₃ représente un atome d'hydrogène, un groupe méthoxy ou hydroxyle et R₁, R₂ et R₄ représentent un atome d'hydrogène.

5. Procédé pour la préparation d'une benzofuran-2-one (Ic) selon la revendication 1, comprenant la réaction d'une benzofuran-2-one (XXXa) et d'un composé de formule (XXXb) 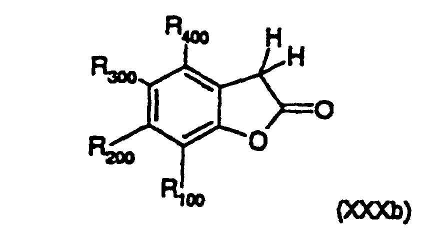 sur un composé de formules (XXXIb), (XXXIIb), (XXXIIIb), (XXXIVb) ou (XXXVb) 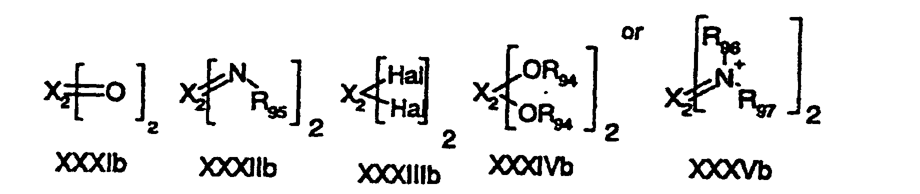 dans lesquelles
X₂ représente X₈ et correspond à la définition dans la revendication de X₂, à condition que X₈ ne représente pas 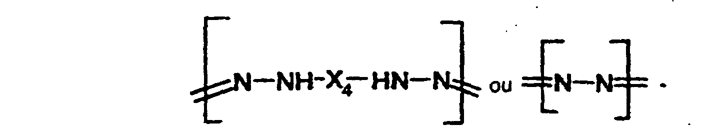 à condition que les composés de formule (XXXa) et (XXXb) soient différents.

6. Procédé pour la préparation d'une benzofuran-2-one (Ia) selon la revendication 1, comprenant la réaction de la 3-oxo-benzofuran-2-one (XXXVIa) 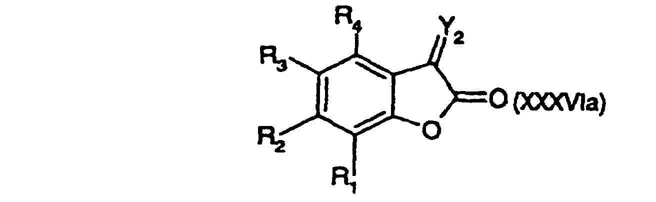 sur un composé de formule (XXXVIIa) 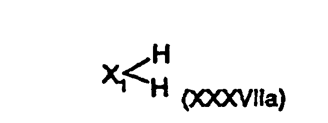 dans laquelle
Y₂ représente O, NR₉₅ ou N⁺(R₉₆R₉₇), NO ou deux atomes de chlore, les atomes de chlore formant chacun une simple liaison avec la benzofuran-2-one (Ia).

7. Procédé pour la préparation d'une benzofuran-2-one (Ic) selon la revendication 1, comprenant la réaction d'une 3-oxo-benzofuran-2-one (XXXVIa) et d'un composé de formule (XXXVIb) 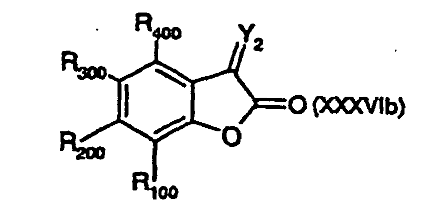 sur un composé de formule (XXXVIIb) 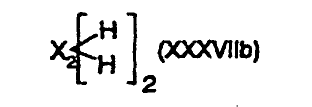 à condition que les composés de formule (XXXVIa) et (XXXVIb) soient différents.

8. Composé aminohydroxylé de formule (XLIa) ou (XLIb) 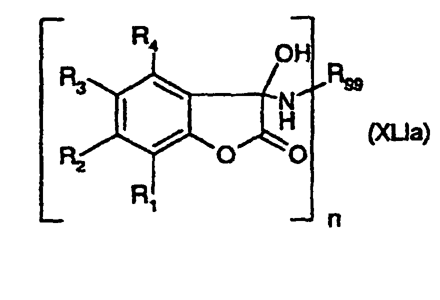 ou 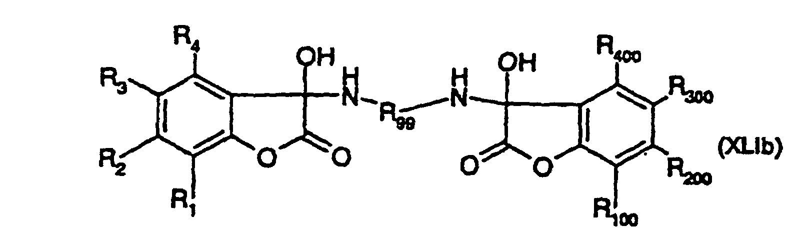 dans laquelle
n vaut 1
R₉₉ représente un atome d'hydrogène ou un groupe non substitué ou substitué alkyle en C₁-C₂₄, cycloalkyle en C₅-C₁₂, alcényle en C₂-C₂₄, aryle en C₆-C₂₄, aralkyle en C₇-C₂₅ ou hétéroaryle en A₅-A₁₈.

9. Procédé pour la préparation d'un composé aminohydroxylé de formule (XLIa) ou (XLIb) selon la revendication 8, qui comprend la réaction la réaction d'une 3-oxo-benzofuran-2-one (XXXVIa) selon la revendication 6 sur un composé de formule (XXXVIIa)
**H―X**_{**1**}**―H** (XXXVIIa)
ou
la réaction d'une 3-oxo-benzofuran-2-one (XXXVIa) et (XXXVIb) de la revendication 7 sur un composé de formule (XXXVIIb)
**H―X**_{**2**}**―H** (XXXVIIb).

10. Procédé pour la préparation d'une benzofuran-2-one (Ia) ou (Ic) selon la revendication 1 dans laquelle X₁ est un composé de formule (IV) 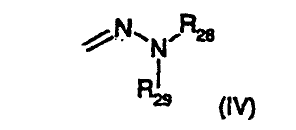 dans laquelle
R₂₈ et R₂₉ indépendamment l'un de l'autre représentent un groupe substitué ou non substitué alkyle en C₁-C₂₄, cycloalkyle en C₅-C₁₂, alcényle en C₂-C₂₄, aryle en C₆-C₂₄, aralkyle en C₇-C₂₅, hétéroaryle en A₅-A₁₈ ou dépendant l'un de l'autre un atome d'hydrogène
et
X₂ est un composé de formule (X) 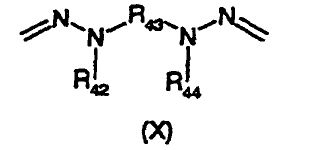 dans laquelle
R₄₂ et R₄₄ indépendamment l'un de l'autre représentent un groupe substitué ou non substitué alkyle en C₁-C₂₄, cycloalkyle en C₅-C₁₂, alcényle en C₂-C₂₄, aryle en C₆-C₂₄, aralkyle en C₇-C₂₅, hétéroaryle en A₅-A₁₈ ou en dépendant l'un de l'autre représentent un atome d'hydrogène,
et
R₄₃ est une liaison directe, un radical arylène en C₆-C₂₄, hétéroarylène en A₅-A₁₈, cycloalkyle en C₅-C₁₂ ou bi(arylène en C₆-C₂₄), bi(hétéroarylène en A₅-A₁₈), alcénylène en C₂-C₂₄, où bi(arylène en C₆-C₂₄), bi(hétéroarylène en A₅-A₁₈), alcénylène en C₂-C₂₄ peuvent être reliés les uns aux autres et/ou interrompus par une liaison directe ou par une ou plusieurs unités intermédiaires telles que -CH=CH-, -CH=N-, -N=N-, -CR₄₄R₄₂-, -CO-, -COO-, -OCO-, -NR₄₂CO-, -CONR₄₂-, -O-, -S-, -SO-, -SO₂ ou -NR₄₂-,
dans lesquelles
R₄₂ et R₄₄ indépendamment l'un de l'autre représentent un atome d'hydrogène, un groupe substitué ou non substitué alkyle en C₁-C₂₄, cycloalkyle en C₅-C₁₂, alcényle en C₂-C₂₄, aryle en C₆-C₂₄, aralkyle en C₇-C₂₅ ou hétéroaryle en A₅-A₁₈,
par couplage d'amines diazotées avec des composants de couplage dans un milieu aqueux, qui comprend la réaction de la benzofuran-2-one (XXXa) de la revendication 4 sur un sel de diazonium de formule (XXXVIIIa) 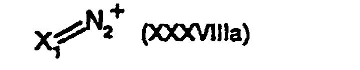 ou la réaction de la benzofuran-2-one (XXXa) et (XXXb) de la revendication 5 sur un sel de diazonium de formule (XXXVIIIb)

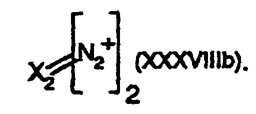

11. Composition constituée par 2 à 10, de préférence 2 ou 3, composés de formules (Ia) et/ou (Ic) selon la revendication 1, et/ou (XLIa) et/ou (XLIb) selon la revendication 8, et/ou des benzofuran-2-ones dimères de formule (XLIIb) 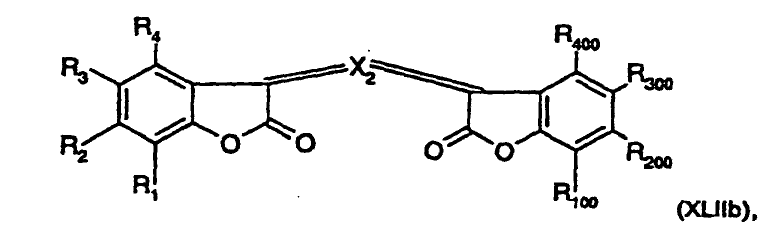 dans laquelle X₂ représente un groupe arylène en C₆-C₂₄, hétéroarylène en A₅-A₁₈ ou un radical polydiméthylène bivalent, polyéther, polyimine, polyamine, ou bi(arylène en C₆-C₂₄) ou bi-(hétéroarylène A₅-A₁₈), le radical bi(arylène en C₆-C₂₄) ou bi(hétéroarylène en A₅-A₁₈) étant lié directement ou par l'intermédiaire d'un atome de carbone, d'azote ou d'oxygène substitué ou non substitué ou d'un diradical (-N=N-), à condition que si R₂, R₄, R₂₀₀, R₄₀₀ représentent un atome d'hydrogène et R₁, R₃, R₁₀₀ et R₃₀₀ représentent un groupe tert-butyle ou un atome d'hydrogène ou R₃, R₄, R₃₀₀, R₄₀₀ représentent un atome d'hydrogène et R₁ et R₂, R₁₀₀ et R₂₀₀ représentent conjointement des radicaux 1,3-butadién-1,4-ylènes bivalents, non substitués, formant un cycle condensé supplémentaire de 6 chaînons, X₂ ne représente pas un groupe CH-(C₆H₄)-CH.

12. Composition en question comprenant une matière organique de haut poids moléculaire et un composé de formule (Ia) selon la revendication 1
dans laquelle
X₁ représente X₁₀, où X₁₀ est un radical hydrazone ou imine substitué ou non substitué ou un radical méthylène 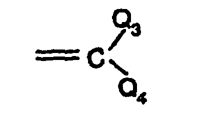 dans laquelle
Q₃ est un radical amine primaire ou secondaire non substitué ou substitué et Q₄ représente un atome d'hydrogène ou un groupe substitué ou non substitué alkyle en C₁-C₂₄, -CO-(alkyle en C₁-C₂₄), -CO-O-(alkyle en C₁-C₂₄), alkoxy en C₁-C₂₄, (alkyl en C₁-C₂₄)thio, cycloalkyle en C₅-C₁₂, cycloalkoxy en C₅-C₁₂, cyclo(alkyl en C₅-C₁₂)thio, alcényle en C₂-C₂₄, aryle en C₆-C₂₄, -CO-O-(aryle en C₆-C₂₄), -CO-(aryle en C₆-C₂₄), aryloxy en C₆-C₂₄, (aryl en C₆-C₁₂)thio, aralkyle en C₇-C₂₅, hétéroaryle en A₅-A₁₈, hétéroaryloxy en A₅-A₁₈ ou (hétéroaryl en A₅-A₁₈)thio,
ou
Q₃ et Q₄ représentent conjointement un radical lactame, quinométhylène, hydantoïne, acénaphtènequinone, azlactone, pyrazolonyle, acide barbiturique, isoindolinone ou isoindoline,
à condition que
Q₄ ne représente pas un atome d'hydrogène si R₃ représente un atome d'hydrogène, un groupe méthoxy ou hydroxyle et R₁, R₂ et R₄ représentent un atome d'hydrogène,
ou
une composition selon la revendication 11, (XLIa) ou (XLIb) selon la revendication 8, dans une quantité colorante efficace.

13. Procédé pour la préparation d'une benzofuran-2-one (Ia) ou (Ic) dans laquelle X₁ représente X₁₀ selon la revendication 12 et X₁ est un composé de formule 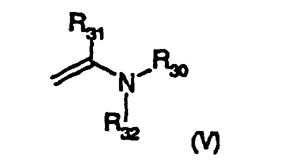 dans laquelle
R₃₁ représente un atome d'hydrogène ou un groupe -NR₈₉R₉₀,
dans lequel R₃₀, R₃₂, R₈₉ et R₉₀ indépendamment les uns des autres représentent un atome d'hydrogène, un groupe alkoxy en C₁-C₂₄, (alkyl en C₁-C₂₄)thio, cycloalkoxy en C₅-C₁₂, cyclo(alkyl en C₅-C₁₂)thio, aryloxy en C₅-C₂₄, -thio ou hétéroaryloxy en A₅-A₁₈, -thio,
ou
représentent une amine secondaire ou tertiaire substituée par un substituant aryle en C₆-C₂₄ ou un groupe aryle en C₆-C₂₄,
et
où X₂ répond à la formule (IX) 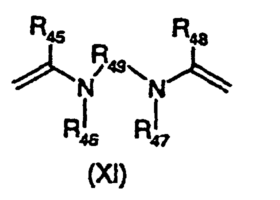 dans laquelle
R₄₆ et R₄₇ indépendamment l'un de l'autre représentent un groupe non substitué ou substitué alkyle en C₁-C₂₄, cycloalkyle en C₅-C₁₂, alcényle en C₂-C₂₄, aryle en C₆-C₂₄, aralkyle en C₇-C₂₅ ou hétéroaryle en A₅-A₁₈,
et
R₄₅ et R₄₈ indépendamment l'un de l'autre représentent un atome d'hydrogène, un groupe alkyle en C₁-C₂₄, alkoxy en C₁-C₂₄, (alkyl en C₁-C₂₄)thio, cycloalkyle en C₅-C₁₂, cycloalkoxy en C₅-C₁₂, cyclo(alkyl en C₅-C₁₂)thio, alcényle en C₂-C₂₄, aryle en C₆-C₂₄, aralkyle en C₇-C₂₅, aryloxy en C₅-C₂₄, -thio ou hétéroaryle en A₅-A₁₈, hétéroaryloxy en A₅-A₁₈, -thio,
et
R₄₉ est une liaison directe, un radical arylène en C₆-C₂₄, hétéroarylène en A₅-A₁₈, cycloalkyle en C₅-C₁₂ ou bi(arylène en C₆-C₂₄), bi(hétéroarylène en A₅-A₁₈), alcénylène en C₂-C₂₄, dans laquelle bi(arylène en C₆-C₂₄), bi (hétéroarylène en A₅-A₁₈), alcénylène en C₂-C₂₄ peuvent être reliés les uns aux autres et/ou interrompus par une liaison directe ou par une ou plusieurs unités intermédiaires telles que -CH=CH-, -CH=N-, -N=N-, -CR₄₄R₄₂-, -CO-, -COO-, -OCO-, -NR₄₂CO-, -CONR₄₂-, -O-, -S-, -SO-, -SO₂ ou -NR₄₂-,
dans lesquelles
R₄₂ et R₄₄ indépendamment l'un de l'autre représentent un atome d'hydrogène, un groupe non substitué ou substitué alkyle en C₁-C₂₄, cycloalkyle en C₅-C₁₂, alcényle en C₂-C₂₄, aryle en C₆-C₂₄, aralkyle en C₇-C₂₅ ou hétéroaryle en A₅-A₁₈ par formylation et réaction ultérieure sur une amine qui comprend la réaction de la benzofruan-2-one (XXXa) de la revendication 4 sur un réactif de formylation de formule (XXXVIII)
**R**_{**35**}**C(OR**_{**36**}**)**_{**3**} (XXXVIII)
dans laquelle
R₃₅ et R₃₆ indépendamment l'un de l'autre représentent un groupe non substitué ou substitué alkyle en C₁-C₂₄, cycloalkyle en C₅-C₁₂, alcényle en C₂-C₂₄, aryle en C₆-C₂₄, aralkyle en C₇-C₂₅ ou hétéroaryle en A₅-A₁₈,
et un composé de formule (IXLa) 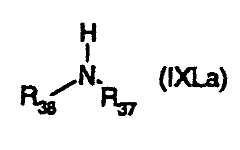 dans laquelle
R₃₇ et R₃₈ indépendamment l'un de l'autre représentent un atome d'hydrogène, un groupe non substitué ou substitué alkyle en C₁-C₂₄, alkoxy en C₁-C₂₄, cycloalkoxy en C₅-C₁₂, cyclo(alkyl en C₅-C₁₂)thio, cycloalkyle en C₅-C₆, alcényle en C₂-C₂₄, aryle en C₆-C₂₄, aryloxy en C₅-C₂₄, (aryl en C₅-C₂₄)thio, aralkyle en C₇-C₂₅, un radical amine primaire ou secondaire, hétéroaryle en A₅-A₁₈, hétéroaryloxy en A₅-A₁₈ ou (hétéroaryl en A₅-A₁₈)thio, d'une benzofuran-2-one (XXXa) ou (XXXa) et (XXXb) de la revendication 5 sur un réactif de formylation de formule (XXXVIII) et un composé de formule (IXLb) 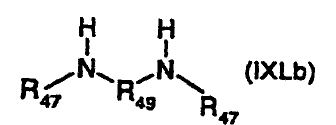 dans laquelle
R₄₆ et R₄₇ indépendamment l'un de l'autre représentent un groupe non substitué ou substitué alkyle en C₁-C₂₄, cycloalkyle en C₅-C₁₂, alcényle en C₂-C₂₄, aryle en C₆-C₂₄, aralkyle en C₇-C₂₅ ou hétéroaryle en A₅-A₁₈, un radical bivalent polyéther, polyimine, polyamine, bi(arylène en C₆-C₂₄), bi (hétéroarylène en A₅-A₁₈), alcénylène en C₂-C₂₄, dans lesquelles bi(arylène en C₆-C₂₄), bi(hétéroarylène en A₅-A₁₈) ou alcénylène en C₂-C₂₄ peuvent être interrompus par une ou plusieurs unités intermédiaires telles que -CH=CH-, -CH=N-, -N=N-, -CR₄₄R₄₂-, -CO-, -COO-, -OCO-, -NR₄₂CO-, -CONR₄₂-, -O-, -S-, -SO-, -SO₂ ou -NR₄₂-,
dans lesquelles
R₄₂ et R₄₄ indépendamment l'un de l'autre représentent un atome d'hydrogène, un groupe substitué ou non substitué alkyle en C₁-C₂₄, cycloalkyle en C₅-C₁₂, alcényle en C₂-C₂₄, aryle en C₆-C₂₄, aralkyle en. C₇-C₂₅ ou hétéroaryle en A₅-A₁₈
et
R₄₉ est une liaison directe, un groupe non substitué ou substitué arylène en C₆-C₂₄, hétéroarylène en A₅-A₁₈, cycloalkylène en C₅-C₁₂ ou bi(arylène en C₆-C₂₄), bi(hétéroarylène en A₅-A₁₈), alcénylène en C₂-C₂₄, où bi(arylène en C₆-C₂₄), bi(hétéroarylène en A₅-A₁₈) ou alcénylène en C₂-C₂₄ peuvent être interrompus par une ou plusieurs unités intermédiaires telles que -CH=CH-, -CH=N-, -N=N-, -CR₄₄R₄₂-, -CO-, -COO-, -OCO-, -NR₄₂CO-, -CONR₄₂-, -O-, -S-, -SO-, -SO₂ ou -NR₄₂-.

14. Méthode de préparation d'encres ou de matières de revêtement, d'encres d'impression, d'huiles minérales, de graisses lubrifiantes ou de plastiques colorés ou pigmentés, de matières d'impression non mécanique ou de toners, qui comprend l'incorporation d'une quantité efficace colorante d'un composé selon la revendication 1 ou d'une composition selon la revendication 11 ou une composition en question selon la revendication 12.
